# EUROPEAN PATENT APPLICATION

(11) **EP 2 269 989 A1**
(43) Date of publication of application: **05.01.2011**
(21) Application number: 09734611.8
(22) Date of filing: 22.04.2009
(51) Int. Cl.: C07D 213/82, A61K 31/44, A61K 31/4436, A61P 13/02, A61P 43/00, C07D 409/04

(54) **IMINOPYRIDINE DERIVATIVE AND USE THEREOF**

(30) Priority: 23.04.2008 JP 2008113130
(71) Applicant: Takeda Pharmaceutical Company Limited, Osaka 541-0045 (JP)
(72) Inventor: YOSHIDA, Masato, Osaka-shi Osaka 532-8686 (US); KOHARA, Yasuhisa, Osaka-shi Osaka 532-8686 (JP); SAKAUCHI, Nobuki, Osaka-shi Osaka 532-8686 (JP); SATO, Ayumu, Osaka-shi Osaka 532-8686 (JP)
(74) Representative: Jones, Nicholas Andrew
(86) International application number: PCT/JP2009/057961
(87) International publication number: WO 2009/131135

(57) **Abstract**

The present invention aims to provide a compound having a superior selective α_{1D} adrenergic receptor antagonistic action and useful as an agent for the prophylaxis or treatment of a lower urinary tract disease and the like.

The present invention provides a compound represented by the formula (I) 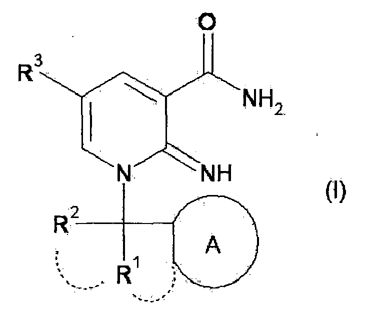 wherein each symbol is as defined in the specification, or a salt thereof.

## Description

### Technical Field

The present invention relates to an iminopyridine derivative having a superior selective α_{1D} adrenergic receptor (hereinafter to be simply also referred to as an α_{1D} receptor) antagonistic action and useful as an agent for the prophylaxis or treatment of a lower urinary tract disease and the like.

### [Background of the Invention]

α₁ Adrenergic receptors (hereinafter to be simply also referred to as an α₁ receptor) are widely distributed in the cardiovascular system, lower urinary tracts and the like, and involved in sympathetic nerve response activities. Since the relationship with pathologies such as hypertension, cardiac hypertrophy and dysuria has been suggested, α₁ receptors have attracted attention for some time, and many attempts have been made to develop therapeutic drugs. In recent years, it has been clarified that α₁ blockers are effective for dysuria associated with benign prostatic hypertrophy (BPH). Coupled with the marketability thereof, extensive interests have been created again (non-patent document 1).
The α₁ receptor gene was cloned from the late 1980s to the early 1990s, and the presence of three subtypes of α_{1A}, α_{1B} and α_{1D} has been clarified. Among these, α_{1D} receptor has been confirmed to express in a number of tissues such as blood vessel, brain, spinal cord, gastrointestinal tract, bladder, kidney and the like. While the physiological function of α_{1D} receptor has not been elucidated, α_{1D} receptor antagonists may provide therapeutic drugs for various diseases since they are localized widely.
A greater distribution of α_{1D} receptors in the bladder, parasympathetic nerve nucleus of the sacral cord, and the like as compared to other subtypes has been confirmed (non-patent documents 2, 3), thus suggesting strong involvement in urine storage. In fact, there is a report on a significant increase in the bladder capacity and the single voided volume in α_{1D} knockout mouse (non-patent document 4). Recent reports have documented that the expression amount of α_{1D} receptor mRNA increases in the bladder of BPH patients and BPH model animal (non-patent documents 5 and 6), the bladder muscle isolated from BPH patients may show a promoted contractile function via α_{1D} receptor (non-patent document 7) and the like, thus suggesting a possible involvement of an α_{1D} receptor expressed in the bladder in the pathology of BPH. From the foregoing, an α_{1D} receptor antagonist is promising as an agent for the prophylaxis or treatment of a lower urinary tract disease and the like.
As examples of the compound showing selective an α_{1D} receptor antagonistic action, non-patent document 8 describes a compound represented by the formula

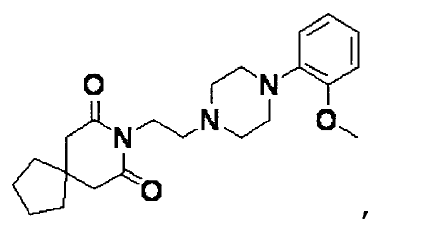

patent document 1 describes a compound represented by the formula

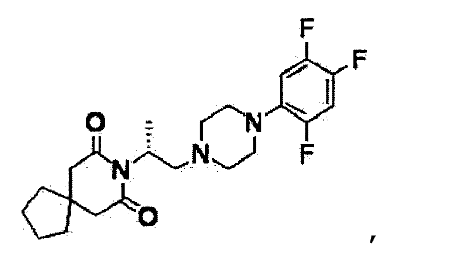

patent document 2 describes a compound represented by the formula

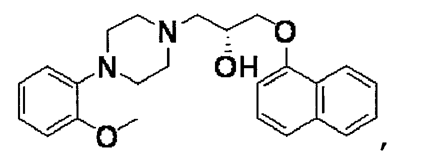

patent document 3 describes a compound represented by the formula

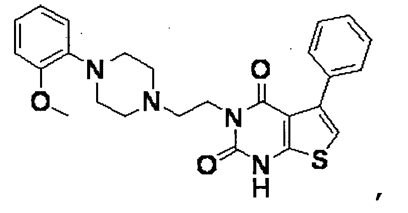

and non-patent document 9 describes a compound represented by the formula

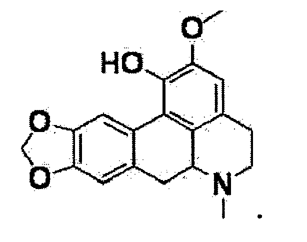

In addition, as iminopyridine derivatives, those described in patent documents 4 to 7 and non-patent documents 10 to 32 are known.

Patent document 8 describes compounds represented by the formulas

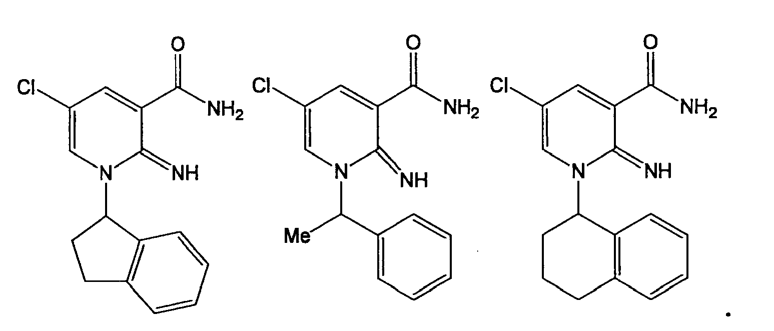

### Citation List

### Patent Literature

patent document 1: WO00/04012
patent document 2: US3997666
patent document 3: WO00/04027
patent document 4: DD 263759
patent document 5: EP47977
patent document 6: DD106377
patent document 7: JP-B-48-40544
patent document 8: WO08/050732

### Non-Patent Literature

non-patent document 1: Yakugaku Zasshi 126, 187-198, 2006
non-patent document 2: Molecular Brain Research 63, 254-261, 1999
non-patent document 3: J. Urol. 160: 937-943., 1998
non-patent document 4: J. Urol. 174: 370-4., 2005
non-patent document 5: J. Urol. 170: 649-653., 2003
non-patent document 6: J. Urol. 167: 1513-1521., 2002
non-patent document 7: J. Urol. 173: 657-61., 2005
non-patent document 8: Eur. J. Pharmacol., 272, (1995), R5-R6
non-patent document 9: Eur. J. Pharmacol., 445, (2002), 21-29
non-patent document 10: Heteroatom Chemistry (2004), 15(4), 293-299
non-patent document 11: Latvijas Kimijas Zurnals (1995), (3-4), 109-113
non-patent document 12: Arzneimittel-Forschung (1995), 45(9), 957-62
non-patent document 13: Journal of the Chinese Chemical Society (Taipei, Taiwan) (1993), 40(2), 181-7
non-patent document 14: Zhurnal Strukturnoi Khimii (1988), 29(5), 169-72
non-patent document 15: Latvijas PSR Zinatnu Akademijas Vestis, Kimijas Serija (1986), (4), 471-8
non-patent document 16: Latvijas PSR Zinatnu Akademijas Vestis, Kimijas Serija (1985), (3), 351-8
non-patent document 17: Latvijas PSR Zinatnu Akademijas Vestis, Kimijas Serija (1985), (2), 200-5
non-patent document 18: Tetrahedron (1980), 36(6), 785-9
non-patent document 19: Zeitschrift fuer Naturforschung, Teil B: Anorganische Chemie, Organische Chemie (1980), 35B(4), 490-3
non-patent document 20: Tetrahedron (1979), 35(21), 2591-3
non-patent document 21: Fette, Seifen, Anstrichmittel (1980), 82(2), 82-6
non-patent document 22: Tetrahedron (1979), 35(6), 809-12
non-patent document 23: Journal of Chemical Society of Japan (1978), (5), 730-6
non-patent document 24: Tetrahedron Letters (1977), (15), 1333-6
non-patent document 25: Journal fuer Praktische Chemie (Leipzig) (1976), 318(5), 705-30
non-patent document 26: Zeitschrift fuer Chemie (1973), 13(9), 342-3
non-patent document 27: Journal of Chemical Society [Section] C: Organic (1971), (10), 1892-5
non-patent document 28: Angewandte Chemie, International Edition in English (1971), 10(1), 68-70
non-patent document 29: Chemical & Pharmaceutical Bulletin (1969), 17(11), 2209-16
non-patent document 30: Chemical & Pharmaceutical Bulletin (1966), 14(8), 861-6
non-patent document 31: Doklady Akademii Nauk SSSR (1949), 66, 647-50
non-patent document 32: Ann. (1925), 443, 272-309

### Summary of the Invention

### Problems to be Solved by the Invention

The present invention aims to provide a compound useful as an agent for the prophylaxis or treatment of a lower urinary tract disease and the like.

### Means of Solving the Problems

The present inventors have conducted intensive studies in view of the above-mentioned situation and found that a compound represented by the formula

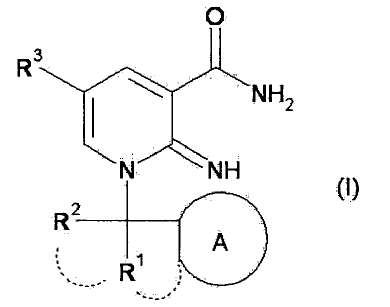

wherein
ring A is a phenyl group, a cycloalkyl group or a 5- or 6-membered aromatic heterocyclic group, each of which optionally has substituent(s),
R¹ is a methyl group, or R¹ and ring A in combination optionally form a fused cyclic group optionally having substituent(s),
R² is a hydrogen atom or a methyl group, or R¹ and R² in combination optionally form, together with the adjacent carbon atom, a cycloalkane ring, and
R³ is a hydrogen atom, a halogen atom, a cyano group, a hydrocarbon group optionally having substituent(s), an acyl group, a heterocyclic group optionally having substituent(s), an amino group optionally having substituent(s), a hydroxy group optionally having a substituent or a mercapto group optionally having a substituent (hereinafter to be abbreviated as compound (I)),
or a salt thereof has an α_{1D} adrenergic receptor antagonistic action based on its specific chemical structure. Based on the finding, they have completed the present invention.

Accordingly, the present invention relates to
[1] a compound represented by the formula

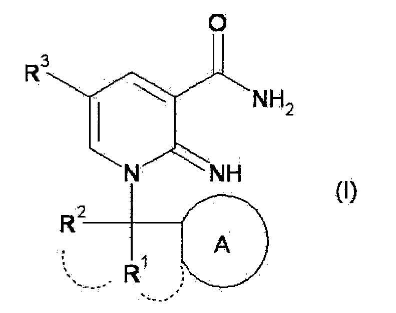

wherein
ring A is a phenyl group, a cycloalkyl group or a 5- or 6-membered aromatic heterocyclic group, each of which optionally has substituent(s),
R¹ is a methyl group, or R¹ and ring A in combination optionally form a fused cyclic group optionally having substituent(s),
R² is a hydrogen atom or a methyl group, or R¹ and R² in combination optionally form, together with the adjacent carbon atom, a cycloalkane ring, and
R³ is a hydrogen atom, a halogen atom, a cyano group, a hydrocarbon group optionally having substituent(s), an acyl group, a heterocyclic group optionally having substituent(s), an amino group optionally having substituent(s), a hydroxy group optionally having a substituent or a mercapto group optionally having a substituent,
provided that
5-chloro-1-(2-,3-dihydro-1H-inden-1-yl)-2-imino-1,2-dihydropyridine-3-carboxamide,
5-chloro-2-imino-1-(1-phenylethyl)-1,2-dihydropyridine-3-carboxamide and
5-chloro-2-imino-1-(1,2,3,4-tetrahydronaphthalen-1-yl)-1,2-dihydropyridine-3-carboxamide
are excluded,
or a salt thereof;
[2] the compound of the above-mentioned [1], wherein ring A is a phenyl group optionally having substituent(s);
[3] the compound of the above-mentioned [1], wherein R³ is a halogen atom;
[4] the compound of the above-mentioned [1], wherein the group represented by the partial structural formula of the formula (I)

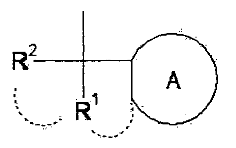

is a group represented by

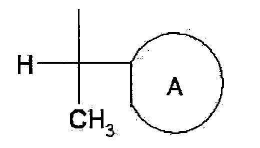

wherein ring A is as defined in the above-mentioned [1];
[5] the compound of the above-mentioned [4], wherein ring A is (1) a phenyl group having 1 to 3 substituents selected from (a) a halogen atom, (b) a cyano group and (c) an alkylsulfonyl group, (2) a pyridyl group optionally having substituent(s), or (3) a thienyl group optionally having substituent(s);
[6] the compound of the above-mentioned [4], wherein ring A is (1) a phenyl group having 1 to 3 substituents selected from (a) a halogen atom, (b) a cyano group and (c) a C₁₋₆ alkylsulfonyl group, (2) a pyridyl group, or (3) a thienyl group;
[7] the compound of the above-mentioned [4], wherein
   ring A is (1) a phenyl group having 1 to 3 substituents selected from (a) a halogen atom, (b) a cyano group and (c) a C₁₋₆ alkylsulfonyl group, (2) a pyridyl group, or (3) a thienyl group, and
   R³ is a halogen atom;
[8] the compound of the above-mentioned [4], wherein
   ring A is a phenyl group having 1 to 2 substituents selected from (a) a halogen atom and (b) a cyano group, and R³ is a halogen atom;
[9] the compound of the above-mentioned [1], wherein the group represented by the partial structural formula of the formula (I)

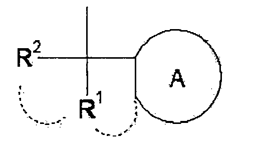

is a fused cyclic group represented by

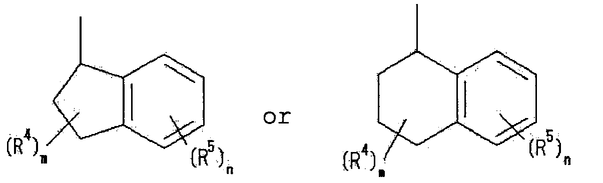

wherein
R⁴ and R⁵ are the same or different and each is a substituent selected from a hydroxy group, a halogen atom, a cyano group and -S(O)ₚ-R⁶ wherein R⁶ is an alkyl group, and p is an integer of 0 to 2,
m is an integer of 0 to 3, and
n is an integer of 0 to 4;
[10] the compound of the above-mentioned [9], wherein
   R⁴ is a hydroxy group,
   R⁵ is a substituent selected from a halogen atom, a cyano group and -S(O)ₚ-R⁶ wherein R⁶ is a C₁₋₆ alkyl group, and p is an integer of 0 to 2, and
   m+n=1 or 2, provided that m and n are the same or different and each is 0 or 1;
[11] the compound of the above-mentioned [9], wherein
   R³ is a halogen atom,
   R⁴ is a hydroxy group,
   R⁵ is a substituent selected from a halogen atom, a cyano group and -S(O)ₚ-R⁶ wherein R⁶ is a C₁₋₆ alkyl group, and p is an integer of 0 to 2, and
   m+n=1 or 2, provided that m and n are the same or different and each is 0 or 1;
[12] the compound of the above-mentioned [9], wherein
   R³ is a halogen atom,
   R⁵ is a halogen atom,
   m is 0, and
   n is 1;
[13] the compound of the above-mentioned [1], wherein the group represented by the partial structural formula of the formula (I)

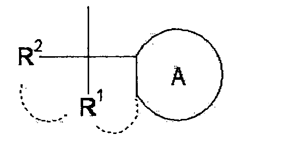

is a fused cyclic group represented by

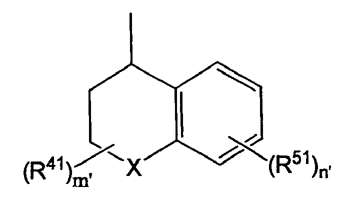

wherein R⁴¹ and R⁵¹ are the same or different and each is a substituent selected from a hydroxy group, a halogen atom, a cyano group and -S(O)ₚ,-R^{6l} wherein R⁶¹ is an alkyl group, and p' is an integer of 0 to 2,
X is S, SO or SO₂,
m' is an integer of 0 to 3, and
n' is an integer of 0 to 4;
[14] the compound of the above-mentioned [13], wherein
   R³ is a halogen atom,
   R⁵¹ is a halogen atom,
   m' is 0, and
   n' is 0 or 1;
[15] the compound of the above-mentioned [13], wherein
   R³ is a halogen atom,
   R⁵¹ is a halogen atom,
   X is SO₂,
   m' is 0, and
   n' is 1;
[16] the compound of the above-mentioned [1], wherein the group represented by the partial structural formula of the formula (I)

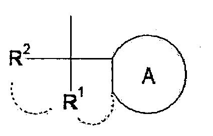

is a group represented by

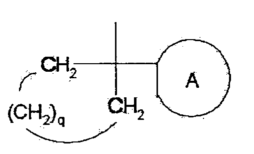

wherein q is an integer of 0 to 4, and ring A is as defined in the above-mentioned [1];
[17] 5-chloro-1-[1-(3-chlorophenyl)ethyl]-2-imino-1,2-dihydropyridine-3-carboxamide or a salt thereof;
[18] 5-chloro-1-(6-chloro-2,3-dihydro-1H-inden-1-yl)-2-imino-1,2-dihydropyridine-3-carboxamide or a salt thereof;
[19] 5-chloro-1-[(1R)-1-(3-fluorophenyl)ethyl]-2-imino-1,2-dihydropyridine-3-carboxamide or a salt thereof;
[20] 5-chloro-1-[(1R)-1-(3,5-difluorophenyl)ethyl]-2-imino-1,2-dihydropyridine-3-carboxamide or a salt thereof;
[21] 5-chloro-1-[(1R)-1-(3-cyanophenyl)ethyl]-2-imino-1,2-dihydropyridine-3-carboxamide or a salt thereof;
[22] 5-chloro-1-(6-chloro-1,1-dioxido-3,4-dihydro-2H-thiochromen-4-yl)-2-imino-1,2-dihydropyridine-3-carboxamide or a salt thereof;
[23] a prodrug of the compound of the above-mentioned [1];
[24] a pharmaceutical agent comprising the compound of the above-mentioned [1] or a prodrug thereof;
[25] the pharmaceutical agent of the above-mentioned [24], which is α_{1D} adrenoceptor antagonist;
[26] the pharmaceutical agent of the above-mentioned [24], which is an agent for the prophylaxis or treatment of lower urinary tract diseases;
[27] a method for the prophylaxis or treatment of lower urinary tract diseases in a mammal, which comprises administering an effective amount of the compound of the above-mentioned [1] or a prodrug thereof to the mammal;
[28] use of the compound of the above-mentioned [1] or a prodrug thereof for the production of an agent for the prophylaxis or treatment of lower urinary tract diseases;
and the like.

### Effect of the Invention

The compound (I) of the present invention has a superior selective α_{1D} adrenergic receptor antagonistic action, and is useful as an agent for the prophylaxis or treatment of a lower urinary tract disease and the like.

### [Detailed Description of the Invention]

The present invention is explained in detail in the following.
Unless otherwise specified, the "halogen atom" in the present specification means a fluorine atom, a chlorine atom, a bromine atom or an iodine atom.

In the formula (I), ring A is a phenyl group, a cycloalkyl group or a 5- or 6-membered aromatic heterocyclic group, each of which optionally has substituent(s).

Examples of the "cycloalkyl group" include a C₃₋₈ cycloalkyl group such as cyclopropyl, cyclobutyl, cyclopentyl, cyclohexyl and the like.

Examples of the "5- or 6-membered aromatic heterocyclic group" include a 5- or 6-membered aromatic heterocyclic group containing, besides carbon atoms, 1 to 4 hetero atoms selected from a nitrogen atom, a sulfur atom and an oxygen atom (e.g., furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl etc.).

Examples of the substituent that the "phenyl group, cycloalkyl group or 5- or 6-membered aromatic heterocyclic group" optionally has include
(1) a halogen atom (e.g., fluorine atom, chlorine atom, bromine atom, iodine atom etc.),
(2) nitro,
(3) cyano,
(4) hydroxy,
(5) C₁₋₆ alkoxy optionally having 1 to 3 halogen atoms (e.g., fluorine atom, chlorine atom, bromine atom, iodine atom) (e.g., methoxy, ethoxy, propoxy, isopropoxy, butoxy, isobutoxy, sec-butoxy, pentyloxy, hexyloxy, fluoromethoxy etc.),
(6) C₆₋₁₄ aryloxy (e.g., phenyloxy, naphthyloxy etc.),
(7) C₇₋₁₆ aralkyloxy (e.g., benzyloxy, phenethyloxy, diphenylmethyloxy, 1-naphthylmethyloxy, 2-naphthylmethyloxy, 2,2-diphenylethyloxy, 3-phenylpropyloxy, 4-phenylbutyloxy, 5-phenylpentyloxy etc.),
(8) mercapto,
(9) alkylthio (preferably C₁₋₆ alkylthio) optionally having 1 to 3 halogen atoms (e.g., fluorine atom, chlorine atom, bromine atom, iodine atom) (e.g., methylthio, difluoromethylthio, trifluoromethylthio, ethylthio, propylthio, isopropylthio, butylthio, 4,4,4-trifluorobutylthio, pentylthio, hexylthio etc.),
(10) C₆₋₁₄ arylthio (e.g., phenylthio, naphthylthio etc.),
(11) C₇₋₁₆ aralkylthio (e.g., benzylthio, phenethylthio, diphenylmethylthio, 1-naphthylmethylthio, 2-naphthylmethylthio, 2,2-diphenylethylthio, 3-phenylpropylthio, 4-phenylbutylthio, 5-phenylpentylthio etc.),
(12) amino,
(13) mono-C₁₋₆ alkylamino (e.g., methylamino, ethylamino etc.),
(14) mono-C₆₋₁₄ arylamino (e.g., phenylamino, 1-naphthylamino, 2-naphthylamino etc.),
(15) mono-C₇₋₁₆ aralkylamino (e.g., benzylamino etc.),
(16) di-C₁₋₆ alkylamino (e.g., dimethylamino, diethylamino etc.),
(17) di-C₆₋₁₄ arylamino (e.g., diphenylamino etc.),
(18) di-C₇₋₁₆ aralkylamino (e.g., dibenzylamino etc.),
(19) formyl,
(20) C₁₋₆ alkyl-carbonyl (e.g., acetyl, propionyl etc.),
(21) C₆₋₁₄ aryl-carbonyl (e.g., benzoyl, 1-naphthoyl, 2-naphthoyl etc.),
(22) carboxy,
(23) C₁₋₆ alkoxy-carbonyl (e.g., methoxycarbonyl, ethoxycarbonyl, propoxycarbonyl, tert-butoxycarbonyl etc.),
(24) C₆₋₁₄ aryloxy-carbonyl (e.g., phenoxycarbonyl etc.),
(25) carbamoyl,
(26) thiocarbamoyl,
(27) mono-C₁₋₆ alkyl-carbamoyl (e.g., methylcarbamoyl, ethylcarbamoyl etc.),
(28) di-C₁₋₆ alkyl-carbamoyl (e.g., dimethylcarbamoyl, diethylcarbamoyl, ethylmethylcarbamoyl etc.),
(29) C₆₋₁₄ aryl-carbamoyl (e.g., phenylcarbamoyl, 1-naphthylcarbamoyl, 2-naphthylcarbamoyl etc.),
(30) alkylsulfonyl (preferably C₁₋₆ alkylsulfonyl) optionally having 1 to 3 halogen atoms (e.g., fluorine atom, chlorine atom, bromine atom, iodine atom) (e.g., methylsulfonyl, ethylsulfonyl, trifluoromethylsulfonyl etc.),
(31) C₆₋₁₄ arylsulfonyl (e.g., phenylsulfonyl, 1-naphthylsulfonyl, 2-naphthylsulfonyl etc.),
(32) alkylsulfinyl (preferably C₁₋₆ alkylsulfinyl) (e.g., methylsulfinyl, ethylsulfinyl etc.),
(33) C₆₋₁₄ arylsulfinyl (e.g., phenylsulfinyl, 1-naphthylsulfinyl, 2-naphthylsulfinyl etc.),
(34) formylamino,
(35) C₁₋₆ alkyl-carbonylamino (e.g., acetylamino etc.),
(36) C₆₋₁₄ aryl-carbonylamino (e.g., benzoylamino, naphthoylamino etc.),
(37) C₁₋₆ alkoxy-carbonylamino (e.g., methoxycarbonylamino, ethoxycarbonylamino, propoxycarbonylamino, butoxycarbonylamino etc.),
(38) C₁₋₆ alkylsulfonylamino (e.g., methylsulfonylamino, ethylsulfonylamino etc.),
(39) C₆₋₁₄ arylsulfonylamino (e.g., phenylsulfonylamino, 2-naphthylsulfonylamino, 1-naphthylsulfonylamino etc.),
(40) C₁₋₆ alkyl-carbonyloxy (e.g., acetoxy, propionyloxy etc.),
(41) C₆₋₁₄ aryl-carbonyloxy (e.g., benzoyloxy, naphthylcarbonyloxy etc.),
(42) C₁₋₆ alkoxy-carbonyloxy (e.g., methoxycarbonyloxy, ethoxycarbonyloxy, propoxycarbonyloxy, butoxycarbonyloxy etc.),
(43) mono-C₁₋₆ alkyl-carbamoyloxy (e.g., methylcarbamoyloxy, ethylcarbamoyloxy etc.),
(44) di-C₁₋₆ alkyl-carbamoyloxy (e.g., dimethylcarbamoyloxy, diethylcarbamoyloxy etc.),
(45) C₆₋₁₄ aryl-carbamoyloxy (e.g., phenylcarbamoyloxy, naphthylcarbamoyloxy etc.),
(46) 5- to 7-membered saturated cyclic amino optionally containing, besides carbon atoms and one nitrogen atom, one or two kinds of 1 to 4 hetero atoms selected from a nitrogen atom, a sulfur atom and an oxygen atom (e.g., pyrrolidin-1-yl, piperidino, piperazin-1-yl, morpholino, thiomorpholino, hexahydroazepin-1-yl etc.),
(47) a 5- to 10-membered aromatic heterocyclic group containing, besides carbon atoms, one or two kinds of 1 to 4 hetero atoms selected from a nitrogen atom, a sulfur atom and an oxygen atom (e.g., 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl etc.),
(48) C₁₋₃ alkylenedioxy (e.g., methylenedioxy, ethylenedioxy etc.),
(49) C₃₋₇ cycloalkyl (e.g., cyclopropyl, cyclobutyl, cyclopentyl, cyclohexyl, cycloheptyl etc.),
(50) C₁₋₆ alkyl (e.g., methyl, ethyl, n-propyl, isopropyl, n-butyl, isobutyl, sec-butyl, tert-butyl, n-pentyl, sec-pentyl, isopentyl, neopentyl, n-hexyl, isohexyl etc.) optionally having 1 to 3 substituents selected from a halogen atom (e.g., fluorine atom, chlorine atom, bromine atom, iodine atom) and hydroxy,
(51) C₂₋₆ alkenyl (e.g., allyl, isopropenyl, isobutenyl, 1-methylallyl, 2-pentenyl, 2-hexenyl etc.) optionally having 1 to 3 halogen atoms (e.g., fluorine atom, chlorine atom, bromine atom, iodine atom),
(52) C₂₋₆ alkynyl (e.g., propargyl, 2-butynyl, 3-butynyl, 3-pentynyl, 3-hexynyl etc.),
(53) mono-C₃₋₇ cycloalkyl-carbamoyl (e.g., cyclopropylcarbamoyl, cyclobutylcarbamoyl etc.),
(54) 5- to 10-membered heterocyclyl-carbonyl containing, besides carbon atoms, one or two kinds of 1 to 4 hetero atoms selected from a nitrogen atom, a sulfur atom and an oxygen atom (e.g., 4-morpholinocarbonyl etc.),
and the like. The number of substituent is 1 to 5 (preferably 1 to 3).

The substituent is preferably a hydroxy group, a halogen atom, a cyano group and -S(O)ₚ-R⁶ wherein R⁶ is an alkyl group, and p is an integer of 0 to 2, more preferably a hydroxy group, a halogen atom, a cyano group and -S(O)ₚ-R⁶ wherein R⁶ is a C₁₋₆ alkyl group, and p is an integer of 0 to 2, still more preferably a halogen atom, a cyano group and -S(O)ₚ-R⁶ wherein R⁶ is a C₁₋₆ alkyl group, and p is an integer of 0 to 2, particularly preferably a halogen atom and a cyano group, most preferably a halogen atom.

The "alkyl group" for R⁶ is preferably a C₁₋₆ alkyl group such as methyl, ethyl, n-propyl, isopropyl, n-butyl, isobutyl, sec-butyl, tert-butyl, n-pentyl, sec-pentyl, isopentyl, neopentyl, n-hexyl, isohexyl and the like, particularly preferably methyl.

Ring A is preferably a phenyl group, a C₃₋₆ cycloalkyl group or a 5- or 6-membered aromatic heterocyclic group (preferably furyl group, a thienyl group, a pyridyl group), each of which optionally has substituent(s), more preferably a phenyl group, a thienyl group or a pyridyl group, each of which optionally has substituent(s), particularly preferably a phenyl group optionally having substituent(s).

Specifically, ring A is
preferably a phenyl group, a C₃₋₆ cycloalkyl group or a 5- or 6-membered aromatic heterocyclic group (preferably a furyl group, a thienyl group, a pyridyl group) (preferably a phenyl group, a thienyl group, a pyridyl group, particularly preferably a phenyl group), each of which optionally has 1 to 3 substituents selected from a hydroxy group, a halogen atom, a cyano group and -S(O)ₚ-R⁶ wherein R⁶ is an alkyl group, and p is an integer of 0 to 2,
more preferably a phenyl group, a C₃₋₆ cycloalkyl group or a 5-or 6-membered aromatic heterocyclic group (preferably a furyl group, a thienyl group, a pyridyl group) (preferably a phenyl group, a thienyl group, a pyridyl group, particularly preferably a phenyl group), each of which optionally has 1 to 3 substituents selected from a hydroxy group, a halogen atom, a cyano group and -S(O)ₚ-R⁶ wherein R⁶ is a C₁₋₆ alkyl group, and p is an integer of 0 to 2,
still more preferably a phenyl group, a C₃₋₆ cycloalkyl group or a 5- or 6-membered aromatic heterocyclic group (preferably furyl group, a thienyl group, a pyridyl group) (preferably a phenyl group, a thienyl group, a pyridyl group, particularly preferably a phenyl group), each of which optionally has 1 to 3 substituents selected from a halogen atom, a cyano group and -S(O)ₚ-R⁶ wherein R⁶ is a C₁₋₆ alkyl group, and p is an integer of 0 to 2,
particularly preferably a phenyl group, a C₃₋₆ cycloalkyl group or a 5- or 6-membered aromatic heterocyclic group (preferably furyl group, a thienyl group, a pyridyl group) (preferably a phenyl group, a thienyl group, a pyridyl group, particularly preferably a phenyl group), each of which optionally has 1 to 3 (preferably 1 or 2) substituents selected from a halogen atom and a cyano group,
most preferably a phenyl group, a C₃₋₆ cycloalkyl group or a 5-or 6-membered aromatic heterocyclic group (preferably furyl group, a thienyl group, a pyridyl group) (preferably a phenyl group, a thienyl group, a pyridyl group, particularly preferably a phenyl group), each of which optionally has 1 to 3 (preferably 1) halogen atoms.

In another embodiment, ring A is
preferably (1) a phenyl group having 1 to 3 substituents selected from (a) a halogen atom, (b) a cyano group and (c) an alkylsulfonyl group, (2) a pyridyl group optionally having substituent(s), or (3) a thienyl group optionally having substituent(s),
more preferably (1) a phenyl group having 1 to 3 substituents selected from (a) a halogen atom, (b) a cyano group and (c) a C₁₋₆ alkylsulfonyl group, (2) a pyridyl group, or (3) a thienyl group,
particularly preferably a phenyl group having 1 to 2 substituents selected from (a) a halogen atom and (b) a cyano group.

R¹ is a methyl group, or R¹ and ring A in combination optionally form a fused cyclic group optionally having substituent(s)
When R¹ is a methyl group, preferable embodiment of the group represented by the partial structural formula:

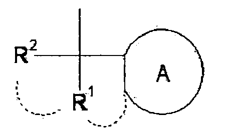

is a group represented by

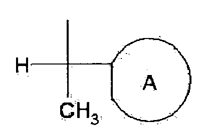

wherein ring A is as defined above,
(provided that R² is a hydrogen atom).
In this embodiment, ring A is
preferably a phenyl group, a C₃₋₆ cycloalkyl group or a 5- or 6-membered aromatic heterocyclic group (preferably a furyl group, a thienyl group, a pyridyl group) (preferably a phenyl group,
a thienyl group, a pyridyl group, particularly preferably a phenyl group), each of which optionally has 1 to 3 substituents selected from a hydroxy group, a halogen atom, a cyano group and -S(O)ₚ-R⁶ wherein R⁶ is an alkyl group, and p is an integer of 0 to 2,
more preferably a phenyl group, a C₃₋₆ cycloalkyl group or a 5-or 6-membered aromatic heterocyclic group (preferably a furyl group, a thienyl group, a pyridyl group) (preferably a phenyl group, a thienyl group, a pyridyl group, particularly preferably a phenyl group), each of which optionally has 1 to 3 substituents selected from a hydroxy group, a halogen atom, a cyano group and -S(O)ₚ-R⁶ wherein R⁶ is a C₁₋₆ alkyl group, and p is an integer of 0 to 2,
still more preferably a phenyl group, a C₃₋₆ cycloalkyl group or a 5- or 6-membered aromatic heterocyclic group (preferably furyl group, a thienyl group, a pyridyl group) (preferably a phenyl group, a thienyl group, a pyridyl group, particularly preferably a phenyl group), each of which optionally has 1 to 3 substituents selected from a halogen atom, a cyano group and -S(O)ₚ-R⁶ wherein R⁶ is a C₁₋₆ alkyl group, and p is an integer of 0 to 2,
particularly preferably a phenyl group, a C₃₋₆ cycloalkyl group or a 5- or 6-membered aromatic heterocyclic group (preferably furyl group, a thienyl group, a pyridyl group) (preferably a phenyl group, a thienyl group, a pyridyl group, particularly preferably a phenyl group), each of which optionally has 1 to 3 (preferably 1 or 2) substituents selected from a halogen atom and a cyano group,
most preferably a phenyl group, a C₃₋₆ cycloalkyl group or a 5-or 6-membered aromatic heterocyclic group (preferably furyl group, a thienyl group, a pyridyl group) (preferably a phenyl group, a thienyl group, a pyridyl group, particularly preferably a phenyl group), each of which optionally has 1 to 3 (preferably 1) halogen atoms.

In another embodiment, ring A is
preferably (1) a phenyl group having 1 to 3 substituents selected from (a) a halogen atom, (b) a cyano group and (c) an alkylsulfonyl group, (2) a pyridyl group optionally having substituent(s), or (3) a thienyl group optionally having substituent(s),
more preferably (1) a phenyl group having 1 to 3 substituents selected from (a) a halogen atom, (b) a cyano group and (c) a C₁₋₆ alkylsulfonyl group, (2) a pyridyl group, or (3) a thienyl group,
particularly preferably a phenyl group having 1 to 2 substituents selected from (a) a halogen atom and (b) a cyano group.

When R¹ and ring A in combination optionally form a fused cyclic group optionally having substituent(s), preferable embodiment of the group represented by the partial structural formula:

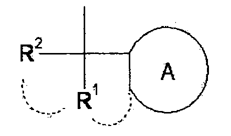

is a fused cyclic group selected from groups represented by

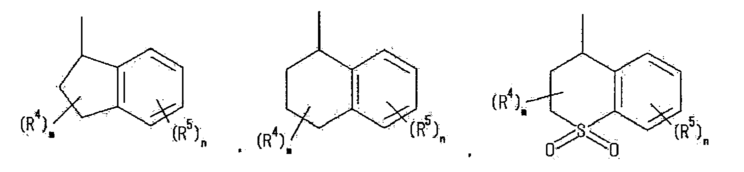

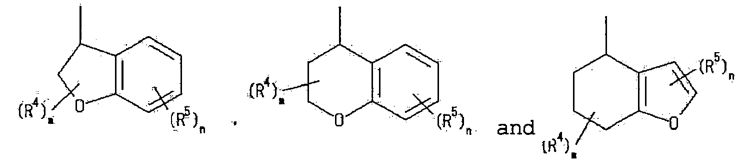

R⁴ and R⁵ are the same or different and each is a substituent selected from a hydroxy group, a halogen atom, a cyano group and -S(O)ₚ-R⁶ wherein R⁶ is an alkyl group, and p is an integer of 0 to 2,
m is an integer of 0 to 3, and
n is an integer of 0 to 4
(provided that R² is a hydrogen atom).
When m is an integer of 2 or more (i.e., the fused cyclic group has 2 or more R⁴ at substitutable positions), each of R⁴ may be the same or different.
In addition, when n is an integer of 2 or more (i.e., the fused cyclic group has 2 or more R⁵ at substitutable positions), each of R⁵ may be the same or different.
R⁴ is a substituent presented on the ring bonded to the pyridine ring, and R⁵ is a substituent presented on the other ring.

Of the above-mentioned fused cyclic group, a fused cyclic group represented by

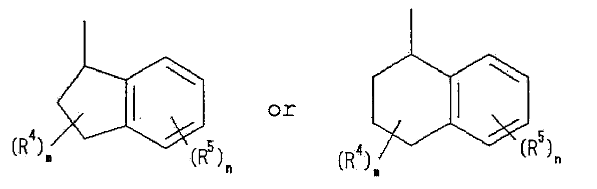

wherein the symbols in the formula are as defined above, is preferable.

In this embodiment, preferable embodiment is shown in the following.
R⁴ and R⁵ are preferably the same or different and each is a substituent selected from a hydroxy group, a halogen atom, a cyano group and -S(O)ₚ-R⁶ wherein R⁶ is a C₁₋₆ alkyl group, and p is an integer of 0 to 2.
R⁴ is more preferably a hydroxy group.
R⁵ is more preferably a substituent selected from a halogen atom, a cyano group and -S(O)ₚ-R⁵ wherein R⁶ is a C₁₋₆ alkyl group, and p is an integer of 0 to 2, particularly preferably a halogen atom.
As preferable m and n, m+n=1 or 2 (provided that m and n are the same or different and each is 0 or 1), preferably m is 0, and n is 1.

Alternatively, when R¹ and ring A in combination optionally form a fused cyclic group optionally having substituent(s), another preferable embodiment of the group represented by the partial structural formula:

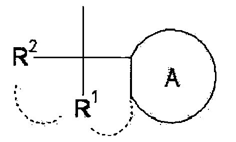

is a fused cyclic group represented by

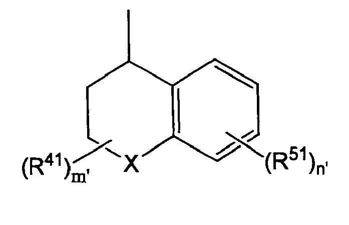

wherein R⁴¹ and R⁵¹ are the same or different and each is a substituent selected from a hydroxy group, a halogen atom, a cyano group and -S(O)_{p'}-R⁶¹ wherein R⁶¹ is an alkyl group, and p' is an integer of 0 to 2,
X is S, SO or SO₂,
m' is an integer of 0 to 3, and
n' is an integer of 0 to 4;
(provided that R² is a hydrogen atom).

Examples of the "alkyl group" for R⁶¹ include those similar to the "alkyl group" for R⁶. Of these, a C₁₋₆ alkyl group is preferable, and methyl is particularly preferable.

In this embodiment, preferable embodiment is shown in the following.
X is preferably SO₂.
R⁴¹ and R⁵¹ are preferably the same or different and each is a substituent selected from a hydroxy group, a halogen atom, a cyano group and -S(O)_{p'}-R⁶¹ wherein R⁶¹ is a C₁₋₆ alkyl group, and p' is an integer of 0 to 2.
R⁴¹ is more preferably a hydroxy group.
R⁵¹ is more preferably a substituent selected from a halogen atom, a cyano group and -S(O)_{p'}-R⁶¹ wherein R⁶¹ is a C₁₋₆ alkyl group, and p' is an integer of 0 to 2, particularly preferably a halogen atom.
As preferable m' and n', m'+n'=1 or 2 (provided that m' and n' are the same or different and each is 0 or 1), preferably m' is 0, and n' is 0 or 1 (preferably 1).

R² is a hydrogen atom or a methyl group, or R¹ and R² in combination optionally form, together with the adjacent carbon atom, a cycloalkane ring.
Examples of the cycloalkane ring include a C₃₋₇ cycloalkane ring (e.g., cyclopropyl, cyclobutyl, cyclopentyl, cyclohexyl, cycloheptyl etc.) and the like. In this case, the group represented by the partial structural formula:

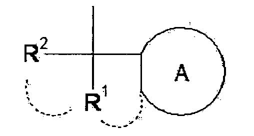

is represented by

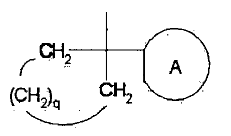

wherein q is an integer of 0 to 4, and ring A is as defined above.

In this embodiment, ring A is
preferably a phenyl group, a C₃₋₆ cycloalkyl group or a 5- or 6-membered aromatic heterocyclic group (preferably a furyl group, a thienyl group, a pyridyl group) (preferably a phenyl group, a thienyl group, a pyridyl group, particularly preferably a phenyl group), each of which optionally has 1 to 3 substituents selected from a hydroxy group, a halogen atom, a cyano group and -S(O)ₚ-R⁶ wherein R⁶ is an alkyl group, and p is an integer of 0 to 2,
more preferably a phenyl group, a C₃₋₆ cycloalkyl group or a 5-or 6-membered aromatic heterocyclic group (preferably a furyl group, a thienyl group, a pyridyl group) (preferably a phenyl group, a thienyl group, a pyridyl group, particularly preferably a phenyl group), each of which optionally has 1 to 3 substituents selected from a hydroxy group, a halogen atom, a cyano group and -S(O)ₚ-R⁶ wherein R⁶ is a C₁₋₆ alkyl group, and p is an integer of 0 to 2,
still more preferably a phenyl group, a C₃₋₆ cycloalkyl group or a 5- or 6-membered aromatic heterocyclic group (preferably furyl group, a thienyl group, a pyridyl group) (preferably a phenyl group, a thienyl group, a pyridyl group, particularly preferably a phenyl group), each of which optionally has 1 to 3 substituents selected from a halogen atom, a cyano group and -S(O)ₚ-R⁶ wherein R⁶ is a C₁₋₆ alkyl group, and p is an integer of 0 to 2,

When R¹ and R² in combination optionally form, together with the adjacent carbon atom, a cycloalkane ring, R¹ and ring A in combination do not preferably form a fused cyclic group optionally having substituent(s),
q is preferably an integer of 0 to 3, particularly preferably an integer of 0 to 2.

R³ is a hydrogen atom, a halogen atom, a cyano group, a hydrocarbon group optionally having substituent(s), an acyl group, a heterocyclic group optionally having substituent(s), an amino group optionally having substituent(s), a hydroxy group optionally having a substituent or a mercapto group optionally having a substituent.

Examples of the halogen atom for R³ include a fluorine atom, a chlorine atom, a bromine atom and an iodine atom.

Preferable examples of the "hydrocarbon group" of the "hydrocarbon group optionally having substituent(s)" for R³ include a chain or cyclic hydrocarbon group (e.g., alkyl, alkenyl, alkynyl, cycloalkyl, aryl, aralkyl etc.). Of these, a chain or cyclic hydrocarbon group having 1 to 16 carbon atoms and the like.
Examples of the above-mentioned alkyl include C₁₋₆ alkyl (e.g., methyl, ethyl, propyl, isopropyl, butyl, isobutyl, sec-butyl, tert-butyl, pentyl, hexyl etc.) and the like.
Examples of the above-mentioned alkenyl include C₂₋₆ alkenyl (e.g., vinyl, allyl, isopropenyl, 1-butenyl, 2-butenyl, 3-butenyl, 2-methyl-2-propenyl, 1-methyl-2-propenyl, 2-methyl-1-propenyl etc.) and the like.
Examples of the above-mentioned alkynyl include C₂₋₆ alkynyl (e.g., ethynyl, propargyl, 1-butynyl, 2-butynyl, 3-butynyl, 1-hexynyl etc.) and the like.
Examples of the above-mentioned cycloalkyl include C₃₋₇ cycloalkyl (e.g., cyclopropyl, cyclobutyl, cyclopentyl, cyclohexyl, cycloheptyl etc.) and the like.
Examples of the above-mentioned aryl include C₆₋₁₄ aryl (e.g., phenyl, 1-naphthyl, 2-naphthyl, 2-biphenylyl, 3-biphenylyl, 4-biphenylyl, 2-anthryl etc.) and the like.
Examples of the above-mentioned aralkyl include C₇₋₁₆ aralkyl (e.g., phenyl-C₁₋₆ alkyl such as benzyl, phenethyl, 3-phenylpropyl, 4-phenylbutyl, 5-phenylpentyl and the like; naphthyl-C₁₋₆ alkyl such as 1-naphthylmethyl, 2-naphthylmethyl and the like; diphenyl-C₁₋₄ alkyl such as diphenylmethyl, 2,2-diphenylethyl and the like etc.) and the like.

When the "hydrocarbon group" is alkyl, alkenyl or alkynyl, it is optionally substituted by 1 to 3 substituents selected from
(1) a halogen atom (e.g., a fluorine atom, a chlorine atom, a bromine atom, an iodine atom etc.),
(2) nitro,
(3) cyano,
(4) hydroxy,
(5) C₁₋₆ alkoxy optionally having 1 to 3 halogen atoms (e.g., a fluorine atom, a chlorine atom, a bromine atom, an iodine atom) (e.g., methoxy, ethoxy, propoxy, isopropoxy, butoxy, isobutoxy, sec-butoxy, pentyloxy, hexyloxy, fluoromethoxy etc.),
(6) C₆₋₁₄ aryloxy (e.g., phenyloxy, naphthyloxy etc.),
(7) C₇₋₁₆ aralkyloxy (e.g., benzyloxy, phenethyloxy, diphenylmethyloxy, 1-naphthylmethyloxy, 2-naphthylmethyloxy, 2,2-diphenylethyloxy, 3-phenylpropyloxy, 4-phenylbutyloxy, 5-phenylpentyloxy etc.),
(8) mercapto,
(9) C₁₋₆ alkylthio optionally having 1 to 3 halogen atoms (e.g., a fluorine atom, a chlorine atom, a bromine atom, an iodine atom) (e.g., methylthio, difluoromethylthio, trifluoromethylthio, ethylthio, propylthio, isopropylthio, butylthio, 4,4,4-trifluorobutylthio, pentylthio, hexylthio etc.),
(10) C₆₋₁₄ arylthio (e.g., phenylthio, naphthylthio etc.),
(11) C₇₋₁₆ aralkylthio (e.g., benzylthio, phenethylthio, diphenylmethylthio, 1-naphthylmethylthio, 2-naphthylmethylthio, 2,2-diphenylethylthio, 3-phenylpropylthio, 4-phenylbutylthio, 5-phenylpentylthio etc.),
(12) amino,
(13) mono-C₁₋₆ alkylamino (e.g., methylamino, ethylamino etc.),
(14) mono-C₆₋₁₄ arylamino (e.g., phenylamino, 1-naphthylamino, 2-naphthylamino etc.),
(15) mono-C₇₋₁₆ aralkylamino (e.g., benzylamino etc.),
(16) di-C₁₋₆ alkylamino (e.g., dimethylamino, diethylamino etc.),
(17) di-C₆₋₁₄ arylamino (e.g., diphenylamino etc.),
(18) di-C₇₋₁₆ aralkylamino (e.g., dibenzylamino etc.),
(19) formyl,
(20) C₁₋₆ alkyl-carbonyl (e.g., acetyl, propionyl etc.),
(21) C₆₋₁₄ aryl-carbonyl (e.g., benzoyl, 1-naphthoyl, 2-naphthoyl etc.),
(22) carboxy,
(23) C₁₋₆ alkoxy-carbonyl (e.g., methoxycarbonyl, ethoxycarbonyl, propoxycarbonyl, tert-butoxycarbonyl etc.),
(24) C₆₋₁₄ aryloxy-carbonyl (e.g., phenoxycarbonyl etc.),
(25) carbamoyl,
(26) thiocarbamoyl,
(27) mono-C₁₋₆ alkyl-carbamoyl (e.g., methylcarbamoyl, ethylcarbamoyl etc.),
(28) di-C₁₋₆ alkyl-carbamoyl (e.g., dimethylcarbamoyl, diethylcarbamoyl, ethylmethylcarbamoyl etc.),
(29) C₆₋₁₄ aryl-carbamoyl (e.g., phenylcarbamoyl, 1-naphthylcarbamoyl, 2-naphthylcarbamoyl etc.),
(30) C₁₋₆ alkylsulfonyl (e.g., methylsulfonyl, ethylsulfonyl etc.),
(31) C₆₋₁₄ arylsulfonyl (e.g., phenylsulfonyl, 1-naphthylsulfonyl, 2-naphthylsulfonyl etc.),
(32) C₁₋₆ alkylsulfinyl (e.g., methylsulfinyl, ethylsulfinyl etc.),
(33) C₆₋₁₄ arylsulfinyl (e.g., phenylsulfinyl, 1-naphthylsulfinyl, 2-naphthylsulfinyl etc.),
(34) formylamino,
(35) C₁₋₆ alkyl-carbonylamino (e.g., acetylamino etc.),
(36) C₆₋₁₄ aryl-carbonylamino (e.g., benzoylamino, naphthoylamino etc.),
(37) C₁₋₆ alkoxy-carbonylamino (e.g., methoxycarbonylamino, ethoxycarbonylamino, propoxycarbonylamino, butoxycarbonylamino etc.),
(38) C₁₋₆ alkylsulfonylamino (e.g., methylsulfonylamino, ethylsulfonylamino etc.),
(39) C₆₋₁₄ arylsulfonylamino (e.g., phenylsulfonylamino, 2-naphthylsulfonylamino, 1-naphthylsulfonylamino etc.),
(40) C₁₋₆ alkyl-carbonyloxy (e.g., acetoxy, propionyloxy etc.),
(41) C₆₋₁₄ aryl-carbonyloxy (e.g., benzoyloxy, naphthylcarbonyloxy etc.),
(42) C₁₋₆ alkoxy-carbonyloxy (e.g., methoxycarbonyloxy, ethoxycarbonyloxy, propoxycarbonyloxy, butoxycarbonyloxy etc.),
(43) mono-C₁₋₆ alkyl-carbamoyloxy (e.g., methylcarbamoyloxy, ethylcarbamoyloxy etc.),
(44) di-C₁₋₆ alkyl-carbamoyloxy (e.g., dimethylcarbamoyloxy, diethylcarbamoyloxy etc.),
(45) C₆₋₁₄ aryl-carbamoyloxy (e.g., phenylcarbamoyloxy, naphthylcarbamoyloxy etc.),
(46) 5- to 7-membered saturated cyclic amino optionally containing, besides carbon atoms and one nitrogen atom, one or two kinds of 1 to 4 hetero atoms selected from a nitrogen atom, a sulfur atom and an oxygen atom (e.g., pyrrolidin-1-yl, piperidino, piperazin-1-yl, morpholino, thiomorpholino, hexahydroazepin-1-yl etc.),
(47) a 5- to 10-membered aromatic heterocyclic group containing, besides carbon atoms, one or two kinds of 1 to 4 hetero atoms selected from a nitrogen atom, a sulfur atom and an oxygen atom (e.g., 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl etc.),
(48) C₁₋₃ alkylenedioxy (e.g., methylenedioxy, ethylenedioxy etc.),
(49) C₃₋₇ cycloalkyl (e.g., cyclopropyl, cyclobutyl, cyclopentyl, cyclohexyl, cycloheptyl etc.)
and the like.

In addition, when the above-mentioned "hydrocarbon group" is cycloalkyl, aryl or aralkyl, it is optionally substituted by 1 to 5 (preferably 1 to 3) substituents selected from
(1) a halogen atom (e.g., a fluorine atom, a chlorine atom, a bromine atom, an iodine atom etc.),
(2) nitro,
(3) cyano,
(4) hydroxy,
(5) C₁₋₆ alkoxy optionally having 1 to 3 halogen atoms (e.g., a fluorine atom, a chlorine atom, a bromine atom, an iodine atom) (e.g., methoxy, ethoxy, propoxy, isopropoxy, butoxy, isobutoxy, sec-butoxy, pentyloxy, hexyloxy, fluoromethoxy etc.),
(6) C₆₋₁₄ aryloxy (e.g., phenyloxy, naphthyloxy etc.),
(7) C₇₋₁₆ aralkyloxy (e.g., benzyloxy, phenethyloxy, diphenylmethyloxy, 1-naphthylmethyloxy, 2-naphthylmethyloxy, 2,2-diphenylethyloxy, 3-phenylpropyloxy, 4-phenylbutyloxy, 5-phenylpentyloxy etc.),
(8) mercapto,
(9) C₁₋₆ alkylthio optionally having 1 to 3 halogen atoms (e.g., a fluorine atom, a chlorine atom, a bromine atom, an iodine atom) (e.g., methylthio, difluoromethylthio, trifluoromethylthio, ethylthio, propylthio, isopropylthio, butylthio, 4,4,4-trifluorobutylthio, pentylthio, hexylthio etc.),
(10) C₆₋₁₄ arylthio (e.g., phenylthio, naphthylthio etc.),
(11) C₇₋₁₆ aralkylthio (e.g., benzylthio, phenethylthio, diphenylmethylthio, 1-naphthylmethylthio, 2-naphthylmethylthio, 2,2-diphenylethylthio, 3-phenylpropylthio, 4-phenylbutylthio, 5-phenylpentylthio etc.),
(12) amino,
(13) mono-C₁₋₆ alkylamino (e.g., methylamino, ethylamino etc.),
(14) mono-C₆₋₁₄ arylamino (e.g., phenylamino, 1-naphthylamino, 2-naphthylamino etc.),
(15) mono-C₇₋₁₆ aralkylamino (e.g., benzylamino etc.),
(16) di-C₁₋₆ alkylamino (e.g., dimethylamino, diethylamino etc.),
(17) di-C₆₋₁₄ arylamino (e.g., d-iphenylamino etc.),
(18) di-C₇₋₁₆ aralkylamino (e.g., dibenzylamino etc.),
(19) formyl,
(20) C₁₋₆ alkyl-carbonyl (e.g., acetyl, propionyl etc.),
(21) C₆₋₁₄ aryl-carbonyl (e.g., benzoyl, 1-naphthoyl, 2-naphthoyl etc.),
(22) carboxy,
(23) C₁₋₆ alkoxy-carbonyl (e.g., methoxycarbonyl, ethoxycarbonyl, propoxycarbonyl, tert-butoxycarbonyl etc.),
(24) C₆₋₁₄ aryloxy-carbonyl (e.g., phenoxycarbonyl etc.),
(25) carbamoyl,
(26) thiocarbamoyl,
(27) mono-C₁₋₆ alkyl-carbamoyl (e.g., methylcarbamoyl, ethylcarbamoyl etc.),
(28) di-C₁₋₆ alkyl-carbamoyl (e.g., dimethylcarbamoyl, diethylcarbamoyl, ethylmethylcarbamoyl etc.),
(29) C₆₋₁₄ aryl-carbamoyl (e.g., phenylcarbamoyl, 1-naphthylcarbamoyl, 2-naphthylcarbamoyl etc.),
(30) C₁₋₆ alkylsulfonyl optionally having 1 to 3 halogen atoms (e.g., a fluorine atom, a chlorine atom, a bromine atom, an iodine atom) (e.g., methylsulfonyl, ethylsulfonyl, trifluoromethylsulfonyl etc.),
(31) C₆₋₁₄ arylsulfonyl (e.g., phenylsulfonyl, 1-naphthylsulfonyl, 2-naphthylsulfonyl etc.),
(32) C₁₋₆ alkylsulfinyl (e.g., methylsulfinyl, ethylsulfinyl etc.),
(33) C₆₋₁₄ arylsulfinyl (e.g., phenylsulfinyl, 1-naphthylsulfinyl, 2-naphthylsulfinyl etc.),
(34) formylamino,
(35) C₁₋₆ alkyl-carbonylamino (e.g., acetylamino etc.),
(36) C₆₋₁₄ aryl-carbonylamino (e.g., benzoylamino, naphthoylamino etc.),
(37) C₁₋₆ alkoxy-carbonylamino (e.g., methoxycarbonylamino, ethoxycarbonylamino, propoxycarbonylamino, butoxycarbonylamino etc.),
(38) C₁₋₆ alkylsulfonylamino (e.g., methylsulfonylamino, ethylsulfonylamino etc.),
(39) C₆₋₁₄ arylsulfonylamino (e.g., phenylsulfonylamino, 2-naphthylsulfonylamino, 1-naphthylsulfonylamino etc.),
(40) C₁₋₆ alkyl-carbonyloxy (e.g., acetoxy, propionyloxy etc.),
(41) C₆₋₁₄ aryl-carbonyloxy (e.g., benzoyloxy, naphthylcarbonyloxy etc.),
(42) C₁₋₆ alkoxy-carbonyloxy (e.g., methoxycarbonyloxy, ethoxycarbonyloxy, propoxycarbonyloxy, butoxycarbonyloxy etc.),
(43) mono-C₁₋₆ alkyl-carbamoyloxy (e.g., methylcarbamoyloxy, ethylcarbamoyloxy etc.),
(44) di-C₁₋₆ alkyl-carbamoyloxy (e.g., dimethylcarbamoyloxy, diethylcarbamoyloxy etc.),
(45) C₆₋₁₄ aryl-carbamoyloxy (e.g., phenylcarbamoyloxy, naphthylcarbamoyloxy etc.),
(46) 5- to 7-membered saturated cyclic amino optionally containing, besides carbon atoms and one nitrogen atom, one or two kinds of 1 to 4 hetero atoms selected from a nitrogen atom, a sulfur atom and an oxygen atom (e.g., pyrrolidin-1-yl, piperidino, piperazin-1-yl, morpholino, thiomorpholino, hexahydroazepin-1-yl etc.),
(47) a 5- to 10-membered aromatic heterocyclic group containing, besides carbon atoms, one or two kinds of 1 to 4 hetero atoms selected from a nitrogen atom, a sulfur atom and an oxygen atom (e.g., 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl etc.),
(48) C₁₋₃ alkylenedioxy (e.g., methylenedioxy, ethylenedioxy etc.),
(49) C₃₋₇ cycloalkyl (e.g., cyclopropyl, cyclobutyl, cyclopentyl, cyclohexyl, cycloheptyl etc.),
(50) C₁₋₆ alkyl (e.g., methyl, ethyl, n-propyl, isopropyl, n-butyl, isobutyl, sec-butyl, tert-butyl, n-pentyl, sec-pentyl, isopentyl, neopentyl, n-hexyl, isohexyl etc.) optionally having 1 to 3 substituents selected from 1 to 3 halogen atoms (e.g., a fluorine atom, a chlorine atom, a bromine atom, an iodine atom) and hydroxy,
(51) C₂₋₆ alkenyl (e.g., allyl, isopropenyl, isobutenyl, 1-methylallyl, 2-pentenyl, 2-hexenyl etc.) optionally having 1 to 3 halogen atoms (e.g., a fluorine atom, a chlorine atom, a bromine atom, an iodine atom),
(52) C₂₋₆ alkynyl (e.g., propargyl, 2-butynyl, 3-butynyl, 3-pentynyl, 3-hexynyl etc.),
(53) mono-C₃₋₇ cycloalkyl-carbamoyl (e.g., cyclopropylcarbamoyl, cyclobutylcarbamoyl etc.),
(54) 5- to 10-membered heterocyclyl-carbonyl containing, besides carbon atoms, one or two kinds of 1 to 4 hetero atoms selected from a nitrogen atom, a sulfur atom and an oxygen atom (e.g., 4-morpholinocarbonyl etc.)
and the like.

Examples of the "acyl group" for R³ include an acyl group having 1 to 20 carbon atoms derived from an organic carboxylic acid. Preferable examples thereof include a C₁₋₇ alkanoyl group (e.g., formyl; C₁₋₆ alkyl-carbonyl such as acetyl, propionyl, butyryl, isobutyryl, pentanoyl, hexanoyl, heptanoyl and the like, etc.), a C₆₋₁₄ aryl-carbonyl group (e.g., benzoyl, naphthalenecarbonyl etc.), a C₁₋₆ alkoxy-carbonyl group (e.g., methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl etc.), a C₆₋₁₄ aryloxy-carbonyl group (e.g., phenoxycarbonyl group), a C₇₋₁₉ aralkyl-carbonyl group (e.g., phenyl-C₁₋₄ alkylcarbonyl such as benzylcarbonyl, phenethylcarbonyl, phenylpropylcarbonyl and the like; naphthyl-C₁₋₄ alkylcarbonyl such as benzhydrylcarbonyl, naphthylethylcarbonyl and the like, etc.), a C₇₋₁₉ aralkyloxy-carbonyl group (e.g., phenyl-C₁₋₄ alkyloxycarbonyl such as benzyloxycarbonyl and the like, etc.), a 5- or 6-membered heterocyclyl-carbonyl group or a fused heterocyclyl-carbonyl group thereof (e.g., a 5- or 6-membered heterocyclyl-carbonyl group containing 1 to 4 hetero atoms selected from a nitrogen atom (optionally oxidized), an oxygen atom, a sulfur atom (optionally mono- or di-oxidized) and the like, such as pyrrolylcarbonyl such as 2- or 3-pyrrolylcarbonyl and the like; pyrazolylcarbonyl such as 3-, 4- or 5-pyrazolylcarbonyl and the like; imidazolylcarbonyl such as 2-, 4- or 5-imidazolylcarbonyl and the like; triazolylcarbonyl such as 1,2,3-triazol-4-ylcarbonyl, 1,2,4-triazol-3-ylcarbonyl and the like; tetrazolylcarbonyl such as 1H- or 2H-tetrazol-5-ylcarbonyl and the like; furylcarbonyl such as 2- or 3-furylcarbonyl and the like; thienylcarbonyl such as 2- or 3-thienylcarbonyl and the like; oxazolylcarbonyl such as 2-, 4-or 5-oxazolylcarbonyl and the like; isoxazolylcarbonyl such as 3-, 4- or 5-isoxazolylcarbonyl and the like; oxadiazolylcarbonyl such as 1,2,3-oxadiazol-4- or 5-ylcarbonyl, 1,2,4-oxadiazol-3- or 5-ylcarbonyl, 1,2,5-oxadiazol-3- or 4-ylcarbonyl, 1,3,4-oxadiazol-2-ylcarbonyl and the like; thiazolylcarbonyl such as 2-, 4- or 5-thiazolylcarbonyl and the like; isothiazolylcarbonyl such as 3-, 4- or 5-isothiazolylcarbonyl and the like; thiadiazolylcarbonyl such as 1,2,3-thiadiazol-4- or 5-ylcarbonyl, 1,2,4-thiadiazol-3- or 5-ylcarbonyl, 1,2,5-thiadiazol-3- or 4-ylcarbonyl, 1,3,4-thiadiazol-2-ylcarbonyl and the like; pyrrolidinylcarbonyl such as 2- or 3-pyrrolidinylcarbonyl and the like; pyridylcarbonyl such as 2-, 3- or 4-pyridylcarbonyl and the like; pyridylcarbonyl wherein the nitrogen atom is oxidized such as 2-, 3- or 4-pyridyl-N-oxidocarbonyl and the like; pyridazinylcarbonyl such as 3- or 4-pyridazinylcarbonyl and the like; pyridazinylcarbonyl wherein one or both of the nitrogen atom is oxidized such as 3-, 4-, 5- or 6-pyridazinyl-N-oxidocarbonyl and the like; pyrimidinylcarbonyl such as 2-, 4- or 5-pyrimidinylcarbonyl and the like; pyrimidinylcarbonyl wherein one or both of the nitrogen atom is oxidized such as 2-, 4-, 5- or 6-pyrimidinyl-N-oxidocarbonyl and the like; pyrazinylcarbonyl; piperidylcarbonyl such as 2-, 3- or 4-piperidylcarbonyl and the like; piperazinylcarbonyl; indolylcarbonyl such as 3H-indol-2- or 3-ylcarbonyl and the like; pyranylcarbonyl such as 2-, 3- or 4-pyranylcarbonyl and the like; thiopyranylcarbonyl such as 2-, 3- or 4-thiopyranylcarbonyl and the like; quinolylcarbonyl such as 3-, 4-, 5-, 6-, 7- or 8-quinolylcarbonyl and the like; isoquinolylcarbonyl; pyrido[2,3-d]pyrimidinylcarbonyl (e.g., pyrido[2,3-d]pyrimidin-2-ylcarbonyl); naphthyridinylcarbonyl such as 1,5-, 1,6-, 1,7-, 1,8-, 2,6- or 2,7-naphthyridinylcarbonyl (e.g., 1,5-naphthyridin-2- or 3-ylcarbonyl) and the like; thieno[2,3-d]pyridylcarbonyl (e.g., thieno[2,3-d]pyridin-3-ylcarbonyl); pyrazinoquinolylcarbonyl (e.g., pyrazino[2,3-b]quinolin-2-ylcarbonyl); chromenylcarbonyl (e.g., 2H-chromen-2- or 3-ylcarbonyl etc.) and the like), a 5- or 6-membered heterocyclyl-acetyl group (e.g., a 5- or 6-membered heterocyclyl-acetyl group containing 1 to 4 hetero atoms selected from a nitrogen atom (optionally oxidized), an oxygen atom, a sulfur atom (optionally mono- or di-oxidized) and the like, such as 2-pyrrolylacetyl, 3-imidazolylacetyl, 5-isoxazolylacetyl and the like) and the like.

As the substituent for the acyl group, for example, when the "acyl group" is a C₁₋₇ alkanoyl group or a C₁₋₆ alkoxy-carbonyl group, it is optionally substituted by 1 to 3 substituents selected from alkylthio (e.g., C₁₋₄ alkylthio such as methylthio, ethylthio, n-propylthio, isopropylthio and the like, and the like), a halogen atom (e.g., fluorine, chlorine, bromine, iodine), alkoxy (e.g., C₁₋₆ alkoxy such as methoxy, ethoxy, n-propoxy, tert-butoxy, n-hexyloxy and the like, and the like), nitro, alkoxy-carbonyl (e.g., C₁₋₆ alkoxy-carbonyl such as methoxycarbonyl, ethoxycarbonyl, n-propoxycarbonyl, isopropoxycarbonyl, n-butoxycarbonyl, isobutoxycarbonyl, sec-butoxycarbonyl, tert-butoxycarbonyl and the like, and the like), alkylamino (e.g., mono- or di-C₁₋₆ alkylamino such as methylamino, ethylamino, n-propylamino, n-butylamino, tert-butylamino, n-pentylamino, n-hexylamino, dimethylamino, diethylamino, methylethylamino, di-(n-propyl)amino, di-(n-butyl)amino and the like, and the like), alkoxyimino (e.g., C₁₋₆ alkoxyimino such as methoxyimino, ethoxyimino, n-propoxyimino, tert-butoxy imino, n-hexyloxy-imino and the like, and the like), hydroxyimino, and the like.

When the "acyl group" is a C₆₋₁₄ aryl-carbonyl group, a C₆₋-₁₄ aryloxy-carbonyl group, a C₇₋₁₉ aralkyl-carbonyl group, a C₇₋₁₉ aralkyloxy-carbonyl group, a 5- or 6-membered heterocyclyl-carbonyl group or a 5- or 6-membered heterocyclyl-acetyl group, it is optionally substituted by 1 to 5 (preferably 1 to 3) substituents selected from alkyl (e.g., C₁₋₆ alkyl such as methyl, ethyl, n-propyl, isopropyl, n-butyl, isobutyl, sec-butyl, tert-butyl, n-pentyl, sec-pentyl, isopentyl, neopentyl, n-hexyl, isohexyl and the like; C₃₋₆ cycloalkyl such as cyclohexyl and the like, and the like), alkenyl (e.g., C₂₋₆ alkenyl such as allyl, isopropenyl, isobutenyl, 1-methylallyl, 2-pentenyl, 2-hexenyl and the like, and the like), alkynyl (e.g., C₂₋₆ alkynyl such as propargyl, 2-butynyl, 3-butynyl, 3-pentynyl, 3-hexynyl and the like, and the like), alkoxy (e.g., C₁₋₆ alkoxy such as methoxy, ethoxy, n-propoxy, tert-butoxy, n-hexyloxy and the like, and the like), acyl [e.g., C₁₋₇ alkanoyl such as formyl, acetyl, propionyl, butyryl, isobutyryl, pentanoyl, hexanoyl, heptanoyl and the like; C₆₋₁₄ aryl-carbonyl such as benzoyl, naphthalenecarbonyl and the like; C₁₋₆ alkoxy-carbonyl such as methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl and the like; C₆₋₁₄ aryloxy-carbonyl such as phenoxycarbonyl and the like; C₇₋₁₉ aralkyl-carbonyl such as phenyl-C₁₋₄ alkyl-carbonyl (e.g., benzylcarbonyl, phenethylcarbonyl, phenylpropylcarbonyl and the like) and the like; C₇₋₁₉ aralkyloxy-carbonyl such as phenyl-C₁₋₄ alkyloxy-carbonyl (e.g., benzyloxycarbonyl and the like) and the like, and the like], nitro, amino, hydroxy, cyano, sulfamoyl, mercapto, a halogen atom (e.g., fluorine, chlorine, bromine, iodine), alkylthio (C₁₋₄ alkylthio such as methylthio, ethylthio, n-propylthio, isobutylthio and the like, and the like), and the like.

Examples of the "heterocyclic group" of the "heterocyclic group optionally having substituent(s)" for R³ include a 3- to 8-membered heterocyclic group (preferably a 5- or 6-membered heterocyclic group) containing 1 to 4 hetero atoms selected from a nitrogen atom (optionally oxidized), an oxygen atom, a sulfur atom (optionally mono- or di-oxidized) and the like; and a group derived from a fused ring formed by a 3- to 8-membered heterocycle (preferably a 5- or 6-membered heterocycle) containing 1 to 4 hetero atoms selected from a nitrogen atom (optionally oxidized), an oxygen atom, a sulfur atom (optionally mono- or di-oxidized) and the like, and a benzene ring or a 3- to 8-membered heterocycle (preferably a 5- or 6-membered heterocycle) containing 1 to 4 hetero atoms selected from a nitrogen atom (optionally oxidized), an oxygen atom, a sulfur atom (optionally mono- or di-oxidized) and the like, preferably a group derived from a fused ring formed by a 3- to 8-membered heterocycle (preferably a 5- or 6-membered heterocycle) containing 1 to 4 hetero atoms selected from a nitrogen atom (optionally oxidized), an oxygen atom, a sulfur atom (optionally mono- or di-oxidized) and the like, and a benzene ring.

Specific examples thereof include aziridinyl (e.g., 1- or 2-aziridinyl), azirinyl (e.g., 1- or 2-azirinyl), azetyl (e.g., 2-, 3- or 4- azetyl), azetidinyl (e.g., 1-, 2- or 3-azetidinyl), perhydroazepinyl (e.g., 1-, 2-, 3- or 4-perhydroazepinyl), perhydroazocinyl (e.g., 1-, 2-, 3-, 4- or 5-perhydroazocinyl), pyrrolyl (e.g., 1-, 2- or 3-pyrrolyl), pyrazolyl (e.g., 1-, 3-, 4- or 5-pyrazolyl), imidazolyl (e.g., 1-, 2-, 4- or 5-imidazolyl), triazolyl (e.g., 1,2,3-triazol-1-, 4- or -5-yl, 1,2,4-triazol-1-, 3-, 4- or 5-yl), tetrazolyl (e.g., tetrazol-1-, 2- or 5-yl), furyl (e.g., 2- or 3-furyl), thienyl (e.g., 2- or 3-thienyl), thienyl wherein the sulfur atom is oxidized (e.g., 2- or 3-thienyl-1,1-dioxide), oxazolyl (e.g., 2-, 4- or 5-oxazolyl), isoxazolyl (e.g., 3-, 4- or 5-isoxazolyl), oxadiazolyl (e.g., 1,2,3-oxadiazol-4- or 5-yl, 1,2,4-oxadiazol-3- or 5-yl, 1,2,5-oxadiazol-3-yl, 1,3,4-oxadiazol-2-yl), thiazolyl (e.g., 2-, 4- or 5-thiazolyl), isothiazolyl (e.g., 3-, 4- or 5-isothiazolyl), thiadiazolyl (e.g., 1,2,3-thiadiazol-4- or 5-yl, 1,2,4-thiadiazol-3- or 5-yl, 1,2,5-thiadiazol-3-yl, 1,3,4-thiadiazol-2-yl), pyrrolidinyl (e.g., 1-, 2- or 3-pyrrolidinyl), pyridyl (e.g., 2-, 3- or 4-pyridyl), pyridyl wherein the nitrogen atom is oxidized (e.g., 2-, 3- or 4-pyridyl-N-oxide), pyridazinyl (e.g., 3- or 4-pyridazinyl), pyridazinyl wherein one or both of the nitrogen atom is/are oxidized (e.g., 3-, 4-, 5- or 6-pyridazinyl-N-oxide), pyrimidinyl (e.g., 2-, 4- or 5-pyrimidinyl), pyrimidinyl wherein one or both of the nitrogen atom is/are oxidized (e.g., 2-, 4-, 5- or 6-pyrimidinyl-N-oxide), pyrazinyl, piperidyl (e.g., 1-, 2-, 3- or 4-piperidyl), piperazinyl (e.g., 1- or 2-piperazinyl), indolyl (e.g., 3H-indol-2-, 3-, 4-, 5-, 6- or 7-yl), pyranyl (e.g., 2-, 3- or 4-pyranyl), thiopyranyl (e.g., 2-, 3- or 4-thiopyranyl), thiopyranyl wherein the sulfur atom is oxidized (e.g., 2-, 3-or 4-thiopyranyl-1,1-dioxide), morpholinyl (e.g., 2-, 3- or 4-morpholinyl), thiomorpholinyl, quinolyl (e.g., 2-, 3-, 4-, 5-, 6-, 7- or 8-quinolyl), isoquinolyl, pyrido[2,3-d]pyrimidinyl (e.g., pyrido[2,3-d]pyrimidin-2-yl), naphthyridinyl such as 1,5-, 1,6-, 1,7-, 1,8-, 2,6- or 2,7-naphthyridinyl and the like (e.g., 1,5-naphthyridin-2- or 3-yl), thieno[2,3-d]pyridyl (e.g., thieno[2,3-d]pyridin-3-yl), pyrazinoquinolyl (e.g., pyrazino[2,3-d]quinolin-2-yl), chromenyl (e.g., 2H-chromene-2-or 3-yl), 2-benzo[b]thienyl, 3-benzo[b]thienyl, 2-benzo[b]furanyl, 3-benzo[b]furanyl and the like.
Examples of the "substituent" that the "heterocyclic group" optionally has include those similar to the substituents that the "hydrocarbon group" of the above-mentioned "optionally substituted hydrocarbon group" for R³ optionally has when the hydrocarbon group is cycloalkyl, aryl or aralkyl. The number of the substituents is 1 to 5, preferably 1 to 3.

Examples of the "amino group optionally having substituent(s)" for R³ include a group represented by the formula -NR⁷R⁸ wherein R⁷ and R⁸ are each a hydrogen atom, a hydrocarbon group optionally having substituent(s), a heterocyclic group optionally having substituent(s) or an acyl group.
Examples of the "hydrocarbon group optionally having substituent(s)" for R⁷ or R⁸ include those similar to the above-mentioned "hydrocarbon group optionally having substituent(s)" for R³.
Examples of the "heterocyclic group optionally having substituent(s)" for R⁷ or R⁸ include those similar to the above-mentioned "heterocyclic group optionally having substituent(s)" for R³.
Examples of the "acyl group" for R⁷ or R⁸ include those similar to the above-mentioned "acyl group" for R³.

Examples of the "hydroxy group optionally having a substituent" for R³ include a group represented by the formula -OR⁹ wherein R⁹ is a hydrogen atom, a hydrocarbon group optionally having substituent(s), a heterocyclic group optionally having substituent(s) or an acyl group.
Examples of the "hydrocarbon group optionally having substituent(s)" for R⁹ include those similar to the above-mentioned "hydrocarbon group optionally having substituent(s)" for R³.
Examples of the "heterocyclic group optionally having substituent(s)" for R⁹ include those similar to the above-mentioned "heterocyclic group optionally having substituent(s)" for R³.
Examples of the "acyl group" for R⁹ include those similar to the above-mentioned "acyl group" for R³.

Examples of the "mercapto group optionally having a substituent" for R³ include a group represented by the formula -SR¹⁰ wherein R¹⁰ is a hydrogen atom, a hydrocarbon group optionally having substituent(s), a heterocyclic group optionally having substituent(s) or an acyl group.
Examples of the "hydrocarbon group optionally having substituent(s)" for R¹⁰ include those similar to the above-mentioned "hydrocarbon group optionally having substituent(s)" for R³.
Examples of the "heterocyclic group optionally having substituent(s)" for R¹⁰ include those similar to the above-mentioned "heterocyclic group optionally having substituent(s)" for R³.
Examples of the "acyl group" for R¹⁰ include those similar to the above-mentioned "acyl group" for R³.

R³ is preferably a halogen atom is.

Preferable embodiment the compound (I) of the present invention is shown in the following.

### (1) A compound wherein

the group represented by the partial structural formula:

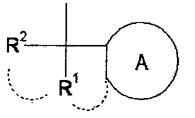

is a group represented by

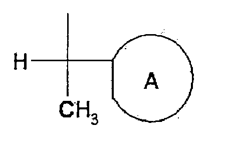

wherein ring A is a phenyl group, a C₃₋₈ cycloalkyl group or a 5- or 6-membered aromatic heterocyclic group (preferably furyl group, a thienyl group, a pyridyl group) (preferably a phenyl group, a thienyl group, a pyridyl group, particularly preferably a phenyl group), each of which optionally has 1 to 3 substituents selected from a hydroxy group, a halogen atom, a cyano group and -S(O)ₚ-R⁶ wherein R⁶ is an alkyl group, and p is an integer of 0 to 2, and
R³ is a halogen atom.

In the compound, preferably
ring A is a phenyl group, a C₃₋₈ cycloalkyl group or a 5- or 6-membered aromatic heterocyclic group (preferably furyl group, a thienyl group, a pyridyl group) (preferably a phenyl group, a thienyl group, a pyridyl group, particularly preferably a phenyl group), each of which optionally has 1 to 3 substituents selected from a hydroxy group, a halogen atom, a cyano group and -S(O)ₚ-R⁶ wherein R⁶ is a C₁₋₆ alkyl group, and p is an integer of 0 to 2, and
R³ is a halogen atom.

More preferably
ring A is a phenyl group, a C₃₋₈ cycloalkyl group or a 5- or 6-membered aromatic heterocyclic group (preferably furyl group, a thienyl group, a pyridyl group) (preferably a phenyl group, a thienyl group, a pyridyl group, particularly preferably a phenyl group), each of which optionally has 1 to 3 substituents selected from a halogen atom, a cyano group and - S(O)ₚ-R⁶ wherein R⁶ is a C₁₋₆ alkyl group, and p is an integer of 0 to 2), and
R³ is a halogen atom.

Still more preferably
ring A is a phenyl group, a C₃₋₈ cycloalkyl group or a 5- or 6-membered aromatic heterocyclic group (preferably furyl group, a thienyl group, a pyridyl group) (preferably a phenyl group, a thienyl group, a pyridyl group, particularly preferably a phenyl group), each of which optionally has 1 to 3 (preferably 1 or 2) substituents selected from a halogen atom and a cyano group, and
R³ is a halogen atom.

Particularly preferably
ring A is a phenyl group, a C₃₋₈ cycloalkyl group or a 5- or 6-membered aromatic heterocyclic group (preferably furyl group, a thienyl group, a pyridyl group) (preferably a phenyl group, a thienyl group, a pyridyl group, particularly preferably a phenyl group), each of which optionally has 1 to 3 (preferably 1) halogen atoms, and
R³ is a halogen atom.

In another embodiment, preferably
ring A is (1) a phenyl group having 1 to 3 substituents selected from (a) a halogen atom, (b) a cyano group and (c) an alkylsulfonyl group, (2) a pyridyl group optionally having substituent(s), or (3) a thienyl group optionally having substituent(s), and
R³ is a halogen atom.

More preferably
ring A is (1) a phenyl group having 1 to 3 substituents selected from (a) a halogen atom, (b) a cyano group and (c) a C₁₋₆ alkylsulfonyl group, (2) a pyridyl group, or (3) a thienyl group, and
R³ is a halogen atom.

Particularly preferably
ring A is a phenyl group having 1 or 2 substituents selected from (a) a halogen atom and (b) a cyano group, and
R³ is a halogen atom.

### (2) A compound wherein

the group represented by the partial structural formula:

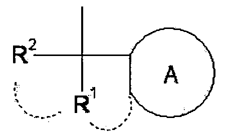

is a fused cyclic group selected from groups represented by

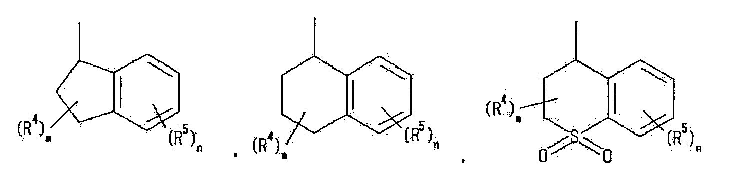

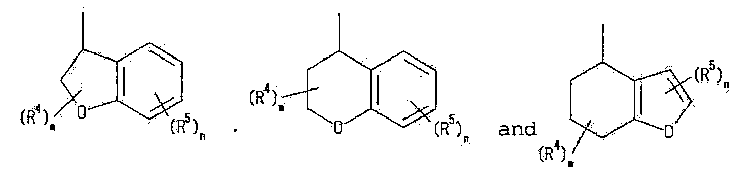

wherein R⁴ and R⁵ are the same or different and each is a substituent selected from a hydroxy group, a halogen atom, a cyano group and -S(O)ₚ-R⁶ wherein R⁶ is an alkyl group, and p is an integer of 0 to 2), m is an integer of 0 to 3, and n is an integer of 0 to 4, and
R³ is a halogen atom.

In the compound, preferably the group represented by

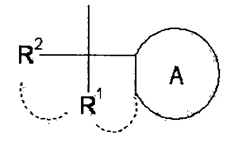

is

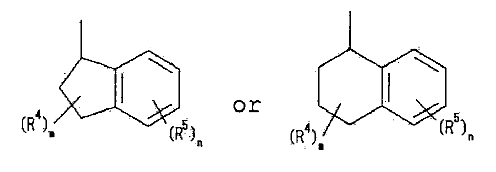

wherein the symbols in the formula are as defined above.

In addition, in the compound, preferably
R⁴ and R⁵ are the same or different and each is a substituent selected from a hydroxy group, a halogen atom, a cyano group and -S(O)ₚ-R⁶ wherein R⁶ is a C₁₋₆ alkyl group, and p is an integer of 0 to 2,
as m and n, m+n=1 or 2 (provided that m and n are the same or different and each is 0 or 1) (preferably m is 0, and n is 1), and
R³ is a halogen atom.

More preferably
R⁴ is a hydroxy group,
R⁵ is a substituent selected from a halogen atom, a cyano group and -S(O)ₚ-R⁶ wherein R⁶ is a C₁₋₆ alkyl group, and p is an integer of 0 to 2) (preferably a halogen atom),
as m and n, m+n=1 or 2 (provided that m and n are the same or different and each is 0 or 1) (preferably m is 0, and n is 1), and
R³ is a halogen atom.

### (3) A compound wherein

the group represented by the partial structural formula:

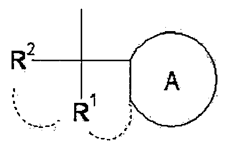

is a fused cyclic group represented by

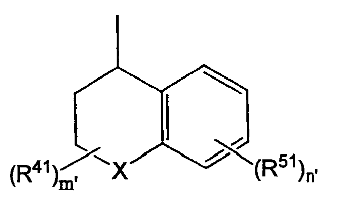

wherein R⁴¹ and R⁵¹ are the same or different and each is a substituent selected from a hydroxy group, a halogen atom, a cyano group and -S(O)ₚ,-R⁶¹ wherein R⁶¹ is an alkyl group, and p' is an integer of 0 to 2, X is S, SO or SO₂, m' is an integer of 0 to 3 , and n' is an integer of 0 to 4, and
R³ is a halogen atom.

In the compound, preferably
X is SO₂,
R⁴¹ and R⁵¹ are the same or different and each is a substituent selected from a hydroxy group, a halogen atom, a cyano group and -S(O)ₚ,-R⁶¹ wherein R⁶¹ is a C₁₋₆ alkyl group, and p' is an integer of 0 to 2,
as m' and n', m'+n'=1 or 2 (provided that m' and n' are the same or different and each is 0 or 1) (preferably m' is 0, and n' is 0 or 1), and
R³ is a halogen atom.

More preferably
X is SO₂,
R⁴¹ is a hydroxy group,
R⁵¹ is a substituent selected from a halogen atom, a cyano group and -S(O)ₚ,-R⁶¹ wherein R⁶¹ is a C₁₋₆ alkyl group, and p' is an integer of 0 to 2) (preferably a halogen atom),
as m' and n', m'+n'=1 or 2 (provided that m' and n' are the same or different and each is 0 or 1) (preferably m' is 0, and n' is 0 or 1), and
R³ is a halogen atom.

### (4) A compound wherein

the group represented by the partial structural formula:

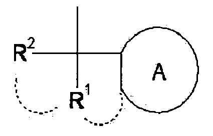

is a group represented by

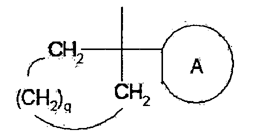

wherein q is an integer of 0 to 4, and ring A is a phenyl group, a C₃₋₈ cycloalkyl group or a 5- or 6-membered aromatic heterocyclic group (preferably furyl group, a thienyl group, a pyridyl group) (preferably a phenyl group, a thienyl group, a pyridyl group, particularly preferably a phenyl group), each of which optionally has 1 to 3 substituents selected from a hydroxy group, a halogen atom, a cyano group and -S(O)ₚ-R⁶ wherein R⁶ is an alkyl group, and p is an integer of 0 to 2, and
R³ is a halogen atom,

Preferably
ring A is a phenyl group, a C₃₋₈ cycloalkyl group or a 5- or 6-membered aromatic heterocyclic group (preferably furyl group, a thienyl group, a pyridyl group) (preferably a phenyl group, a thienyl group, a pyridyl group, particularly preferably a phenyl group), each of which optionally has 1 to 3 substituents selected from a hydroxy group, a halogen atom, a cyano group and -S(O)ₚ-R⁶ wherein R⁶ is a C₁₋₆ alkyl group, and p is an integer of 0 to 2,
q is an integer of 0 to 3 , and
R³ is a halogen atom.

More preferably
ring A is a phenyl group, a C₃₋₈ cycloalkyl group or a 5- or 6-membered aromatic heterocyclic group (preferably furyl group, a thienyl group, a pyridyl group) (preferably a phenyl group, a thienyl group, a pyridyl group, particularly preferably a phenyl group), each of which optionally has 1 to 3 substituents selected from a halogen atom, a cyano group and - S(O)ₚ-R⁶ wherein R⁶ is a C₁₋₆ alkyl group, and p is an integer of 0 to 2,
q is an integer of 0 to 3 , and
R³ is a halogen atom.

Of compound (I),
5-chloro-1-[1-(3-chlorophenyl)ethyl]-2-imino-1,2-dihydropyridine-3-carboxamide (Example 1),
5-chloro-1-(6-chloro-2,3-dihydro-1H-inden-1-yl)-2-imino-1,2-dihydropyridine-3-carboxamide (Example 2),
5-chloro-2-imino-1-(1-(thiophen-3-yl)ethyl)-1,2-dihydropyridine-3-carboxamide (Example 5),
5-chloro-1-[(1R)-1-(3-fluorophenyl)ethyl]-2-imino-1,2-dihydropyridine-3-carboxamide (Example 12),
5-chloro-1-[(1R)-1-(3,5-difluorophenyl)ethyl]-2-imino-1,2-dihydropyridine-3-carboxamide (Example 14),
5-chloro-2-imino-1-(1-phenylcyclopropyl)-1,2-dihydropyridine-3-carboxamide (Example 15),
5-chloro-1-(6-cyano-2,3-dihydro-1H-inden-1-yl)-2-imino-1,2-dihydropyridine-3-carboxamide (Example 19),
5-chloro-1-[(1R)-1-(4-fluorophenyl)ethyl]-2-imino-1,2-dihydropyridine-3-carboxamide (Example 22),
5-chloro-1-[(1R)-1-(3-cyanophenyl)ethyl]-2-imino-1,2-dihydropyridine-3-carboxamide (Example 23),
5-chloro-1-(6-chloro-1,1-dioxido-3,4-dihydro-2H-thiochromen-4-yl)-2-imino-1,2-dihydropyridine-3-carboxamide (Example 30)
and a salt thereof and the like are particularly preferable.

Of these,
5-chloro-1-[1-(3-chlorophenyl)ethyl]-2-imino-1,2-dihydropyridine-3-carboxamide (Example 1),
5-chloro-1-(6-chloro-2,3-dihydro-1H-inden-1-yl)-2-imino-1,2-dihydropyridine-3-carboxamide (Example 2),
5-chloro-1-[(1R)-1-(3-fluorophenyl)ethyl]-2-imino-1,2-dihydropyridine-3-carboxamide (Example 12),
5-chloro-1-[(1R)-1-(3,5-difluorophenyl)ethyl]-2-imino-l,2-dihydropyridine-3-carboxamide (Example 14),
5-chloro-1-[(1R)-1-(3-cyanophenyl)ethyl]-2-imino-1,2-dihydropyridine-3-carboxamide (Example 23),
5-chloro-1-(6-chloro-1,1-dioxido-3,4-dihydro-2H-thiochromen-4-yl)-2-imino-1,2-dihydropyridine-3-carboxamide (Example 30)
and a salt thereof and the like are particularly preferable.

5-Chloro-1-(2,3-dihydro-1H-inden-1-yl)-2-imino-1,2-dihydropyridine-3-carboxamide, 5-chloro-2-imino-1-(1-phenylethyl)-1,2-dihydropyridine-3-carboxamide and 5-chloro-2-imino-1-(1,2,3,4-tetrahydronaphthalen-1-yl)-1,2-dihydropyridine-3-carboxamide are not encompassed in compound (I).
Regarding the "5-chloro-1-(2,3-dihydro-1H-inden-1-yl)-2-imino-1,2-dihydropyridine-3-carboxamide", 5-chloro-1-[(1R)-2,3-dihydro-1H-inden-1-yl]-2-imino-1,2-dihydropyridine-3-carboxamide and 5-chloro-1-[(1S)-2,3-dihydro-1H-inden-1-yl]-2-imino-1,2-dihydropyridine-3-carboxamide are also not encompassed in compound (I).
Like the "5-chloro-2-imino-1-(1-phenylethyl)-1,2-dihydropyridine-3-carboxamide", 5-chloro-2-imino-1-[(1S)-1-phenylethyl]-1,2-dihydropyridine-3-carboxamide and 5-chloro-2-imino-1-[(1R)-1-phenylethyl]-1,2-dihydropyridine-3-carboxamide are also not encompassed in compound (I).

When compound (I) is a salt, examples of such salt include salts with inorganic bases, ammonium salts, salts with organic bases, salts with inorganic acids, salts with organic acids, salts with basic or acidic amino acids and the like.
Preferable examples of the salt with inorganic base include sodium salt, potassium salt and the like alkali metal salt; calcium salt, magnesium salt, barium salt and the like alkaline earth metal salt; aluminum salt and the like.
Preferable examples of the salt with organic base include a salt with trimethylamine, triethylamine, pyridine, picoline, ethanolamine, diethanolamine, triethanolamine, dicyclohexylamine, N,N'-dibenzylethylenediamine and the like.
Preferable examples of the salt with inorganic acid include a salt with hydrochloric acid, hydrobromic acid, nitric acid, sulfuric acid, phosphoric acid and the like.
Preferable examples of the salt with organic acid include a salt with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like.
Preferable examples of the salt with basic amino acid include a salt with arginine, lysine, ornithine and the like.
Preferable examples of the salt with acidic amino acid include a salt with aspartic acid, glutamic acid and the like.
Of these, a pharmaceutically acceptable salt is preferable.

A solvate such as a hydrate is encompassed in the scope of compound (I).
Compound (I) may be labeled with an isotope (e.g., ³H, ¹⁴C, ³⁵S, ¹²⁵I and the like) or the like.
Compound (I) may also a deuterium conversion form.
When compound (I) has an asymmetric center, isomers such as enantiomer, diastereomer and the like may be present. Such isomers and a mixture thereof are all encompassed in the scope of the present invention. When an isomer due to conformation is present, such isomer and a mixture thereof are also encompassed in compound (I) of the present invention.

The production methods of compound (I) or a salt thereof of the present invention is explained in the following.
Compound represented by compound (I) (wherein each symbol is as defined above) can be produced according to the following Method A (wherein X is a halogen atom, and other symbols are as defined above) or a method analogous thereto. Starting material compounds in each step of the following production methods may be used in the form of a salt, and examples of such salt include those similar to the salts of compound (I). In addition, in the following production method, the resultant product may be in the form of a salt which does not adversely affect the reaction.
The compound represented by the formula (I-A)
(hereinafter to be abbreviated as compound (I-A)) is compound (I) wherein R³ is a halogen atom.

### [Method A]

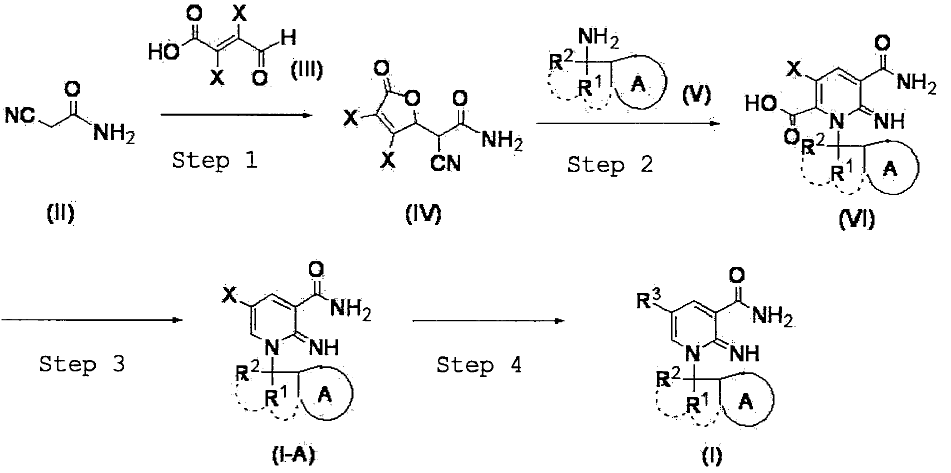

wherein X is a halogen atom, and the other symbols are as defined above.

The compound represented by the formula (II) (hereinafter to be abbreviated as compound (II)) and the compound represented by the formula (V) (hereinafter to be abbreviated as compound (V)), which are used as starting materials for this method, may be a commercially available product, which can be used directly or after isolation and purification, or can be produced according to a method known per se or a method analogous thereto.
The aldehyde represented by the formula (III) (hereinafter to be abbreviated as compound (III)), which is used as a starting material for this method, can be produced according to a method known per se or a method analogous thereto, for example, the method described in J. Am. Chem. Soc., 1953, 75, 1909 or the like.

### (Step 1)

This step is a step of reacting compound (II) with compound (III) in the presence of a base to produce the compound represented by the formula (IV) (hereinafter to be abbreviated as compound (IV)).
This reaction can be generally carried out by reacting compound (II) with compound (III) in the presence of a base, in a solvent inert to the reaction.
Examples of the base used for this reaction include alkali metal salts such as potassium hydroxide, sodium hydroxide, sodium carbonate, potassium carbonate and the like; amines such as pyridine, trimethylamine, N,N-dimethylaniline, 1,8-diazabicyclo[5.4.0]undec-7-en (DBU) and the like; metal alkoxides such as sodium methoxide, sodium ethoxide, potassium tert-butoxide and the like; metal hydrides such as sodium hydride, potassium hydride and the like, and the like.
The amount of the base to be used is generally about 1 to about 20 mol, preferably particularly about 1 to about 3 mol, per 1 mol of compound (II).
The amount of compound (III) to be used is, for example, generally about 1 to about 5 mol, preferably about 1 to about 3 mol, per 1 mol of compound (II).
The solvent for this reaction is not particularly limited as long as the reaction proceeds. Examples thereof include aromatic hydrocarbons such as benzene, toluene, xylene and the like; ethers such as tetrahydrofuran, dimethoxyethane, dioxane, diethyl ether and the like; amides such as N,N-dimethylformamide (DMF), N,N-dimethylacetamide (DMA), 1-methyl-2-pyrrolidone and the like; alcohols such as methanol, ethanol, propanol, tert-butanol, methoxyethanol and the like; sulfoxides such as dimethyl sulfoxide (DMSO) and the like; water; and a mixed solvent thereof.
This reaction is generally carried out at about -50°C to about 200°C, preferably about -10°C to about 100°C. The reaction time of this reaction is generally about 0.5 hr to about 60 hr.
The thus-obtained compound (IV) can be isolated and purified by known separation and purification means such as concentration, concentration under reduced pressure, solvent extraction, crystallization, recrystallization, phase transfer, chromatography and the like.

### (Step 2)

This step is a step of subjecting compound (IV) to a cyclization reaction with the amine represented by the formula (V) (hereinafter to be abbreviated as compound (V)) in the presence of a base, in an inert solvent to produce the compound represented by the formula (VI) (hereinafter to be abbreviated as compound (VI)).
The amount of compound (V) to be used is, for example, generally about 1 to about 10 mol, preferably about 1 to about 3 mol, per 1 mol of compound (IV).
Examples of the base used for this reaction include alkali metal salts such as potassium hydroxide, sodium hydroxide, sodium carbonate, potassium carbonate and the like; amines such as pyridine, trimethylamine, triethylamine, N,N-dimethylaniline, 1,8-diazabicyclo[5.4.0]undec-7-en (DBU) and the like; metal alkoxides such as sodium methoxide, sodium ethoxide, potassium tert-butoxide and the like; organic metals such as n-butyllithium, lithium diisopropylamide (LDA) and the like; metal hydrides such as sodium hydride, potassium hydride and the like; and the like.
The amount of the base to be used is generally about 1 to about 10 mol, preferably about 1 to about 3 mol, per 1 mol of compound (IV).
The solvent for this reaction is not particularly limited as long as the reaction proceeds. Examples thereof include aromatic hydrocarbons such as benzene, toluene, xylene and the like; ethers such as tetrahydrofuran, dimethoxyethane, dioxane, diethyl ether and the like; amides such as N,N-dimethylformamide (DMF), dimethylacetamide (DMA), 1-methyl-2-pyrrolidone and the like; alcohols such as methanol, ethanol, propanol, tert-butanol, methoxyethanol and the like; ketones such as acetone and the like; nitriles such as acetonitrile and the like; sulfoxides such as dimethyl sulfoxide (DMSO) and the like; and a mixed solvent thereof.
This reaction is generally carried out at about -50°C to about 200°C, preferably about -10°C to about 100°C. The reaction time of this reaction is generally about 0.1 hr to about 60 hr.
The thus-obtained compound (VI) can be isolated and purified by known separation and purification means such as concentration, concentration under reduced pressure, solvent extraction, crystallization, recrystallization, phase transfer, chromatography and the like. In addition, compound (VI) may be used in the form of a reaction mixture in the next step (Step 3) without isolation and purification.

### (Step 3)

This step is a step of subjecting compound (VI) to a decarboxylation reaction to produce compound (I-A).
In this decarboxylation reaction, a known decarboxylation reaction can be used. For example, methods such as heating, using an acid or a base with heating if necessary, and the like can be used.
The solvent for this reaction is not particularly limited as long as the reaction proceeds. Examples thereof include aromatic hydrocarbons such as benzene, toluene, xylene and the like; ethers such as tetrahydrofuran, dimethoxyethane, dioxane, diethyl ether and the like; amides such as N,N-dimethylformamide (DMF), N,N-dimethylacetamide (DMA), 1-methyl-2-pyrrolidone and the like; alcohols such as methanol, ethanol, propanol, tert-butanol, methoxyethanol and the like; sulfoxides such as dimethyl sulfoxide (DMSO) and the like; nitriles such as acetonitrile and the like; organic acids such as acetic acid, trifluoroacetic acid and the like; water; and a mixed solvent thereof.
Examples of the base to be used for this reaction include alkali metal salts such as potassium hydroxide, sodium hydroxide, sodium carbonate, potassium carbonate and the like; amines such as pyridine, trimethylamine, N,N-dimethylaniline, 1,8-diazabicyclo[5.4.0]undec-7-en (DBU) and the like; metal alkoxides such as sodium methoxide, sodium ethoxide, potassium tert-butoxide and the like; metal hydrides such as sodium hydride, potassium hydride and the like, and the like. Examples of the acid include inorganic acids such as hydrochloric acid, sulfuric acid, hydrobromic acid and the like; organic acids such as acetic acid, trifluoroacetic acid and the like, and the like.
The amount of the base or acid to be used is, for example, generally about 1 to about 100 mol, preferably about 1 to about 10 mol, per 1 mol of compound (VI).
This reaction is generally carried out at about -50°C to about 200°C, preferably about -10°C to about 100°C. The reaction time of this reaction is generally about 0.1 hr to about 60 hr.
The thus-obtained compound (I-A) can be isolated and purified by known separation and purification means such as concentration, concentration under reduced pressure, solvent extraction, crystallization, recrystallization, phase transfer, chromatography and the like.

### (Step 4)

This step is a step of subjecting compound (I-A) to a known substitution reaction to produce compound (I). Examples of the substitution reaction include insertion reaction of carbon monoxide using a transition metal catalyst, Suzuki coupling reaction, cyanation reaction using zinc cyanide and the like, and the like.
In this step, compound (I-A) protected by a conventional protecting group may be used, as necessary. In this case, the object compound can be obtained by removing the protecting group after reaction, as necessary.
The reaction using a transition metal catalyst can be carried out according to a method known per se [e.g., Chemical Reviews, 1995, vol.95, page 2457, or the like] or a method analogous thereto. For example, the reaction can be carried out in the presence of a transition metal catalyst and a base, in a solvent that does not negatively affect the reaction.
Examples of the transition metal catalyst to be used include palladium catalysts (e.g., palladium(II) acetate, tris(dibenzylideneacetone)dipalladium(0), palladium(II) chloride, tetrakis(triphenylphosphine)palladium(0) and the like), nickel catalysts (e.g., nickel chloride and the like) and the like. Where necessary, a ligand (e.g., triphenylphosphine, tri-tert-butylphosphine and the like) may be added, and a metal oxide (e.g., copper oxide, silver oxide and the like) and the like may be used as a co-catalyst.
While the amount of the catalyst to be used varies depending on the kind of the catalyst, it is generally about 0.0001 to about 1 mol, preferably about 0.01 to about 0.5 mol, per 1 mol of compound (I-A). The amount of the ligand to be used is generally about 0.0001 to about 4 mol, preferably about 0.01 to about 2 mol, per 1 mol of compound (I-A). The amount of the co-catalyst to be used is generally about 0.0001 to about 4 mol, preferably about 0.01 to about 2 mol, per 1 mol of compound (I-A).
Examples of the base to be used include organic amines (e.g., trimethylamine, triethylamine, diisopropylamine, N-methylmorpholine, 1,8-diazabicyclo[5.4.0]undec-7-ene, pyridine, N,N-dimethylaniline and the like), alkali metal salts (e.g., sodium hydrogen carbonate, potassium hydrogen carbonate, sodium carbonate, potassium carbonate, cesium carbonate, sodium phosphate, potassium phosphate, sodium hydroxide, potassium hydroxide and the like), metal hydrides (e.g., potassium hydride, sodium hydride and the like), alkali metal alkoxides (e.g., sodium methoxide, sodium ethoxide, sodium-tert-butoxide, potassium-tert-butoxide and the like), alkali disilazides (e.g., lithium disilazide, sodium disiiazide, potassium disilazide and the like) and the like. Of these, alkali metal salts such as potassium carbonate, cesium carbonate, sodium phosphate, potassium phosphate and the like; alkali metal alkoxides such as sodium-tert-butoxide, potassium-tert-butoxide and the like; organic amines such as triethylamine, diisopropylamine and the like, and the like are preferable.
The amount of the base to be used is generally about 0.1 to about 10 mol, preferably about 1 to about 5 mol, per 1 mol of compound (I-A).
While the solvent to be used is not particularly limited as long as it does not negatively affect the reaction, examples thereof include hydrocarbons (e.g., benzene, toluene, xylene and the like), halogenated hydrocarbons (e.g., chloroform, 1,2-dichloroethane and the like), nitriles (e.g., acetonitrile and the like), ethers (e.g., dimethoxyethane, tetrahydrofuran and the like), alcohols (e.g., methanol, ethanol and the like), aprotic polar solvents (e.g., N,N-dimethylformamide, dimethyl sulfoxide, hexamethylphosphoramide and the like), water, a mixture thereof and the like. The reaction temperature is generally about -10 to about 200°C, preferably about 0 to about 150°C. The reaction time is generally about 0.5 to about 48 hr, preferably about 0.5 to about 16 hr.

Examples of the cyanation reaction include a method known per se [e.g., Synth. Commun., 24. 6. 1994. 887-890 or the like] or a method analogous thereto. The reaction can be carried out, for example, by adding a cyanating agent in a solvent that does not negatively affect the reaction, in the presence of a transition metal catalyst and a base, as necessary.
Examples of the cyanating agent to be used include zinc cyanide, copper cyanide, sodium cyanide, potassium cyanide, trimethylsilylcyanide and the like.
While the amount of the cyanating agent to be used varies depending on the kind of the cyanating agent, it is generally about 1 to about 10 mol, preferably about 1 to about 5 mol, per 1 mol of compound (I-A).
Examples of the transition metal catalyst to be used include palladium catalysts (e.g., palladium(II) acetate, tris(dibenzylideneacetone)dipalladium(0), palladium(II) chloride, tetrakis(triphenylphosphine)palladium(0) and the like), nickel catalysts (e.g., nickel chloride and the like) and the like.
While the amount of the catalyst to be used varies depending on the kind of the catalyst, it is generally about 0.0001 to about 1 mol, preferably about 0.01 to about 0.5 mol, per 1 mol of compound (I-A).
Examples of the base to be used include organic amines (e.g., trimethylamine, triethylamine, diisopropylamine, N-methylmorpholine, 1,8-diazabicyclo[5.4.0]undec-7-ene, pyridine, N,N-dimethylaniline and the like), alkali metal salts (e.g., sodium hydrogen carbonate, potassium hydrogen carbonate, sodium carbonate, potassium carbonate, cesium carbonate, sodium phosphate, potassium phosphate, sodium hydroxide, potassium hydroxide and the like), metal hydrides (e.g., potassium hydride, sodium hydride and the like), alkali metal alkoxides (e.g., sodium methoxide, sodium ethoxide, sodium-tert-butoxide, potassium-tert-butoxide and the like), alkali disilazides (e.g., lithium disilazide, sodium disilazide, potassium disilazide and the like) and the like. Of these, alkali metal salts such as potassium carbonate, cesium carbonate, sodium phosphate, potassium phosphate and the like; alkali metal alkoxides such as sodium-tert-butoxide, potassium-tert-butoxide and the like; organic amines such as triethylamine, diisopropylamine and the like, and the like are preferable.
The amount of the base to be used is generally about 0.1 to about 10 mol, preferably about 1 to about 5 mol, per 1 mol of compound (I-A).
While the solvent to be used is not particularly limited as long as it does not negatively affect the reaction, examples thereof include hydrocarbons (e.g., benzene, toluene, xylene and the like), halogenated hydrocarbons (e.g., chloroform, 1,2-dichloroethane and the like), nitriles (e.g., acetonitrile and the like), ethers (e.g., dimethoxyethane, tetrahydrofuran and the like), alcohols (e.g., methanol, ethanol and the like), aprotic polar solvents (e.g., N,N-dimethylformamide, dimethyl sulfoxide, hexamethylphosphoramide and the like), water, and a mixture thereof. The reaction temperature is generally about -10 to about 200°C, preferably about 0 to about 150°C. The reaction time is generally about 0.5 to about 48 hr, preferably about 0.5 to about 16 hr.
The thus-obtained compound (I) can be isolated and purified by known separation and purification means such as concentration, concentration under reduced pressure, solvent extraction, crystallization, recrystallization, phase transfer, chromatography and the like.

In each of the reactions for the synthesis of the objective compounds and the starting materials, when the starting compounds have an amino group, a carboxyl group or a hydroxyl group as a substituent, such groups may be protected with the protecting groups which are generally used in peptide chemistry etc. In such a case, if necessary, such protecting groups can be removed to obtain the objective compounds after the reactions.
Such a protecting group includes, for example, protecting groups described in "Protective Groups in Organic Synthesis, 3rd Ed. (1999)", edited by Theodara W. Greene, Peter G. M. Wuts, published by Wiley-Interscience.

Examples of the protecting group for the amino group include a formyl group, a C₁₋₆ alkyl-carbonyl group (e.g., an acetyl group, a propionyl group etc.), a phenylcarbonyl group, a C₁₋₆ alkyl-oxycarbonyl group (e.g., methoxycarbonyl group, an ethoxycarbonyl group etc.), an aryloxycarbonyl group (e.g., a phenyloxycarbonyl group etc.), a C₇₋₁₀ aralkyl-carbonyl group (e.g., a benzyloxycarbonyl group etc.), a benzyl group, a benzhydryl group, a trityl group, a phthaloyl etc., each of which may have substituent(s). Examples of such substituent include a halogen atom (e.g., a fluorine atom, a chlorine atom, a bromine atom, an iodine atom etc.), a C₁₋₆ alkyl-carbonyl group (e.g., an acetyl group, a propionyl group, a butylcarbonyl group etc.), a nitro group and the like. The number of substituent(s) is about 1 to 3.

Examples of the protecting group for the carboxyl group include a C₁₋₆ alkyl group (e.g., a methyl group, an ethyl group, a n-propyl group, an isopropyl group, a n-butyl group, a tert-butyl group etc.), a phenyl group, a trityl group, a silyl group and the like, each of which may have substituent(s). Examples of these substituents include a halogen atom (a fluorine atom, a chlorine atom, a bromine atom, an iodine atom), a formyl group, a C₁₋₆ alkyl-carbonyl group (e.g., an acetyl group, a propionyl group, a butylcarbonyl group etc.), a nitro group and the like. The number of substituent(s) is 1 to 3.

Examples of the hydroxyl-protecting group include a C₁₋₆ alkyl group (e.g., a methyl group, an ethyl group, a n-propyl group, an isopropyl group, a n-butyl group, a tert-butyl group etc.), a phenyl group, a C₇₋₁₀ aralkyl group (e.g., a benzyl group etc.), a formyl group, C₁₋₆ alkyl-carbonyl group (e.g., an acetyl group, a propionyl group etc.), an aryloxycarbonyl group (e.g., a phenyloxycarbonyl group etc.), a C₇₋₁₀ aralkyl-carbonyl group (e.g., a benzyloxycarbonyl group etc.), a pyranyl group, a furanyl group, a silyl group and the like, each of which may have substituent(s). Examples of these substituents include a halogen atom (a fluorine atom, a chlorine atom, a bromine atom, an iodine atom), a C₁₋₆ alkyl group, a phenyl group, a C₇₋₁₀ aralkyl group, a nitro group and the like. The number of substituent(s) is 1 to 4.

Such protecting groups can be removed by a known method or the method described in "Protective Groups in Organic Synthesis, 3rd Ed. (1999)", edited by Theodora W. Greene, Peter G. M. Wuts, published by Wiley-Interscience, or the like, or an analogous method thereto. For example, treatment with an acid, a base, reduction, ultraviolet radiation, hydrazine, phenylhydrazine, sodium N-methyldithiocarbamate, tetrabutylammonium fluoride, palladium acetate or the like, can be used.

In the above-mentioned methods, when compound (I) is obtained as a free compound, it can form a salt with, for example, inorganic acid (e.g., hydrochloric acid, sulfuric acid, hydrobromic acid and the like), organic acid (e.g., methanesulfonic acid, benzenesulfonic acid, toluenesulfonic acid, oxalic acid, fumaric acid, maleic acid, tartaric acid and the like), inorganic base (e.g., alkali metals such as sodium, potassium and the like, alkaline earth metals such as calcium, magnesium and the like, aluminum, ammonium and the like) or organic base (e.g., trimethylamine, triethylamine, pyridine, picoline, ethanolamine, diethanolamine, triethanolamine, dicyclohexylamine, N,N'-dibenzylethylenediamine and the like) and the like according to a conventional method. When compound (I) is obtained in the form of a salt, it can also be converted to a free compound or other salt according to a conventional method.
In addition, when the starting compound forms a salt in each of the above-mentioned reactions, the compound may be used as a salt. Such salt includes, for example, those exemplified as the salt of compound (I).

Compound (I) thus prepared by such methods, can be isolated and purified by a typical separation means such as recrystallization, distillation, chromatography and the like.
When compound (I) includes an optical isomer, a stereoisomer, a regioisomer and a rotamer, these are also included in the scope of compound (I), and can be obtained as single products according to synthesis and separation methods known per se (e.g., concentration, solvent extraction, column chromatography, recrystallization etc.). For example, when compound (I) has an optical isomer, the optical isomer resolved from this compound is also encompassed in compound (I).
The optical isomer can be prepared by a method known per se. To be specific, an optically active synthetic intermediate is used, or the final racemate product is subjected to optical resolution according to a conventional method to give an optical isomer.
The method of optical resolution may be a method known per se, such as a fractional recrystallization method, a chiral column method, a diastereomer method etc.

### 1) Fractional recrystallization method

A method wherein a salt of a racemate with an optically active compound (e.g., (+)-mandelic acid, (-)-mandelic acid, (+)-tartaric acid, (-)-tartaric acid, (+)-1-phenethylamine, (-)-1-phenethylamine, cinchonine, (-)-cinchonidine, brucine etc.) is formed, which is separated by a fractional recrystallization method, and if desired, a neutralization step to give a free optical isomer.

### 2) Chiral column method

A method wherein a racemate or a salt thereof is applied to a column for separation of an optical isomer (a chiral column) to allow separation. In the case of a liquid chromatography, for example, a mixture of the optical isomers is applied to a chiral column such as ENANTIO-OVM (manufactured by Tosoh Corporation), CHIRAL series (manufactured by Daicel Chemical Industries, Ltd.) and the like, and developed with water, various buffers (e.g., phosphate buffer, etc.) and organic solvents (e.g., ethanol, methanol, isopropanol, acetonitrile, trifluoroacetic acid, diethylamine etc.) solely or in admixture to separate the optical isomer. In the case of a gas chromatography, for example, a chiral column such as CP-Chirasil-DeX CB (manufactured by GL Sciences Inc.) and the like is used to allow separation.

### 3) Diastereomer method

A method wherein a racemic mixture is prepared into a diastereomeric mixture by chemical reaction with an optically active reagent, which is made into a single substance by a typical separation means (e.g., a fractional recrystallization method, a chromatography method etc.) and the like, and is subjected to a chemical treatment such as hydrolysis and the like to separate an optically active reagent moiety, whereby an optical isomer is obtained. For example, when compound (I) contains hydroxy, or primary or secondary amino group within a molecule, the compound and an optically active organic acid (e.g., MTPA [α-methoxy-α-(trifluoromethyl)phenylacetic acid], (-)-menthoxyacetic acid etc.) and the like are subjected to condensation reaction to give diastereomers of the ester compound or the amide compound, respectively. When compound (I) has a carboxyl group, this compound and an optically active amine or an optically active alcohol are subjected to condensation reaction to give diastereomers of the amide compound or the ester compound, respectively. The separated diastereomer is converted to an optical isomer of the original compound by acid hydrolysis or base hydrolysis.

Compound (I) may be in the form of crystals.
The crystal of compound (I) can be prepared by crystallization of compound (I) by a crystallization method known per se.
Examples of the crystallization method include a method of crystallization from a solution, a method of crystallization from vapor, a method of crystallization from the melts and the like.

The "crystallization from a solution" is typically a method of shifting a non-saturated state to supersaturated state by varying factors involved in solubility of compounds (solvent composition, pH, temperature, ionic strength, redox state etc.) or the amount of solvent. To be specific, for example, a concentration method, a slow cooling method, a reaction method (a diffusion method, an electrolysis method), a hydrothermal growth method, a flux method and the like. Examples of the solvent to be used include aromatic hydrocarbons (e.g., benzene, toluene, xylene etc.), halogenated hydrocarbons (e.g., dichloromethane, chloroform etc.), saturated hydrocarbons (e.g., hexane, heptane, cyclohexane etc.), ethers (e.g., diethyl ether, diisopropyl ether, tetrahydrofuran, dioxane etc.), nitriles (e.g., acetonitrile etc.), ketones (e.g., acetone etc.), sulfoxides (e.g., dimethyl sulfoxide etc.), acid amides (e.g., N,N-dimethylformamide etc.), esters (e.g., ethyl acetate etc.), alcohols (e.g., methanol, ethanol, isopropyl alcohol etc.), water and the like. These solvents are used alone or in a combination of two or more at a suitable ratio (e.g., 1:1 to 1:100 (a volume ratio)). Where necessary, a seed crystal can also be used.
The "crystallization from vapor" is, for example, a vaporization method (a sealed tube method, a gas stream method), a gas phase reaction method, a chemical transportation method and the like.
The "crystallization from the melts" is, for example, a normal freezing method (a Czochralski method, a temperature gradient method and a Bridgman method), a zone melting method (a zone leveling method and a floating zone method), a special growth method (a VLS method and a liquid phase epitaxy method) and the like.

Preferable examples of the crystallization method include a method of dissolving compound (I) in a suitable solvent (e.g., alcohols such as methanol, ethanol etc. and the like) at a temperature of 20 to 120°C, and cooling the resulting solution to a temperature not higher than the temperature of dissolution (e.g., 0 to 50°C, preferably 0 to 20°C) and the like.
The thus obtained crystals of compound (I) can be isolated, for example, by filtration and the like.
As an analysis method of the obtained crystal, crystal analysis by powder X-ray diffraction is generally employed. Moreover, as a method for determining the crystal orientation, a mechanical method, an optical method and the like can also be mentioned.
The crystals of compound (I) obtained in the above-mentioned production method (hereinafter to be abbreviated as "crystal of the present invention") has high purity, high quality and low hygroscopicity, is free of denaturation even after a long-term preservation under normal conditions, and is extremely superior in stability. The crystal is also superior in biological properties (e.g., in vivo kinetics (absorbability, distribution, metabolism, excretion), efficacy expression etc.), and is extremely useful as a pharmaceutical agent.

In the present specification, the melting point means that measured using, for example, a micromelting point apparatus (Yanako, MP-500D) or a DSC (differential scanning calorimetry) device (SEIKO, EXSTAR 6000) and the like.

The prodrug of compound (I) means a compound which is converted to compound (I) with a reaction due to an enzyme, gastric acid, etc. under the physiological condition in the living body, that is, a compound which is converted to compound (I) by enzymatic oxidation, reduction, hydrolysis, etc.; a compound which is converted to compound (I) by hydrolysis etc. due to gastric acid, and the like. A prodrug of compound (I) may be a compound obtained by subjecting an amino group in compound (I) to an acylation, alkylation or phosphorylation (e.g., a compound obtained by subjecting an amino group in compound (I) to an eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation and tert-butylation, etc.); a compound obtained by subjecting a hydroxy group in compound (I) to an acylation, alkylation, phosphorylation or boration (e.g., a compound obtained by subjecting a hydroxy group in compound (I) to an acetylation, palmitoylation, propanoylation, pivaloylation, succinylation, fumarylation, alanylation, dimethylaminomethylcarbonylation, etc.); a compound obtained by subjecting a carboxyl group in compound (I) to an esterification or amidation (e.g., a compound obtained by subjecting a carboxyl group in compound (I) to an ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification and methylamidation, etc.) and the like. Any of these compounds can be produced from compound (I) by a method known per se.
A prodrug for compound (I) may also be one which is converted into compound (I) under a physiological condition, such as those described in IYAKUHIN no KAIHATSU (Development of Pharmaceuticals), Vol.7, Design of Molecules, p.163-198, 1990, Published by HIROKAWA SHOTEN.

Compound (I), a salt thereof and a prodrug thereof are hereinafter collectively abbreviated as "the compound of the present invention".

The compound of the present invention has a superior α_{1D} adrenergic receptor antagonistic action. Specifically, the compound of the present invention has a selective α_{1D} adrenergic receptor antagonistic action. The compound of the present invention is preferably a compound having a selective α_{1D} adrenergic receptor antagonistic action. The selective α_{1D} adrenergic receptor antagonistic action here means the presence of an antagonistic activity at least 10-fold or above for α_{1A} adrenergic receptor, and at least 10-fold or above for α_{1B} adrenergic receptor. Since the compound of the present invention has a selective α_{1D} adrenergic receptor antagonistic action, it decreases blood pressure lowering effect and the like considered to be based on the antagonistic action on the α_{1A} receptor or α_{1B} receptor. Therefore, the compound of the present invention is considered to provide a pharmaceutical agent with a few side effects.

In addition, since the compound of the present invention shows low toxicity (e.g., cardiotoxicity (e.g., human ether-α-go-go related gene (HERG) inhibitory activity), phospholipidosis (PLsis), acute toxicity, chronic toxicity, genotoxicity, reproductive toxicity), drug-drug interaction, carcinogenicity, phototoxicity etc.), it can be safely administered to a mammal (e.g., mouse, rat, hamster, rabbit, cat, dog, bovine, sheep, monkey, human etc.).
Moreover, the compound of the present invention is superior in pharmacokinetics (e.g., absorbability, clearance etc.).

Based on the α_{1D} adrenergic receptor antagonistic action, the compound of the present invention is useful as a drug for the prophylaxis or treatment of any α_{1D} adrenergic receptor associated diseases in mammals (e.g., mouse, rat, hamster, rabbit, cat, dog, bovine, sheep, monkey, human etc.), for example,
(1) lower urinary tract diseases (including all diseases having lower urinary tract symptom as described in the following, e.g., overactive bladder, benign prostatic hyperplasia, interstitial cystitis, chronic prostatitis etc.) storage symptom (daytime urinary frequency, nocturia, urinary urgency, urinary incontinence, stress urinary incontinence, urge urinary incontinence, mixed urinary incontinence, enuresis, nocturnal enuresis, continuous urinary incontinence, other urinary incontinence, enhanced, decreased or missing bladder sensation etc.), voiding symptom (weak urinary stream (or slow stream), split urinary stream (or splitting stream), spraying stream, intermittent urinary stream (or intermittent stream), voiding postponement (or hesitancy), straining at urination (or straining), terminal dribbling (or terminal dribble) etc.), post-micturition symptom (sense of residual urine, post-micturition dribble etc.), symptom due to sexual intercourse (coital pain, vaginal dryness, urinary incontinence etc.), symptom due to pelvic organ prolapse (foreign body sensation, lumbago etc.), genital organ pain or lower urinary tract pain (bladder pain, urethral pain, pudendalgia, vaginodynia, scrotal pain, perineal pain, pelvic pain etc.), genital organ or urinary tract pain syndrome (bladder pain syndrome, urethral pain syndrome, pudendalgia syndrome, vaginal syndrome, scrotal pain syndrome, perineal pain syndrome, pelvic pain syndrome etc.), symptom syndrome suggesting lower urinary tract dysfunction (overactive bladder syndrome, lower urinary tract symptom suggesting bladder outlet obstruction etc.), polyuria, urolithiasis (urinary duct, urethra) and the like],
(2) metabolic diseases [for example, diabetes (insulin dependent diabetes, diabetic complications, diabetic retinopathy, diabetic microangiopathy, diabetic neuropathy etc.), impaired glucose tolerance, obesity, benign prostatic hyperplasia, sexual dysfunction and the like],
(3) central nervous system diseases [for example, neurodegenerative diseases (e.g., Alzheimer's disease, Down's disease, Parkinson's disease, Creutzfeldt-Jakob disease, amyotrophic lateral sclerosis (ALS), Huntington chorea, diabetic neuropathy, multiple sclerosis etc.), mental diseases (e.g., schizophrenia, depression, mania, anxiety neurosis, obsessive-compulsive neurosis, panic disorder, epilepsy, alcohol dependence, drug dependence, anxiety, anxious mental state, emotional abnormality, cyclothymia, nervous erethism, autism, faint, addiction, low sex drive etc.), disorders such as central nervous system and peripheral nerve disorders (e.g., head trauma, spinal damage, brain edema, disorders of sensory function, abnormality of sensory function, disorders of autonomic nervous function, abnormality of autonomic nervous function, whiplash injury etc.), memory disorders (e.g., senile dementia, amnesia, cerebrovascular dementia etc.), cerebrovascular disorder (e.g., cerebral hemorrhage, cerebral infarction and the like and sequelae or complication thereof, asymptomatic cerebrovascular accident, transient cerebral ischemic attack, hypertensive encephalopathia, blood-brain barrier disorder, etc.), recurrence and sequelae of cerebrovascular disorders (e.g., neural symptoms, mental symptoms, subjective symptoms, disorders of daily living activities etc.), central nervous system hypofunction after brain blood vessel occlusion, disorder or abnormality of autoregulation ability of brain circulation or renal circulation etc.], sleep disorder,
(4) genital insufficiency diseases [for example, male erectile dysfunction, dysspermia, female genital insufficiency etc.],
(5) digestive tract diseases [for example, irritable bowel syndrome, inflammatory intestine disease, ulcerative colitis, Crohn's disease, abnormality (e.g., gastritis, gastric ulcer etc.) caused by urease positive herical gram negative bacteria (e.g., Helicobacter pylori etc.), gastric cancer, postgastrostomy disorder, dyspepsia, esophageal ulcer, pancreatitis, colon polyp, cholelithiasis, hemorrhoids, peptic ulcer, situational ileitis, gluttony, constipation, diarrhea, borborygmus etc.],
(6) inflammatory or allergic diseases [for example, allergic rhinitis, conjunctivitis, gastrointestinal allergy, pollinosis, anaphylaxis, dermatitis, herpes, psoriasis, bronchitis, expectoration, retinopathy, postoperative and posttraumatic inflammation, regression of puffiness, pharyngitis, cystitis, meningitidis, inflammatory ocular disease etc.],
(7) osteoarthropathy diseases [for example, rheumatoid arthritis (chronic rheumatoid arthritis), arthritis deformans, rheumatoid myelitis, osteoporosis, abnormal growth of cells, bone fracture, bone refracture, osteomalacia, osteopenia, osseous Behcet's disease, rigid myelitis, articular tissue destruction by gonarthrosis deformans and similar diseases thereto etc.],
(8) respiratory diseases [for example, cold syndrome, pneumonia, asthma, pulmonary hypertension, pulmonary thrombi/pulmonary obliteration, pulmonary sarcoidosis, pulmonary tuberculosis, interstitial pneumonia, silicosis, adult respiratory distress syndrome, chronic obstructive pulmonary diseases, cough etc.],
(9) infectious diseases [HIV infectious diseases, virus infectious diseases due to cytomegalo virus, influenza virus, herpes virus and the like, rickettsia infectious diseases, bacterial infectious diseases, sexually-transmitted diseases, carinii pneumonia, helicobacter pylori infectious disease, systemic fungal infectious diseases, tuberculosis, invasive staphylococcal infectious diseases, acute viral encephalitis, acute bacterial meningitidis, AIDS encephalitis, septicemia, sepsis, sepsis gravis, septic shock, endotoxin shock, toxic shock syndromes etc.],
(10) cancers [for example, primary, metastatic or recurrent breast cancer, prostatic cancer, pancreatic cancer, gastric cancer, lung cancer, colorectal cancer (colon cancer, rectal cancer, anal cancer), esophagus cancer, duodenal cancer, head and neck cancer (cancer of the tongue, pharynx cancer, laryngeal cancer), brain tumor, schwannoma, non-small cell lung cancer, small cell lung cancer, liver cancer, kidney cancer, biliary tract cancer, uterine cancer (uterine body cancer, carcinoma of the uterine cervix), ovary cancer, urinary bladder cancer, skin cancer, Hemangioma, malignant lymphoma, malignant melanoma, thyroid cancer, bone tumor, Hemangioma, vascular fibroma, retinosarcoma, penile cancer, solid cancer in childhood, Kaposi's sarcoma, Kaposi's sarcoma caused by AIDS, maxillary tumor, fibrous histiocytoma, leiomyosarcoma, rhabdomyosarcoma, liposarcoma, fibroid tumors of the uterus, osteoblastoma, osteosarcoma, chondrosarcoma, cancerous mesothelioma, tumors such as leukemia, Hodgkin's disease etc.],
(11) circulatory diseases [for example, acute coronary artery syndromes (e.g., acute myocardial infarction, unstable angina etc.), peripheral arterial obstruction, Raynaud's disease; Buerger disease; restenosis after coronary-artery intervention (percutaneous transluminal coronary angioplasty (PTCA), directional coronary atherectomy (DCA), stenting etc.), restenosis after coronary-artery bypass operation, restenosis after intervention (angioplasty, atherectomy, stenting etc.) or bypass operation in other peripheral artery, ischemic cardiac diseases (e.g., myocardial infarction, angina etc.), myocarditis, intermittent claudication, lacunar infarction, arteriosclerosis (e.g., atherosclerosis etc.), cardiac failure (acute cardiac failure, chronic cardiac failure accompanied by congestion), arrhythmia, progress of atherosclerotic plaque, thrombosis, hypertension, hypertensive tinnitus; hypotension etc.],
(12) pain [for example, headache, migraine, neuralgia and pelvic organ pain including bladder pain etc.],
(13) autoimmune diseases [for example, collagen disease, systemic lupus erythematosus, scleroderma, polyarteritis, myasthenia gravis, multiple sclerosis, Sjogren's syndrome, Behcet's disease etc.],
(14) hepatic diseases [e.g., hepatitis (including chronic hepatitis), cirrhosis, interstitial hepatic diseases etc.],
(15) pancreatic diseases [e.g., pancreatitis (including chronic pancreatitis) etc.],
(16) renal diseases [e.g., nephritis, glomerulonephritis, glomerulosclerosis, renal failure, thrombotic microangiopathy, dialysis complications, organ disorders including nephropathia by radiation, diabetic nephropathy etc.],
(17) endocrine diseases [e.g., Addison's disease, Cushing's syndrome, melanocytoma, primary aldosteronism etc.],
(18) other diseases such as
   (a) transplant rejection [e.g., posttransplantational rejection, posttransplantational polycythemia, hypertension, organ disorder and/or vascular hypertrophy, graft-versus-host disease etc.],
   (b) abnormality in characteristic of blood and/or blood components [e.g., enhancement in platelet aggregation, abnormality of erythrocyte deformability, enhancement in leukocyte adhesiveness, increase in blood viscosity, polycythemia, vascular peliosis, autoimmune hemolytic anemia, disseminated intravascular coagulation syndrome (DIC), multiple myelopathy etc.],
   (c) gynecologic diseases [e.g., climacteric disorder, gestational toxicosis, endometriosis, hysteromyoma, ovarian disease, mammary disease, premenstrual syndrome, pelvic organ prolapse (e.g., prolapse of anterior wall of the vagina, prolapse of vaginal apex, prolapse of posterior wall of vagina, prolapse of uterus etc.), other diseases where organ is prolapsed from the normal position due to weakened pelvic floor muscle (e.g., rectal prolapse etc.) and the like],
   (d) dermatic diseases [e.g., keloid, Hemangioma, psoriasis, pruritus, etc.],
   (e) ophthalmic diseases [e.g., glaucoma, ocular hypertension disease etc.],
   (f) otolaryngological diseases [e.g., Menuel syndrome, tinnitus, gustation disorder, dizziness, disequilibrium, dysphagia etc.],
   (g) diseases due to environmental and/or occupational factors (e.g., radiation disorder, disorders by ultraviolet ray·infrared ray·laser ray, altitude sickness etc.),
   (h) ataxia, rigidity, tremor, motion impairment, akinesia,
   (i) chronic fatigue syndrome,
   (j) sudden infant death syndrome,
   (k) hiccup,
   (l) diseases causing palpitation, vertigo, heartburn and the like.

Among these diseases, the compound of the present invention is particularly useful as an improving agent of lower urinary tract diseases such as hyperactive bladder, stress urinary incontinence of urine, prostatomegaly and the like, as well as a drug for the prophylaxis or treatment of these lower urinary tract diseases.
A preparation comprising the compound of the present invention may be any of solid preparations such as powder, granule, tablet, capsule, orally disintegrable films and the like and liquids such as syrup, emulsion, injection and the like.
An agent for the prophylaxis or treatment of the present invention can be produced by any conventional method, for example, blending, kneading, granulation, tabletting, coating, sterilization, emulsification etc., in accordance with the form of the preparation to be produced. For the production of such pharmaceutical preparations, for example, reference can be made to each of the items in general principles for pharmaceutical preparations in the Japanese Pharmacopeia. In addition, the preparation of the present invention may be formulated into a sustained release preparation containing an active ingredient and a biodegradable polymer compound. The sustained release preparation can be produced according to the method described in JP-A-9-263545.

In the preparations of the present invention, the content of the compound of the present invention varies depending on the forms of the preparations, but is generally 0.01 to 100% by weight, preferably 0.1 to 50% by weight, more preferably 0.5 to 20% by weight, relative to the whole preparation.
When the compound of the present invention is used in the above-mentioned pharmaceutical product, it may be used alone, or in admixture with a suitable, pharmaceutically acceptable carrier, for example, excipients (e.g., starch, lactose, sucrose, calcium carbonate, calcium phosphate etc.), binders (e.g., starch, arabic gum, carboxymethyl cellulose, hydroxypropyl cellulose, crystalline cellulose, alginic acid, gelatin, polyvinyl pyrrolidone etc.), lubricants (e.g., stearic acid, magnesium stearate, calcium stearate, talc etc.), disintegrants (e.g., calcium carboxymethylcellulose, talc etc.), diluents (e.g., water for injection, physiological saline etc.) and if desired, with the additives (e.g., a stabilizer, a preservative, a colorant, a fragrance, a solubilizing agent, an emulsifier, a buffer, an isotonic agent etc.) and the like, by ordinary methods. It can be formulated into the solid preparations such as powders, fine granules, granules, tablets, capsules etc., or into the liquid preparations such as injections etc., and can be administered orally or parenterally. In this case, injection is preferably prepared. It can also be administered as a parenteral agent for topical administration (e.g., intramuscular, subcutaneous, organ or joint injection etc., solid preparation such as implant agent, granules, powder and the like, liquid such as suspension and the like, ointment etc.) and the like.

For example, to produce an injection, the compound of the present invention is prepared into an aqueous suspension together with a dispersing agent (e.g., surfactant such as Tween 80, HCO-60 and the like, polysaccharides such as carboxymethylcellulose, sodium alginate, hyaluronic acid and the like, polysorbate etc.), a preservative (e.g., methylparaben, propylparaben etc.), an isotonicity agent (e.g., sodium chloride, mannitol, sorbitol, glucose etc.), a buffering agent (e.g., calcium carbonate etc.), a pH adjuster (e.g., sodium phosphate, potassium phosphate etc.) and the like, whereby a practical preparation for injection is obtained. In addition, compound (I) is dispersed together with a vegetable oil such as sesame oil, corn oil and the like or a mixture thereof with a phospholipid such as lecithin and the like, or medium-chain fatty acid triglyceride (e.g., miglyol 812 etc.) to give an oily suspension for practical injection.

The prophylactic or therapeutic agent of the present invention can also be used together with other pharmaceutical agents.
A drug which is mixed or combined with the compound of the present invention (hereinafter briefly referred to as a combination drug) includes the following:
(2) Agent for treating diabetes
   Insulin preparations (e.g., animal insulin preparations extracted from the bovine or swine pancreas; human insulin preparations synthesized by a genetic engineering technique using Escherichia coli or a yeast; insulin zinc; protamine zinc insulin; a fragment or a derivative of insulin (e.g., INS-1 etc.), and the like), agents for potentiating insulin sensitivity (e.g., pioglitazone hydrochloride, troglitazone, rosiglitazone or its maleate, JTT-501, MCC-555, YM-440, GI-262570, KRP-297, FK-614, CS-011 etc.), α-glucosidase inhibitors (e.g., voglibose, acarbose, miglitol, emiglitate etc.), biguanides (e.g., phenformin, metformin, buformin etc.), sulfonylureas (e.g., tolbutamide, glibenclamide, gliclazide, chlorpropamide, tolazamide, acetohexamide, glyclopyramide, glimepiride etc.) and other insulin secretagogues (e.g., repaglinide, senaglinide, mitiglinide or its calcium salt hydrate, GLP-1, nateglinide etc.), dipeptidyl peptidase IV inhibitor (e.g., NVP-DPP-278, PT-100, P32/98 etc.), β3 agonists (e.g., CL-316243, SR-58611-A, UL-TG-307, AJ-9677, AZ40140 etc.), amylin agonists (e.g., pramlintide etc.), phosphotyrosine phosphatase inhibitors (e.g., vanadic acid etc.), gluconeogenesis inhibitors (e.g., glycogen phosphorylase inhibitors, glucose-6-phosphatase inhibitors, glucagon antagonists etc.), SGLT (sodium-glucose cotransporter) inhibitors (e.g., T-1095 etc.) and the like.
(3) Agent for treating diabetic complications
   Aldose reductase inhibitors (e.g., tolrestat, epalrestat, zenarestat, zopolrestat, fidarestat (SNK-860), minalrestat (ARI-509), CT-112 etc.), neurotrophic factors (e.g., NGF, NT-3 etc.), AGE inhibitors (e.g., ALT-945, pimagedine, pyratoxathine, N-phenacylthiazolium bromide (ALT-766), EXO-226 etc.), active oxygen scavengers (e.g., thioctic acid etc.), cerebral vasodilators (e.g., tiapuride etc.) and the like.
(4) Antihyperlipidemic agent
   Statin compounds inhibiting cholesterol synthesis (e.g., pravastatin, simvastatin, lovastatin, atorvastatin, fluvastatin, cerivastatin or their salt (e.g., sodium salt etc.) and the like), squalene synthase inhibitors or fibrate compounds having triglyceride lowering action (e.g., bezafibrate, clofibrate, simfibrate, clinofibrate etc.) and the like.
(5) Hypotensive agent
   Angiotensin converting enzyme inhibitors (e.g., captopril, enalapril, delapril etc.), angiotensin II antagonists (e.g., losartan, candesartan cilexetil etc.), calcium antagonists (e.g., manidipine, nifedipine, amlodipine, efonidipine, nicardipine etc.), clonidine, and the like.
(6) Antiobesity agent
   Antiobesity drugs acting on the central nervous system (e.g. dexfenfluramine, fenfluramine, phentermine, sibutramine, anfepramone, dexamphetamine, mazindol, phenylpropanolamine, clobenzorex etc.), pancreatic lipase inhibitors (e.g. orlistat etc.), β₃ agonists (e.g. CL-316243, SR-58611-A, UL-TG-307, AJ-9677, AZ40140 etc.), anorectic peptides (e.g. leptin, CNTF (Ciliary Neurotrophic Factor) etc.), cholecystokinin agonists (e.g. lintitript, FPL-15849 etc.), serotonin2Creceptoragonist (e.g., APD-356, SCA-136, ATHX-105, WAY-163909, YM-348) and the like.
(7) Diuretic agent
   Xanthine derivatives (e.g., theobromine sodium salicylate, theobromine calcium salicylate etc.), thiazide preparations (e.g., ethiazide, cyclopenthiazide, trichlormethiazide, hydrochlorothiazide, hydroflumethiazide, benzylhydrochlorothiazide, penflutizide, polythiazide, methyclothiazide etc.), antialdosterone preparations (e.g., spironolactone, triamterene etc.), carbonic anhydrase inhibitors (e.g., acetazolamide etc.), chlorobenzenesulfonamide preparations (e.g., chlorthalidone, mefruside, indapamide etc.), azosemide, isosorbide, ethacrynic acid, piretanide, bumetanide, furosemide etc.
(8) Chemotherapeutic agent
   Alkylating agents (e.g., cyclophosphamide, ifosfamide etc.), metabolic antagonists (e.g., methotrexate, 5-fluorouracil etc.), antitumor antibiotics (e.g., mitomycin, adriamycin etc.), plant-derived antitumor agents (e.g., vincristine, vindesine, taxol etc.), cisplatin, carboplatin, etoposide etc. Among these, 5-fluorouracil derivatives such as Furtulon and Neo-Furtulon are preferred.
(9) Immunotherapeutic agent
   Microorganism- or bacterium-derived components (e.g., muramyl dipeptide derivatives, Picibanil etc.), immunopotentiator polysaccharides (e.g., lentinan, schizophyllan, krestin etc.), genetically engineered cytokines (e.g., interferons, interleukins (IL) etc.), colony stimulating factors (e.g., granulocyte colony stimulating factor, erythropoietin etc.) and the like. Among these, IL-1, IL-2, IL-12 etc. are preferred.
(10) Therapeutic agent recognized to ameliorate cachexia in animal models or clinical practice
   Progesterone derivatives (e.g., megestrol acetate) [Journal of Clinical Oncology, vol. 12, pp. 213-225, 1994], metoclopramide pharmaceuticals, tetrahydrocannabinol pharmaceuticals (the above reference is applied to both), fat metabolism ameliorating agents (e.g., eicosapentanoic acid) [British Journal of Cancer, vol. 68, pp. 314-318, 1993], growth hormones, IGF-1, and antibodies to the cachexia-inducing factors such as TNF-α, LIF, IL-6 and oncostatin M.
(11) Antiinflammatory agent
   Steroids (e.g., dexamethasone etc.), sodium hyaluronate, cyclooxygenase inhibitors (e.g., indomethacin, ketoprofen, loxoprofen, meloxicam, ampiroxicam, celecoxib, rofecoxib etc.) and the like.
(12) Others
   Glycosylation inhibitors (e.g., ALT-711 etc.), nerve regeneration promoting drugs (e.g., Y-128, VX853, prosaptide etc.), drugs acting on the central nervous system (e.g., antidepressants such as desipramine, amitriptyline, imipramine, fluoxetine, paroxetine, doxepin etc.), anticonvulsants (e.g., lamotrigine, carbamazepine), antiarrhythmic drugs (e.g., mexiletine), acetylcholine receptor ligands (e.g., ABT-594), endothelin receptor antagonists (e.g., ABT-627), monoamine uptake inhibitors (e.g., tramadol), indoleamine uptake inhibitors (e.g., fluoxetine, paroxetine), narcotic analgesics (e.g., morphine), GABA receptor agonists (e.g., gabapentin), GABA uptake inhibitors (e.g., tiagabine), α₂ receptor agonists (e.g., clonidine), local analgesics (e.g., capsaicin), protein kinase C inhibitors (e.g., LY-333531), antianxiety drugs (e.g., benzodiazepines), phosphodiesterase inhibitors (e.g., sildenafil), dopamine receptor agonists (e.g., apomorphine), dopamine receptor antagonists (e.g., haloperidol), serotonin receptor agonists (e.g., tandospirone citrate, sumatryptan), serotonin receptor antagonists (e.g., cyproheptadine hydrochloride, ondansetron), serotonin uptake inhibitors (e.g., fluvoxamine maleate, fluoxetine, paroxetine), sleep-inducing drugs (e.g., triazolam, zolpidem), anticholinergic agents, α₁ receptor blocking agents (e.g., tamsulosin), muscle relaxants (e.g., baclofen etc.), potassium channel openers (e.g., nicorandil), calcium channel blocking agents (e.g., nifedipine), agents for preventing or treating Alzheimer's disease (e.g., donepezil, rivastigmine, galanthamine), agents for treating Parkinson's disease (e.g., L-dopa), agents for preventing or treating multiple sclerosis (e.g., interferon β-1a), histamine H₁ receptor inhibitors (e.g., promethazine hydrochloride), proton pump inhibitors (e.g., lansoprazole, omeprazole), antithrombotic agents (e.g., aspirin, cilostazol), NK-2 receptor antagonists, agents of treating HIV infection (saquinavir, zidovudine, lamivudine, nevirapine), agents of treating chronic obstructive pulmonary diseases (salmeterol, thiotropium bromide, cilomilast) and the like.

Anticholinergic agents include, for example, atropine, scopolamine, homatropine, tropicamide, cyclopentolate, butyl scopolamine bromide, propantheline bromide, methylbenactyzium bromide, mepenzolate bromide, flavoxate, pirenzepine, ipratropium bromide, trihexyphenidyl, oxybutynin, propiverine, darifenacin, tolterodine, temiverine, trospium chloride or a salt thereof (e.g., atropine sulfate, scopolamine hydrobromide, homatropine hydrobromide, cyclopentolate hydrochloride, flavoxate hydrochloride, pirenzepine hydrochloride, trihexyphenidyl hydrochloride, oxybutynin hydrochloride, tolterodine tartrate etc.) and the like, preferably oxybutynin, propiverine, darifenacin, tolterodine, temiverine, trospium chloride or a salt thereof (e.g., oxybutynin hydrochloride, tolterodine tartrate etc.). In addition, acetylcholine esterase inhibitors (e.g., distigmine etc.) and the like can be used.

NK-2 receptor antagonists include, for example, a piperidine derivative such as GR159897, GR149861, SR48968 (saredutant), SR144190, YM35375, YM38336, ZD7944, L-743986, MDL105212A, ZD6021, MDL105172A, SCH205528, SCH62373, R-113281 etc., a perhydroisoindole derivative such as RPR-106145 etc., a quinoline derivative such as SB-414240 etc., a pyrrolopyrimidine derivative such as ZM-253270 etc., a pseudopeptide derivative such as MEN11420 (nepadutant), SCH217048, L-659877, PD-147714 (CAM-2291), MEN10376, S16474 etc., and others such as GR100679, DNK333, GR94800, UK-224671, MEN10376, MEN10627, or a salt thereof, and the like.

For a combined use, the administration time of the compound of the present invention and the concomitant drug is not restricted, and the compound of the present invention or a pharmaceutical composition thereof and the concomitant drug or a pharmaceutical composition thereof can be administered to an administration subject simultaneously, or may be administered at different times. The dosage of the concomitant drug may be determined according to the dose clinically used, and can be appropriately selected depending on an administration subject, administration route, disease, combination and the like.
The administration mode of the concomitant drug is not particularly limited, and the compound of the present invention and the concomitant drug only need to be combined on administration. Examples of such administration mode include the following:
(1) administration of a single preparation obtained by simultaneously processing the compound of the present invention or a pharmaceutical composition thereof and the concomitant drug, (2) simultaneous administration of two kinds of preparations of the compound of the present invention or a pharmaceutical composition thereof and the concomitant drug or a pharmaceutical composition thereof, which have been separately produced, by the same administration route, (3) administration of two kinds of preparations of the compound of the present invention or a pharmaceutical composition thereof and the concomitant drug or a pharmaceutical composition thereof, which have been separately produced, by the same administration route in a staggered manner, (4) simultaneous administration of two kinds of preparations of the compound of the present invention or a pharmaceutical composition thereof and the concomitant drug or a pharmaceutical composition thereof, which have been separately produced, by different administration routes, (5) administration of two kinds of preparations of the compound of the present invention or a pharmaceutical composition thereof and the concomitant drug or a pharmaceutical composition thereof, which have been separately produced, by different administration routes in a staggered manner (e.g., administration in the order of the compound of the present invention or a pharmaceutical composition thereof and the concomitant drug or a pharmaceutical composition thereof, or in the reverse order) and the like.
The compounding ratio of the compound of the present invention to the concomitant drug in the combination agent of the present invention can be appropriately selected depending on the administration subject, administration route, diseases and the like.
For example, the content of the compound of the present invention in the combination agent of the present invention varies depending on the form of a preparation, and usually from about 0.01 to 100 wt%, preferably from about 0.1 to 50 wt%, further preferably from about 0.5 to 20 wt%, based on the whole preparation.

While the content of the concomitant drug in the combination agent of the present invention varies depending on the form of a preparation, it is usually from about 0.01 to 100 wt%, preferably from about 0.1 to 50 wt%, further preferably from about 0.5 to 20 wt%, based on the whole preparation.
While the content of the additives such as carrier and the like in the combination agent of the present invention varies depending on the form of a preparation, it is generally about 1 to 99.99 wt%, preferably about 10 to 90 wt%, based on the whole preparation.
Similar contents can be employed for individual preparations of the compound of the present invention and the concomitant drug.
While the dose varies depending on the kind of the compound of the present invention or a pharmaceutically acceptable salt thereof, administration route, symptom, age of patient and the like, it is, for example, about 0.005 - 50 mg/kg body weight/day, preferably about 0.05 - 10 mg/kg body weight/day, more preferably about 0.2 - 4 mg/kg body weight/day, as the compound of the present invention for oral administration to an adult patient with stress urinary incontinence, which can be administered in about 1 to 3 portions.
When the pharmaceutical composition of the present invention is a sustained-release preparation, the dose varies depending on the kind and content of the compound of the present invention, dosage form, duration of drug release, subject animal of administration (e.g., mammal such as human, rat, mouse, cat, dog, rabbit, cow, pit and the like), and administration object. For parenteral administration, for example, about 0.1 to about 100 mg of the compound of the present invention is designed to be released from the administered preparation in one week.
The dose of the combination drug may be set such that it causes no problems of side effects. The daily dose as the combination drug varies depending on severity of symptoms, age, sex, weight and sensitivity of the subject to be administered, time and interval of administration, property, formulation and kinds of pharmaceutical preparation, kinds of active ingredients, etc., and is not particularly limited. In the case of oral administration, a daily dosage in terms of the concomitant drug is generally in the order of about 0.001 to 2000 mg, preferably about 0.01 to 500 mg, and more preferably about 0.1 to 100 mg, per 1 kg body weight of mammals, which may be administered once a day or in two to four divided portions a day.

In administering the combination drug of the present invention, it may be administered at the same time or, the combination drugs may be administered before administering the compound of the present invention, and vice versa. In case of staggered administration, the time interval varies depending on the active ingredients to be administered, a formulation and an administration route. For example, if the combination drugs are administered first, the compound of the present invention may be administered 1 minute to 3 days, preferably 10 min to 1 day, more preferably 15 min to 1 hr. after administering the combination drugs. If the compound of the present invention is administered first, the combination drugs may be administered 1 minute to 1 day, preferably 10 min to 6 hr, more preferably 15 min to 1 hr. after administering the compound of the present invention.
The pharmaceutical composition of the present invention has low toxicity and can be used safely. Particularly, since the Example compounds shown below are superior in the absorbability by oral administration, they can be advantageously used for oral preparation.

### Example

The present invention is explained in detail in the following by referring to Reference Examples, Examples, Formulation Examples and Experimental Examples. However, the present invention is not limited to the Examples, and may be modified without departing from the scope of the invention.

### (LC-MS measurement condition)

In the following Example, HPLC-mass spectrum (LC-MS) was measured under the following conditions. measurement device: Quattro Micro manufactured by Micromass, HP1100 manufactured by Agilent Technologies, or high-speed liquid chromatography mass spectrometer LCMS-2010A manufactured by SHIMADZU Corporation, or MUX system manufactured by Waters (Micromass ZQ)
column: Capcelpak C18 UG-120 manufactured by Shiseido Co., Ltd., 1.5 X 35 mm, or Develosil Combi-RP-5, 2.0 X 35 mm Nomura Chemical Co., Ltd.
solvent:
SOLUTION A; 5mM ammonium acetate/2% acetonitrile/water,
SOLUTION B; 5mM ammonium acetate/95% acetonitrile/water gradient cycle: 0.00 min (SOLUTION A 100%), 2.00 min (SOLUTION B 100%), 3.00 min (SOLUTION B 100%),3.01 min (SOLUTION A 100%), 3.80 min (SOLUTION A 100%)
flow rate: 0.5 ml/min
detection method: UV 220nm
ionization method: electron impact ionization method (Electron Spray Ionization: ESI)

### (Preparative HPLC conditions)

In the following Example, the purification using preparative HPLC was performed under the following conditions. instrument: High-throughput purification system manufactured by Gilson Inc.
column: Capcelpak C18 UG-120, S-5 µM, 20 x 50 mm manufactured by Shiseido Co., Ltd., or Combi Prep Hydrosphere C18 HS-340-CC, S-5 µM, 20 x 50 mm manufactured by YMC
solvent:
SOLUTION A; 0.1% trifluoroacetic acid containing water,
SOLUTION B; 0.1% trifluoroacetic acid containing acetonitrile
gradient cycle: 0.00 min (SOLUTION A /SOLUTION B =95/5), 1.10 min (SOLUTION A /SOLUTION B =95/5), 5.00 min (SOLUTION A /SOLUTION B =0/100), 6.40 min (SOLUTION A /SOLUTION B =0/100), 6.50 min (SOLUTION A /SOLUTION B =95/5)
flow rate: 20 ml/min
detection method: UV 220nm

### (Other conditions)

¹H-NMR spectrum was measured using AV-400M (400 MHz), AVANCE 300 (300 MHz) or AVANCE II 300 (300 MHz) manufactured by Bruker or VNMRS-400 (400MHz) manufactured by Varian, and using tetramethylsilane as the internal standard, and all δ values were shown by ppm. Unless otherwise specified, the numerical values shown for mixed solvents are volume mixing ratios of respective solvents. Unless otherwise specified, % means weight%. The room temperature (ambient temperature) in the present specification is a temperature of about 10°C to about 35°C.
Unless otherwise specified, elution by column chromatography in Reference Examples and Examples was performed under observation by TLC (thin layer chromatography). For TLC observation, 60F254 manufactured by Merck or TLC (NH) manufactured by FUJI SILYSIA was used as a TLC plate, and the solvent used as an elution solvent for column chromatography was used as an eluent. For detection, a UV detector was employed. Silica gel 60 (70-230 mesh) manufactured by Merck was used as silica gel for column chromatography, and silica gel (CHROMATOREX NH) manufactured by FUJI SILYSIA was used as a basic silica gel.
Other abbreviations used in the description mean the following.
s: singlet
d: doublet
t: triplet
q: quartet
dd: double doublet
br: broad
J: coupling constant
Hz: Hertz
CDCl₃: deuterated chloroform
¹H NMR: proton nuclear magnetic resonance

### Reference Example 1

### 2-cyano-2-(3,4-dichloro-5-oxo-2,5-dihydrofuran-2-yl)acetamide

Mucochloric acid (15.1 g) and 2-cyanoacetamide (7.53 g) were dissolved in methanol (53.6 ml), and 2.5N aqueous sodium hydroxide solution (53.6 ml) was added dropwise with stirring under ice-cooling. The mixture was allowed to warm to room temperature, and further stirred at room temperature for 3 hr. The reaction mixture was poured into 1N hydrochloric acid containing ice water, methanol was evaporated under reduced pressure, and the mixture was extracted with ethyl acetate. The extract was washed with saturated brine, and dried over anhydrous magnesium sulfate. The solvent was evaporated under reduced pressure. The residue was crystallized from ethanol-diisopropyl ether to give the title compound (3.74 g) as pale-brown crystals.
¹H NMR (DMSO-d₆) δ ppm 4.84 (1 H, d, J=3.2 Hz), 5.91 (1 H, d, J=4.0 Hz), 7.85 (1 H, br. s.), 8.03 (1 H, br. s.).

### Example 1

### 5-chloro-1-[1-(3-chlorophenyl)ethyl]-2-imino-1,2-dihydropyridine-3-carboxamide

To a solution of 2-cyano-2-(3,4-dichloro-5-oxo-2,5-dihydrofuran-2-yl)acetamide (0.5 g) in tetrahydrofuran (10 ml) was added a suspension (3 ml) of 1-(3-chlorophenyl)ethanamine hydrochloride (0.62 g) and triethylamine (0.64 g) in tetrahydrofuran under ice-cooling, and the mixture was allowed to warm to room temperature, and stirred for 18 hr. The reaction mixture was concentrated, and the crystals were collected by filtration. The obtained crystals were added to a mixture of dimethyl sulfoxide (5 ml)-water (5 ml), and the mixture was stirred at 80°C for 1 hr. The reaction mixture was concentrated, and the residue was purified by silica gel chromatography to give the title compound (40 mg).
HPLC purity 97%
MS m/z 310 (M+H⁺)
¹H NMR (400 MHz, CDCl₃) δ ppm 1.78 (3 H, d, J=6.8 Hz), 5.32 (1 H, br. s.), 5.79 (1 H, br. s.), 6.35 (1 H, br. s.), 7.08-7.12 (1 H, m), 7.19 (1 H, d, J=2.4 Hz), 7.22 (1 H, s), 7.33-7.38 (2 H, m), 8.16 (1 H, br. s.), 10.94 (1 H, br. s.).

### Example 2

### 5-chloro-1-(6-chloro-2,3-dihydro-1H-inden-1-yl)-2-imino-1,2-dihydropyridine-3-carboxamide

According to the method of Example 1, the title compound was synthesized by reacting 2-cyano-2-(3,4-dichloro-5-oxo-2,5-dihydrofuran-2-yl)acetamide with 6-chloro-2,3-dihydro-1H-inden-1-amine hydrochloride.
HPLC purity 96%
MS m/z 322 (M+H⁺)
¹H NMR (400 MHz, CDCl₃) δ ppm 2.06-2.22 (1 H, m), 2.68-2.81 (1 H, m), 2.95-3.16 (2 H, m), 5.61 (1 H, br. s.), 5.85 (1 H, br. s.), 6.53 (1 H, br. s.), 6.77 (1 H, br. s.), 7.18 (1 H, s), 7.32 (1 H, d, J=8.0 Hz), 7.35-7.39 (1 H, m), 8.14 (1 H, br. s.), 11.07 (1 H, br. s.).

### Example 3

### 5-chloro-1-(7-chloro-1,2,3,4-tetrahydronaphthalen-1-yl)-2-imino-1,2-dihydropyridine-3-carboxamide

According to the method of Example 1, the title compound was synthesized by reacting 2-cyano-2-(3,4-dichloro-5-oxo-2,5-dihydrofuran-2-yl)acetamide with 7-chloro-1,2,3,4-tetrahydronaphthalen-1-amine hydrochloride.
HPLC purity 94%
MS m/z 336 (M+H⁺)
¹H NMR (400 MHz, CDCl₃) δ ppm 1.89 (2 H, m), 2.01-2.21 (2 H, m), 2.79-2.97 (2 H, m), 5.39 (1 H, d, J=4.6 Hz), 5.82 (1 H, br. s.), 6.72 (1 H, s), 6.84 (1 H, br. s.), 6.97 (1 H, d, J=1.7 Hz), 7.16-7.20 (1 H, m), 8.14 (1 H, d, J=2.4 Hz), 10.65 (1 H, br. s.).

### Example 4

### 5-chloro-1-[1-(3-fluorophenyl)ethyl]-2-imino-1,2-dihydropyridine-3-carboxamide hydrochloride

To a solution of 2-cyano-2-(3,4-dichloro-5-oxo-2,5-dihydrofuran-2-yl)acetamide (0.5 g) in ethanol (10 ml) were added stirred 1-(3-fluorophenyl)ethanamine hydrochloride (0.75 g) and triethylamine (0.85 g) at room temperature, and the mixture was allowed to warm to room temperature, and stirred at 50°C for 18 hr. The reaction mixture was concentrated, dimethyl sulfoxide (5 ml) was added, and the mixture was stirred at 80°C for 2 hr. The reaction mixture was concentrated, and the residue was purified by silica gel chromatography. The obtained oil was dissolved in ethyl acetate, 4N hydrogen chloride-ethyl acetate solution was added, and the precipitated crystals were collected by filtration to give the title compound (45 mg).
¹H NMR (400 MHz, DMSO-d₆) δ ppm 1.90 (3 H, d, J=6.6 Hz), 6.25 (1 H, q, J=6.4 Hz), 7.22-7.31 (2 H, m), 7.39 (1 H, d, J=10.3 Hz), 7.45-7.55 (1 H, m), 8.24 (1 H, s), 8.43 (1 H, d, J=2.0 Hz), 8.63 (1 H, d, J=2.0 Hz), 8.73 (1 H, s), 9.88 (2 H, br. s.).

### Example 5

### 5-chloro-2-imino-1-(1-(thiophen-3-yl)ethyl)-1,2-dihydropyridine-3-carboxamide hydrochloride

To a suspension of 2-cyano-2-(3,4-dichloro-5-oxo-2,5-dihydrofuran-2-yl)acetamide (0.5 g) in methanol (5 ml) was added a solution of 1-(thiophen-3-yl)ethanamine hydrochloride (1.06 g) and triethylamine (0.65 g) in methanol (3 ml) at room temperature, and the mixture was heated to 50°C, and stirred overnight. The reaction mixture was concentrated, DMSO (5 ml) was added, and the mixture was stirred at 80°C for 2 hr. The solvent was evaporated under reduced pressure, and the residue was partitioned with ethyl acetate and water. The organic layer was dried over magnesium sulfate, and the solvent was evaporated under reduced pressure. The residue was purified by preparative HPLC. To the obtained yellow oil was added 4N hydrogen chloride-ethyl acetate solution at room temperature, and the precipitated crystals were collected by filtration and recrystallized to give the title compound (23 mg).
¹H NMR (300 MHz, DMSO-d₆) δ ppm 1.83-1.93 (3H, m), 6.00-6.20 (1H, m), 7.13-7.25 (1H, m), 7.64-7.71 (1H, m), 7.73-7.79 (1H, m), 8.22 (1H, s), 8.24-8.32 (1H, m), 8.51-8.58 (1H, m), 8.62 (1H, s), 9.65-9.87 (2H, m).

### Example 6

### 5-chloro-2-imino-1-[1-(2,4,5-trifluorophenyl)ethyl]-1,2-dihydropyridine-3-carboxamide hydrochloride

(Step 1) 1-(2,4,5-Trifluorophenyl)ethanone (5.0 g) and (aminooxy)methane hydrochloride (2.88 g) were stirred in pyridine (20 ml) at room temperature for 3 hr. The reaction mixture was quenched with water, and extracted with ethyl acetate. The extract was washed successively with 1N hydrochloric acid and saturated brine, and dried over magnesium sulfate. The solvent was evaporated under reduced pressure. To a solution of the obtained residue in tetrahydrofuran (50 ml) was added tetrahydrofuran-borane (90 ml, 1M tetrahydrofuran solution) at 0°C. The reaction mixture was heated under reflux for 4 hr, and quenched with ice, and 1N hydrochloric acid (150 ml) was added. The mixture was stirred at 90°C for 2 hr, and ethyl acetate was added to the reaction mixture. The separated aqueous layer was basified with 8N sodium hydroxide solution, and extracted with ethyl acetate. The extract was washed with saturated brine, and dried over magnesium sulfate. The solvent was evaporated under reduced pressure. The obtained residue was dissolved in methanol, 4N hydrogen chloride-ethyl acetate solution (10 ml) was added, and the obtained precipitate was collected by filtration, and washed with ethyl acetate to give 1-(2,4,5-trifluorophenyl)ethanamine hydrochloride (3.81 g).
   ¹H NMR (300 MHz, DMSO-d₆) δ ppm 1.52 (3 H, d, J=6.78 Hz), 4.49-4.68 (1 H, m), 7.60-7.74 (1 H, m), 7.84-8.02 (1 H, m), 8.76 (3 H, s).
(Step 2) According to the method of Example 5, the title compound was synthesized by reacting 2-cyano-2-(3,4-dichloro-5-oxo-2,5-dihydrofuran-2-yl)acetamide with 1-(2,4,5-trifluorophenyl)ethanamine hydrochloride obtained in Step 1.
   ¹H NMR (300 MHz, DMSO-d₆) δ ppm 1.78-1.90 (3H, m), 6.27-6.43 (1H, m), 7.61-7.75 (1H, m), 7.82-7.96 (1H, m), 8.24 (2H, d, J= 2.3 Hz), 8.58 (1H, d, J= 2.3 Hz), 8.70 (1H, s), 9.97 (2H, s).

### Example 7

### 5-chloro-1-[1-(3,5-difluorophenyl)ethyl]-2-imino-1,2-dihydropyridine-3-carboxamide hydrochloride

According to the method of Example 5, the title compound was synthesized by reacting 2-cyano-2-(3,4-dichloro-5-oxo-2,5-dihydrofuran-2-yl)acetamide with 1-(3,5-difluorophenyl)ethanamine hydrochloride.
¹H NMR (300 MHz, DMSO-d₆) δ ppm 1.83-1.95 (3H, m), 6.16-6.32 (1H, m), 7.20-7.39 (3H, m), 8.22 (1H, s), 8.40-8.45 (1H, m), 8.60-8.65 (1H, m), 8.71 (1H, s), 9.78-9.99 (2H, m).

### Example 8

### 5-chloro-1-(6-fluoro-2,3-dihydro-1H-inden-1-yl)-2-imino-1,2-dihydropyridine-3-carboxamide hydrochloride

According to the method of Example 5, the title compound was synthesized by reacting 2-cyano-2-(3,4-dichloro-5-oxo-2,5-dihydrofuran-2-yl)acetamide with 6-fluoro-2,3-dihydro-1H-inden-1-amine hydrochloride.
¹H NMR (300 MHz, DMSO-d₆) δ ppm 2.18-2.36 (1H, m), 2.77-3.00 (2H, m), 3.03-3.19 (1H, m), 6.23 (1H, t, J= 6.6 Hz), 7.22-7.34 (2H, m), 7.44-7.53 (1H, m), 7.73 (1H, s), 8.23 (1H, s), 8.57 (1H, s), 8.67 (1H, s), 9.83 (2H, br.s).

### Example 9

### 5-chloro-1-[1-(4-fluorophenyl)ethyl]-2-imino-1,2-dihydropyridine-3-carboxamide hydrochloride

According to the method of Example 5, the title compound was synthesized by reacting 2-cyano-2-(3,4-dichloro-5-oxo-2,5-dihydrofuran-2-yl)acetamide with 1-(4-fluorophenyl)ethanamine hydrochloride.
¹H NMR (300 MHz, DMSO-d₆) δ ppm 1.89 (3H, d, J= 6.8 Hz), 6.05-6.19 (1H, m), 7.23-7.37 (2H, m), 7.51 (2H, dd, J= 8.7, 5.3 Hz), 8.21 (1H, s), 8.36 (1H, d, J= 1.9 Hz), 8.49-8.67 (2H, m), 9.60-9.83 (2H, m).

### Example 10

### 5-chloro-1-[1-(2,5-difluorophenyl)ethyl]-2-imino-1,2-dihydropyridine-3-carboxamide hydrochloride

According to the method of Example 5, the title compound was synthesized by reacting 2-cyano-2-(3,4-dichloro-5-oxo-2,5-dihydrofuran-2-yl)acetamide with 1-(2,5-difluorophenyl)ethanamine hydrochloride.
¹H NMR (300 MHz, DMSO-d₆) δ ppm 1.87 (3H, d, J= 6.4 Hz), 6.31 (1H, q, J= 6.4 Hz), 7.29-7.46 (2H, m), 7.54-7.64 (1H, m), 8.20-8.29 (2H, m), 8.55 (1H, d, J= 2.3 Hz), 8.65 (1H, s), 9.89 (2H, br.s)..

### Example 11

### 5-chloro-1-[1-(3-cyanophenyl)ethyl]-2-imino-1,2-dihydropyridine-3-carboxamide hydrochloride

According to the method of Example 5, the title compound was synthesized by reacting 2-cyano-2-(3,4-dichloro-5-oxo-2,5-dihydrofuran-2-yl)acetamide with 3-(1-aminoethyl)benzonitrile hydrochloride.
¹H NMR (300 MHz, DMSO-d₆) δ ppm 1.92 (3H, d, J= 6.7 Hz), 6.19 (1H, q, J= 6.7 Hz), 7.61-7.69 (1H, m), 7.71-7.79 (1H, m), 7.89 (1H, d, J= 7.6 Hz), 7.97 (1H, s), 8.22 (1H, s), 8.43 (1H, d, J= 2.3 Hz), 8.59 (1H, d, J= 2.3 Hz), 8.65 (1H, s), 9.79 (2H, br.s).

### Example 12

### 5-chloro-1-[(1R)-1-(3-fluorophenyl)ethyl]-2-imino-1,2-dihydropyridine-3-carboxamide hydrochloride

To a suspension of 2-cyano-2-(3,4-dichloro-5-oxo-2,5-dihydrofuran-2-yl)acetamide (1.0 g) in methanol (10 ml) was added a solution of (1R)-1-(3-fluorophenyl)ethanamine (1.78 g) and triethylamine (1.31 g) in methanol (5 ml) at room temperature, and the mixture was heated to 50°C, and stirred overnight. The reaction mixture was concentrated, DMSO (10 ml) was added, and the mixture was stirred at 80°C for 2 hr. The solvent was evaporated under reduced pressure, the residue was partitioned with ethyl acetate and water, and the organic layer was dried over magnesium sulfate. The solvent was evaporated under reduced pressure. The residue was purified by basic silica gel chromatography (ethyl acetate:methanol=1:0→19:1). To the obtained yellow oil was added 4N hydrogen chloride-ethyl acetate solution at room temperature, and the precipitated crystals were collected by filtration and recrystallized to give the title compound (388 mg).
¹H NMR (300 MHz, DMSO-d₆). δ ppm 1.90 (3H, d, J= 6.4 Hz), 6.19 (1H, s), 7.20-7.30 (2H, m), 7.33-7.42 (1H, m), 7.43-7.55 (1H, m), 8.22 (1H, s), 8.41 (1H, d, J= 2.3 Hz), 8.55-8.74 (2H, m), 9.80 (2H, br.s).
[α]²⁵_{D}= +128.1 (c 0.5, MeOH).

### Example 13

### 5-chloro-1-[1-(4-cyanophenyl)ethyl]-2-imino-1,2-dihydropyridine-3-carboxamide hydrochloride

According to the method of Example 5, the title compound was synthesized by reacting 2-cyano-2-(3,4-dichloro-5-oxo-2,5-dihydrofuran-2-yl)acetamide with 4-(1-aminoethyl)benzonitrile hydrochloride.
¹H NMR (300 MHz, DMSO-d₆) δ ppm 1.93 (3H, d, J= 6.4 Hz), 6.14-6.41 (1H, m), 7.60 (2H, d, J= 8.0 Hz), 7.93 (2H, d, J= 8.7 Hz), 8.22 (1H, s), 8.46 (1H, d, J= 1.9 Hz), 8.56-8.78 (2H, m), 9.67-9.97 (2H, m).

### Example 14

### 5-chloro-1-[(1R)-1-(3,5-difluorophenyl)ethyl]-2-imino-1,2-dihydropyridine-3-carboxamide hydrochloride

To a suspension of 2-cyano-2-(3,4-dichloro-5-oxo-2,5-dihydrofuran-2-yl)acetamide (1.0 g) in methanol (10 ml) was added a solution of (1R)-1-(3,5-difluorophenyl)ethanamine (2.01 g) and triethylamine (1.31 g) in methanol (5 ml) at room temperature, and the mixture was heated to 50°C, and stirred overnight. The reaction mixture was concentrated, DMSO (10 ml) was added, and the mixture was stirred at 80°C for 3 hr. The solvent was evaporated under reduced pressure, the residue was partitioned with ethyl acetate and water, and the organic layer was dried over magnesium sulfate. The solvent was evaporated under reduced pressure. The residue was purified by basic silica gel chromatography (ethyl acetate:methanol=1:0→19:1). To the obtained yellow oil was added 4N hydrogen chloride-ethyl acetate solution at room temperature, and the precipitated crystals were collected by filtration and recrystallized to give the title compound (338 mg).
¹H NMR (300 MHz, DMSO-d₆) δ ppm 1.89 (3H, d, J= 6.8 Hz), 6.12-6.28 (1H, m), 7.21-7.38 (3H, m), 8.22 (1H, s), 8.39-8.45 (1H, m), 8.58-8.64 (1H, m), 8.69 (1H, s), 9.87 (2H, br.s).
[α]²⁵_{D}= +108.3 (c 1.0, MeOH).

### Example 15

### 5-chloro-2-imino-1-(1-phenylcyclopropyl)-1,2-dihydropyridine-3-carboxamide

To a suspension of 2-cyano-2-(3,4-dichloro-5-oxo-2,5-dihydrofuran-2-yl)acetamide (0.5 g) in methanol (10 ml) was added a solution of 1-phenylcyclopropylamine hydrochloride (1.1 g) and triethylamine (0.89 ml) in methanol (5 ml) at room temperature, and the mixture was stirred overnight at 50°C. The solvent was evaporated under reduced pressure, acetic acid (5 ml) was added, and the mixture was stirred at 50°C for 2 hr. The solvent was evaporated under reduced pressure, the residue was partitioned with ethyl acetate and aqueous sodium bicarbonate, and the organic layer was washed with saturated brine, and dried over magnesium sulfate. The solvent was evaporated under reduced pressure. The residue was purified by basic silica gel column chromatography (ethyl acetate:hexane=7:3→1:0). The obtained yellow oil was purified by preparative HPLC, and the obtained fraction was treated with PL-HCO₃ MP (200 mg cartridge, Polymer Laboratory) to give the title compound (2.5 mg).
¹H NMR (300 MHz, CDCl₃) δ ppm 1.03-2.15 (4 H, m), 5.86 (1 H, br. s.), 6.92 (2 H, d, J=7.2 Hz), 7.29-7.50 (4 H, m), 8.26 (1 H, d, J=2.7 Hz), 10.98 (1 H, br. s.).

### Example 16

### 5-chloro-2-imino-1-{1-[3-(methylsulfonyl)phenyl]ethyl}-1,2-dihydropyridine-3-carboxamide hydrochloride

(Step 1) To a solution of 1-[3-(methylsulfonyl)phenyl]ethanol (22.0 g), triphenylphosphine (43.2 g) and phthalimide (24.3 g) in tetrahydrofuran (440 mL) was added diisopropyl azodicarboxylate (32 ml) at 0°C, and the mixture was stirred overnight at room temperature. The solvent was evaporated under reduced pressure, and the residue was partitioned with methylene chloride and 2N hydrochloric acid. The organic layer was washed with aqueous sodium bicarbonate and saturated brine, and dried over anhydrous sodium sulfate. The solvent was evaporated under reduced pressure. The residue was dissolved in diethyl ether (250 ml), and the solution was stirred at 40°C for 1 hr. The precipitated solid was collected by filtration, recrystallized from ethyl acetate to give 2-{1-[3-(methylsulfonyl)phenyl]ethyl}-1H-isoindole-1,3(2H)-dione (21.0 g) as a white solid.
   ¹H NMR (400 MHz, CDCl₃) δ ppm 1.95 (3 H, d, J=7.6 Hz), 3.06 (3 H, s), 5.64 (1 H, q, J=7.2 Hz), 7.55 (1 H, t, J=7.8 Hz), 7.70-7.75 (2 H, m), 7.79-7.87 (4 H, m), 8.07 (1 H, t, J=1.4 Hz).
(Step 2) To a solution (300 ml) of 2-{1-[3-(methylsulfonyl)phenyl]ethyl}-1H-isoindole-1,3(2H)-dione (21.0 g) obtained in Step 1 in ethanol was added hydrazine monohydrate (27.2 ml) at room temperature, and the mixture was heated under reflux for 1 hr. The solvent was evaporated under reduced pressure, and the residue was partitioned with methylene chloride and water. The organic layer was washed with water and saturated brine, and dried over anhydrous sodium sulfate. The solvent was evaporated under reduced pressure. The residue was dissolved in diethyl ether (30 ml), 4N hydrogen chloride-1,4-dioxane solution (31.8 ml) was added under ice-cooling, and the mixture was stirred for 1 hr at room temperature. The precipitated solid was collected by filtration to give 1-[3-(methylsulfonyl)phenyl]ethanamine hydrochloride (12.8 g) as a white solid.
   ¹H NMR (400 MHz, DMSO-d₆) δ ppm 1.55 (3 H, d, J=6.82 Hz), 3.25 (3 H, s), 4.57 (1 H, m), 7.73 (1 H, t, J=7.8 Hz), 7.89 (1 H, d, J=7.6 Hz), 7.95 (1 H, d, J=8.0 Hz), 8.12 (1 H, s), 8.58 (3 H, br.s.).
(Step 3) To a solution of 2-cyano-2-(3,4-dichloro-5-oxo-2,5-dihydrofuran-2-yl)acetamide (1.0 g) and potassium carbonate (1.8 g) in ethanol (10 ml) was added 1-[3-(methylsulfonyl)phenyl]ethanamine hydrochloride (2.1 g) obtained in Step 2 at room temperature, and the mixture was stirred overnight at 70°C. The reaction mixture was filtered through celite, and the solvent was evaporated under reduced pressure. The residue was purified by basic silica gel column chromatography (ethyl acetate:hexane=7:3→1:0). To the obtained yellow oil was added 4N hydrogen chloride-ethyl acetate solution (1 ml), and the precipitated crystals were collected by filtration and recrystallized to give the title compound (350 mg).
   ¹H NMR (300 MHz, DMSO-d₆) δ ppm 1.95 (3 H, d, J=6.6 Hz), 3.27 (3 H, s), 6.31 (1 H, q), 7.63-7.79 (2 H, m), 7.88-8.02 (1 H, m), 8.05 (1 H, br. s.), 8.23 (1 H, br. s.), 8.48 (1 H, d, J=2.1 Hz), 8.62 (1 H, d, J=2.3 Hz), 8.71 (1 H, br. s.), 9.88 (2 H, br. s.)

### Example 17

### 5-chloro-1-[1-(2-fluorophenyl)ethyl]-2-imino-1,2-dihydropyridine-3-carboxamide hydrochloride

2-Cyano-2-(3,4-dichloro-5-oxo-2,5-dihydrofuran-2-yl)acetamide (2.0 g), 1-(2-fluorophenyl)ethanamine (1.42 g) and potassium carbonate (1.76 g) were stirred in ethanol (30 ml) at 70°C for 12 hr. The reaction mixture was quenched with 1N hydrochloric acid, and partitioned with ethyl acetate. The aqueous layer was basified with 8N sodium hydroxide solution, and extracted with ethyl acetate. The extract was washed with saturated brine, and dried over magnesium sulfate. The solvent was evaporated under reduced pressure. The obtained residue was dissolved in methanol, 4N hydrogen chloride-ethyl acetate solution (3 ml) was added, and the mixture was crystallized from ethyl acetate. The precipitated crystals were collected by filtration and recrystallized to give the title compound (60 mg).
¹H NMR (300 MHz, DMSO-d₆) δ ppm 1.89 (3 H, d, J=6.78 Hz), 6.22-6.42 (1 H, m), 7.22-7.45 (2 H, m), 7.49-7.61 (1 H, m), 7.65-7.76 (1 H, m), 8.18 (1 H, d, J=2.26 Hz), 8.25 (1 H, s), 8.55 (1 H, d, J=2.26 Hz), 8.66 (1 H, s), 9.88 (2 H, s).

### Example 18

### 5-chloro-2-imino-1-[6-(methylsulfonyl)-2,3-dihydro-1H-inden-1-yl]-1,2-dihydropyridine-3-carboxamide hydrochloride

(Step 1) 6-(Methylsulfonyl)-2,3-dihydro-1H-inden-1-one (17.2g) was suspended in methanol (250 ml), and sodium borohydride (1.99 g) was slowly added at room temperature. The mixture was stirred for 1 hr, and quenched with acetone (10 ml). The solvent was evaporated under reduced pressure. The residue was partitioned with methylene chloride and 2N hydrochloric acid, and the organic layer was washed with saturated aqueous sodium hydrogen carbonate and saturated brine, and dried over sodium sulfate. The solvent was evaporated under reduced pressure. The precipitated solid was collected by filtration to give 6-(methylsulfonyl)-2,3-dihydro-1H-inden-1-ol (15.6 g) as white crystals.
   ¹H NMR (400 MHz, CDCl₃) δ ppm 1.98-2.09 (1 H, m), 2.12 (1 H, d, J=6.4 Hz), 2.53-2.64 (1 H, m), 2.85-2.96 (1 H, m), 3.06 (3 H, s), 3.14 (1 H, ddd, J=16.8, 8.6, 4.4 Hz), 5.32 (1 H, q, J=6.4 Hz), 7.43 (1 H, d, J=7. 6 Hz), 7.84 (1 H, dd, J=7.8, 1.8 Hz), 7.99 (1 H, s).
(Step 2) To a solution of 6-(methylsulfonyl)-2,3-dihydro-1H-inden-1-ol (15.6 g) obtained in Step 1, triphenylphosphine (28.8 g) and phthalimide (16.2 g) in tetrahydrofuran (340 ml) was added diisopropyl azodicarboxylate (21.4 ml) at 0°C, and the mixture was stirred at room temperature for 2 hr. The solvent was evaporated under reduced pressure, and the residue was partitioned with methylene chloride and 2N hydrochloric acid. The organic layer was washed with saturated aqueous sodium hydrogen carbonate and saturated brine, and dried over anhydrous sodium sulfate. The solvent was evaporated under reduced pressure. The residue was suspended in a mixed solvent of ethyl acetate (50 ml), diisopropyl ether (250 ml) and hexane(300 ml), and the mixture was stirred for 1 hr. The precipitated solid was collected by filtration, and washed with diisopropyl ether. The obtained solid was suspended in ethyl acetate (100 ml), and the suspension was stirred at 50°C for 1 hr, and allowed to cool to room temperature. The solid was collected by filtration, and washed with diethyl ether to give 2-[6-(methylsulfonyl)-2,3-dihydro-1H-inden-1-yl]-1H-isoindole-1,3(2H)-dione (13.0 g) as a white solid.
   ¹H NMR (400 MHz, CDCl₃) δ ppm 2.58 (2 H, m), 3.00 (3 H, s), 3.02-3.14 (1 H, m), 3.45 (1 H, ddd, J=16.8, 9.2, 4.8 Hz), 5.88-5.95 (1 H, t, J=8.0 Hz), 7.49 (1 H, d, J=8.0 Hz), 7.67 (1 H, s), 7.72-7.78 (2 H, m), 7.80-7.88 (3 H, m).
(Step 3) To a suspension (260 ml) of 2-[6-(methylsulfonyl)-2,3-dihydro-1H-inden-1-yl]-1H-isoindole-1,3(2H)-dione (13.0 g) obtained in Step 2 in ethanol was added hydrazine monohydrate (15.3 ml) at room temperature, and the mixture was heated under reflux for 1 hr. The solvent was evaporated under reduced pressure, and the residue was partitioned with methylene chloride and water. The organic layer was washed with water and saturated brine, and dried over anhydrous sodium sulfate. The solvent was evaporated under reduced pressure. The residue was dissolved in methylene chloride, 4N hydrochloric acid-1,4-dioxane (9.54 ml) was added under ice-cooling, and the mixture was stirred at room temperature for 1 hr. The precipitated solid was collected by filtration to give 6-(methylsulfonyl)-2,3-dihydro-1H-inden-1-amine hydrochloride (8.42 g) as a white solid.
   ¹H NMR (400 MHz, DMSO-d₆) δ ppm 1.99-2.13 (1 H, m), 2.54 (1 H, dd, J=6.76, 3.59 Hz), 2.92-3.04 (1 H, m), 3.11-3.19 (1 H, m), 3.20 (3 H, s), 4.81 (1 H, t, J= 6.8 Hz), 7.61 (1 H, d, J=8.0 Hz), 7.91 (1 H, dd, J=8.0, 1.6 Hz), 8.20 (1 H, s), 8.53 (3 H, s).
(Step 4) To a suspension of 2-cyano-2-(3,4-dichloro-5-oxo-2,5-dihydrofuran-2-yl)acetamide (0.5 g) and 6-(methylsulfonyl)-2,3-dihydro-1H-inden-1-amine hydrochloride (0.69 g) obtained in Step 3 in ethanol (10 ml) was added potassium carbonate (0.88 g) at room temperature, and the mixture was stirred overnight at 70°C. The reaction mixture was filtered through celite, and the solvent was evaporated under reduced pressure. The residue was purified by basic silica gel column chromatography (ethyl acetate:hexane=7:3→1:0). To the obtained yellow oil was added 4N hydrogen chloride-ethyl acetate solution (1 ml), and the precipitated crystals were collected by filtration and recrystallized to give the title compound (161 mg).
   ¹H NMR (300 MHz, DMSO-d₆) δ ppm 2.16-2.39 (1 H, m), 2.80-3.12 (2 H, m), 3.12-3.30 (1 H, m), 3.19 (3 H, s), 6.39 (1 H, br. s.), 7.71 (1 H, d, J=8.1 Hz), 7.88 (1 H, br. s.), 7.99 (1 H, s), 7.96 (1 H, d, J=1.7 Hz), 8.24 (1 H, br. s.), 8.61 (1 H, br. s.), 8.73 (1 H, br. s.), 9.97 (2 H, br. s.).

### Example 19

### 5-chloro-1-(6-cyano-2,3-dihydro-1H-inden-1-yl)-2-imino-1,2-dihydropyridine-3-carboxamide hydrochloride

To a suspension of 2-cyano-2-(3,4-dichloro-5-oxo-2,5-dihydrofuran-2-yl)acetamide (1.0 g) and potassium carbonate (0.88 g) in ethanol (10 ml) was added 3-amino-2,3-dihydro-1H-inden-5-carbonitrile hydrochloride (0.99 g) at room temperature, and the mixture was stirred overnight at 70°C. The reaction mixture was quenched with 1N hydrochloric acid, and the mixture was washed with ethyl acetate. The aqueous layer was basified with aqueous sodium hydroxide solution, and extracted with diisopropyl ether. The extract was washed with saturated brine, and the solvent was evaporated under reduced pressure. The residue was purified by basic silica gel column chromatography (ethyl acetate:hexane=7:3→1:0). To the obtained yellow oil was added 4N hydrogen chloride-ethyl acetate solution (1 ml), and the precipitated crystals were collected by filtration and recrystallized to give the title compound (122 mg).
¹H NMR (300 MHz, DMSO-d₆) δ ppm 2.16-2.37 (1 H, m), 2.79-3.12 (2 H, m), 3.19-3.29 (1 H, m), 6.25-6.44 (1 H, m), 7.65 (1 H, d, J=7.6 Hz), 7.84-7.94 (3 H, m), 8.24 (1 H, br. s.), 8.60 (1 H, br. s.), 8.73 (1 H, br. s.), 9.93 (2 H, br. s.).

### Example 20

### 5-chloro-1-[(1R)-1-(2-fluorophenyl)ethyl]-2-imino-1,2-dihydropyridine-3-carboxamide hydrochloride

2-Cyano-2-(3,4-dichloro-5-oxo-2,5-dihydrofuran-2-yl)acetamide (2.81 g), (1R)-1-(2-fluorophenyl)ethanamine (2.0 g) and potassium carbonate (2.48 g) were stirred in ethanol (30 ml) at 70°C for 8 hr. The reaction mixture was quenched with 1N sodium hydroxide solution, and extracted with ethyl acetate. The organic layer was washed with saturated brine, and dried over magnesium sulfate. The solvent was evaporated under reduced pressure. The residue was purified by basic silica gel column chromatography (ethyl acetate). The obtained residue was dissolved in methanol, 4N hydrogen chloride-ethyl acetate solution (3 ml) was added, and the mixture was crystallized from ethyl acetate. The precipitated crystals were collected by filtration and recrystallized to give the title compound (1.80 g).
¹H NMR (300 MHz, DMSO-d₆) δ ppm 1.89 (3 H, d, J=6.78 Hz), 6.30-6.36 (1 H, m), 7.23-7.33 (1 H, m), 7.34-7.44 (1 H, m), 7.48-7.61 (1 H, m), 7.70 (1 H, t, J=7.63 Hz), 8.18 (1 H, d, J=2.07 Hz), 8.24 (1 H, s), 8.56 (1 H, dd, J=3.39, 2.07 Hz), 8.67 (1 H, s), 9.89 (2 H, s).
[α]²⁰_{D}= +188.7 (c 0.50, MeOH).

### Example 21

### 5-chloro-1-[(1R)-1-(2,4-difluorophenyl)ethyl]-2-imino-1,2-dihydropyridine-3-carboxamide hydrochloride

2-Cyano-2-(3,4-dichloro-5-oxo-2,5-dihydrofuran-2-yl)acetamide (2.49 g), (1R)-1-(2,4-difluorophenyl)ethanamine (2.0 g) and potassium carbonate (2.2 g) were stirred in ethanol (30 ml) at 70°C for 18 hr. The reaction mixture was quenched with 1N sodium hydroxide solution, and extracted with ethyl acetate. The organic layer was washed with saturated brine, and dried over magnesium sulfate. The solvent was evaporated under reduced pressure. The residue was purified by basic silica gel column chromatography (ethyl acetate). The obtained residue was dissolved in methanol, 4N hydrogen chloride-ethyl acetate solution (3 ml) was added, and the mixture was crystallized from ethyl acetate. The precipitated crystals were collected by filtration and recrystallized to give the title compound (2.35 g).
¹H NMR (300 MHz, DMSO-d₆) δ ppm 1.87 (3 H, d, J=6.59 Hz), 6.33 (1 H, s), 7.22-7.44 (2 H, m), 7.70-7.84 (1 H, m), 8.19 (1 H, d, J=2.26 Hz), 8.25 (1 H, s), 8.53-8.61 (1 H, m), 8.69 (1 H, s), 9.93 (2 H, s).
[α]²⁰_{D}= +153.6 (c 0.43, MeOH).

### Example 22

### 5-chloro-1-[(1R)-1-(4-fluorophenyl)ethyl]-2-imino-1,2-dihydropyridine-3-carboxamide hydrochloride

2-Cyano-2-(3,4-dichloro-5-oxo-2,5-dihydrofuran-2-yl)acetamide (2.81 g), (1R)-1-(4-fluorophenyl)ethanamine (2.0 g) and potassium carbonate (2.48 g) were stirred in ethanol (30 ml) at 70°C for 14 hr. The reaction mixture was quenched with 1N sodium hydroxide solution, and extracted with ethyl acetate. The organic layer was washed with saturated brine, and dried over magnesium sulfate. The solvent was evaporated under reduced pressure. The residue was purified by basic silica gel column chromatography (ethyl acetate). The obtained residue was dissolved in methanol, 4N hydrogen chloride-ethyl acetate solution (3 ml) was added, and the mixture was crystallized from ethyl acetate. The precipitated crystals were collected by filtration and recrystallized to give the title compound (1.60 g).
¹H NMR (300 MHz, DMSO-d₆) δ ppm 1.89 (3 H, d, J=6.78 Hz), 6.25 (1 H, s), 7.23-7.38 (2 H, m), 7.45-7.63 (2 H, m), 8.22 (1 H, s), 8.37 (1 H, d, J=2.07 Hz), 8.61 (1 H, d, J=6.03 Hz), 8.72 (1 H, d, J=6.03 Hz), 9.88 (2 H, s).
[α]²⁰D= +127. 6 (c 0.45, MeOH).

### Example 23

### 5-chloro-1-[(1R)-1-(3-cyanophenyl)ethyl]-2-imino-1,2-dihydropyridine-3-carboxamide hydrochloride

To a suspension of 2-cyano-2-(3,4-dichloro-5-oxo-2,5-dihydrofuran-2-yl)acetamide (0.49 g) in methanol (10 ml) was added a solution of (1R)-1-(3-cyanophenyl)ethanamine (0.45 g) and triethylamine (0.58 ml) in methanol (5 ml) at room temperature, and the mixture was stirred overnight at 50°C. The reaction solvent was evaporated under reduced pressure, acetic acid (5 ml) was added, and the mixture was stirred at 50°C for 2 hr. The solvent was evaporated under reduced pressure, and the residue was partitioned with ethyl acetate, aqueous sodium bicarbonate and 1N aqueous sodium hydroxide solution. The organic layer was dried over sodium sulfate, and the solvent was evaporated under reduced pressure. The residue was purified by basic silica gel column chromatography (ethyl acetate:hexane=7:3→1:0). The obtained residue was dissolved in methanol, 4N hydrogen chloride-ethyl acetate solution (1 ml) was added, and the mixture was crystallized from ethyl acetate. The precipitated crystals were collected by filtration and recrystallized. The crystals were stirred in n-heptane at 60°C for 3 hr, filtered and dried to give the title compound (0.26 g)
¹H NMR (300 MHz, DMSO-d₆) δ ppm 1.92 (3 H, d, J=6.82 Hz), 6.11-6.26 (1 H, m), 7.65 (1 H, t, J=7.76 Hz), 7.71-7.79 (1 H, m), 7.89 (1 H, d, J=7.57 Hz), 7.97 (1 H, s), 8.22 (1 H, s), 8.43 (1 H, d, J=2.27 Hz), 8.59 (1 H, d, J=1.89 Hz), 8.65 (1 H, s), 9.78 (2 H, s).
[α]²⁰_{D}= +146.8 (c 0.45, MeOH).

### Example 24

### 5-chloro-1-[1-(3-cyano-5-fluorophenyl)ethyl]-2-imino-1,2-dihydropyridine-3-carboxamide hydrochloride

(Step 1) To a solution of 3-bromo-5-fluorobenzonitrile (10.0 g) in tetrahydrofuran (50 ml) was added methylmagnesium bromide (50.0 ml, 3.0 M ether solution) at 0°C under a nitrogen atmosphere. The mixture was allowed to warm to room temperature, stirred for 1.5 hr, and again cooled to 0°C. Lithium aluminum hydride (5.69 g) was added, and the mixture was stirred for 10 min, quenched with water, and partitioned with methylene chloride and water. The organic layer was dried over anhydrous sodium sulfate, and the solvent was evaporated under reduced pressure. The residue was purified by silica gel column chromatography (methylene chloride:methanol=15:1) to give 1-(3-bromo-5-fluorophenyl)ethanamine (5.23 g) as a yellow oil.
   ¹H NMR (400 MHz, CDCl₃) δ ppm 1.36 (3 H, d, J=6.8 Hz), 4.10 (1 H, q, J=6.8 Hz), 7.03 (1 H, dt, J=9.6, 1.6 Hz), 7.11 (1 H, dt, J=8.0, 2.2 Hz), 7.30 (1 H, s).
(Step 2) To a solution of 1-(3-bromo-5-fluorophenyl)ethanamine (5.23 g) obtained in Step 1 in methylene chloride (30 ml) were added di-tert-butyl dicarbonate (5.23 g) and triethylamine (6.69 ml) at 0°C. The mixture was stirred at room temperature for 2 hr, and the solvent was evaporated under reduced pressure. The residue was partitioned with methylene chloride and water, the organic layer was dried over anhydrous sodium sulfate, and the solvent was evaporated under reduced pressure. The residue was purified by silica gel column chromatography (hexane:ethyl acetate=7:1) to give tert-butyl [1-(3-bromo-5-fluorophenyl)ethyl]carbamate (7.10 g) as a white solid.
   ¹H NMR (400 MHz, CDCl₃) δ ppm 1.37 (3 H, s), 1.42 (9 H, s), 4.73 (1 H, br. s.), 4.79 (1 H, br. s.), 6.95 (1 H, d, J=9.2 Hz), 7.12 (1 H, dt, J=8.0, 2.0 Hz), 7.23 (1 H, s).
(Step 3) To a solution of tert-butyl [1-(3-bromo-5-fluorophenyl)ethyl]carbamate (5.2 g) obtained in Step 2 in N,N-dimethylacetamide (30 mL) were added zinc cyanide (0.89 g), tris(dibenzylideneacetone)-dipalladium(0) (0.600 g) and 1,1'-bis(diphenylphosphino)ferrocene (0.73 g) at room temperature. The solution was stirred at 120°C for 3 hr under a nitrogen atmosphere, and the solvent was evaporated under reduced pressure. The residue was filtered, and washed with methanol. The filtrate was concentrated under reduced pressure, and the residue was purified by silica gel column chromatography (hexane:ethyl acetate = 7:1) to give tert-butyl [1-(3-cyano-5-fluorophenyl)ethyl]carbamate (3.20 g) as a white solid.
   ¹H NMR (400 MHz, CDCl₃) δ ppm 1.43 (9 H, s), 1.44 (3 H, d, J=6.8 Hz), 4.81 (1 H, br. s.), 4.86 (1 H, br. s.), 7.23-7.29 (2 H, m), 7.41 (1 H, s).
(Step 4) To a solution of tert-butyl [1-(3-cyano-5-fluorophenyl)ethyl]carbamate (3.90 g) obtained in Step 3 in methylene chloride (20 ml) was added 4N hydrogen chloride-1,4-dioxane solution (2 ml) at 0°C. The mixture was stirred at room temperature for 18 hr, and the solid was collected by filtration to give 3-(1-aminoethyl)-5-fluorobenzonitrile hydrochloride (2.20 g) as a white solid.
   ¹H NMR (400 MHz, DMSO-d₆) δ ppm 1.53 (3 H, d, J=6.8 Hz), 4.52 (1 H, q, J=6. Hz) , 7.85-7.93 (3 H, m), 8.67 (3 H, br. s.).
(Step 5) To a suspension of 2-cyano-2-(3,4-dichloro-5-oxo-2,5-dihydrofuran-2-yl)acetamide (0.5 g) in methanol (10 ml) was added a solution of 3-(1-aminoethyl)-5-fluorobenzonitrile hydrochloride (0.7 g) obtained in Step 4 and triethylamine (1.2 ml) in methanol (5 ml) at room temperature, and the mixture was stirred overnight at 50°C. The reaction solvent was evaporated under reduced pressure, acetic acid (5 ml) was added, and the mixture was stirred at 50°C for 2 hr. The solvent was evaporated under reduced pressure, and the residue was partitioned with ethyl acetate, aqueous sodium bicarbonate and 1N aqueous sodium hydroxide solution. The organic layer was dried over sodium sulfate, and the solvent was evaporated under reduced pressure. The residue was purified by basic silica gel column chromatography (ethyl acetate:hexane=7:3→1:0). To the obtained yellow oil was added 4N hydrogen chloride-ethyl acetate solution (1 ml), and the precipitated crystals were collected by filtration and recrystallized to give the title compound (310 mg).
   ¹H NMR (300 MHz, DMSO-d₆) δ ppm 1.91 (3 H, d, J=6.6 Hz), 6.17 (1 H, q), 7.76 (1 H, dd, J=10.1, 1.4 Hz), 7.83 (1 H, br. s.), 7.89-7.97 (1 H, m), 8.20 (1 H, br. s.), 8.41 (1 H, br. s.), 8.57 (1 H, br. s.), 8.65 (1 H, br. s.), 9.79 (2 H, br. s.).

### Example 25

### 5-chloro-1-[1-(3-chloro-5-cyanophenyl)ethyl]-2-imino-1,2-dihydropyridine-3-carboxamide hydrochloride

(Step 1) To a solution of 3-bromo-5-chlorobenzonitrile (15.0 g) in tetrahydrofuran (50 ml) was added methylmagnesium bromide (69.3 ml, 3.0 M ether solution) at 0°C under a nitrogen atmosphere. The mixture was allowed to warm to room temperature, stirred for 1.5 hr, and again cooled to 0°C. Lithium aluminum hydride (5.69 g) was added, and the mixture was stirred for 10 min, quenched with water, and partitioned with methylene chloride and water. The organic layer was dried over anhydrous sodium sulfate, and the solvent was evaporated under reduced pressure. The residue was purified by silica gel column chromatography (methylene chloride:methanol=15:1) to give 1-(3-bromo-5-chlorophenyl)ethanamine (5.6 g) as a yellow oil.
   ¹H NMR (400 MHz, CDCl₃) δ ppm 1.35 (3 H, d, J=6.8 Hz), 4.07 (1 H, q, J=6.8 Hz), 7.29 (1 H, t, J=1.6 Hz), 7.37 (1 H, t, J=1.8 Hz), 7.41 (1 H, m).
(Step 2) To a solution of 1-(3-bromo-5-chlorophenyl)ethanamine (5.6 g) obtained in Step 1 in methylene chloride (30 ml) were added di-tert-butyl dicarbonate (6.8 g) and triethylamine (6.7 ml) at 0°C. The mixture was stirred at room temperature for 2 hr, and the solvent was evaporated under reduced pressure. The residue was partitioned with methylene chloride and water, the organic layer was dried over anhydrous sodium sulfate, and the solvent was evaporated under reduced pressure. The residue was purified by silica gel column chromatography (hexane:ethyl acetate=7:1) to give tert-butyl [1-(3-bromo-5-chlorophenyl)ethyl]carbamate (6.08 g) as a white solid.
   ¹H NMR (400 MHz, CDCl₃) δ ppm 1.40 (9 H, s), 1.42 (3 H, s), 4.71 (1 H, br. s.), 4.79 (1 H, br. s.), 7.22 (1 H, t, J=1.4 Hz), 7.32 (1 H, t, J=1.4 Hz), 7.39 (1 H, t, J=1.8 Hz).
(Step 3) To a solution of tert-butyl [1-(3-bromo-5-chlorophenyl)ethyl]carbamate (6.08 g) obtained in Step 2 in N,N-dimethylacetamide (30 ml) were added zinc cyanide (0.99 g), tris(dibenzylideneacetone)-dipalladium(0) (0.67 g) and 1,1'-bis(diphenylphosphino)ferrocene (0.81 g) at room temperature. The solution was stirred at 120°C for 3 hr under a nitrogen atmosphere, and the solvent was evaporated under reduced pressure. The residue was filtered, and washed with methanol. The filtrate was concentrated under reduced pressure, and the residue was purified by silica gel column chromatography (hexane:ethyl acetate=7:1) to give tert-butyl [1-(3-cyano-5-chlorophenyl)ethyl]carbamate (3.9 g) as a white solid.
   ¹H NMR (400 MHz, CDCl₃) δ ppm 1.42 (9 H, s), 1.44 (3 H, s), 4.75 (1 H, br. s.), 4.83 (1 H, br. s.), 7.49 (1 H, s), 7.52 (2 H, m).
(Step 4) To a solution of tert-butyl [1-(3-cyano-5-chlorophenyl)ethyl]carbamate (3.90 g) obtained in Step 3 in methylene chloride (20 ml) was added 4N hydrogen chloride-1,4-dioxane solution (2 ml) at 0°C. The mixture was stirred at room temperature for 18 hr, and the solid was collected by filtration to give 3-(1-aminoethyl)-5-chlorobenzonitrile hydrochloride (2.39 g) as a white solid.
   ¹H NMR (400 MHz, DMSO-d₆) δ ppm 1.52 (3 H, d, J=7.0 Hz), 4.50 (1 H, q, J=7.0 Hz), 8.03 (2 H, d, J=2.0 Hz), 8.10 (1 H, t, J=1.6 Hz), 8.58 (3 H, br. s.).
(Step 5) To a suspension of 2-cyano-2-(3,4-dichloro-5-oxo-2,5-dihydrofuran-2-yl)acetamide (0.65 g) in methanol (10 ml) was added a solution of 3-(1-aminoethyl)-5-chlorobenzonitrile hydrochloride (1.0 g) obtained in Step 4 and triethylamine (1.5 ml) in methanol (5 ml) at room temperature. The mixture was stirred overnight at 50°C, and the reaction solvent was evaporated under reduced pressure. Acetic acid (5 ml) was added, and the mixture was stirred at 50°C for 2 hr. The solvent was evaporated under reduced pressure, and the residue was partitioned with ethyl acetate, aqueous sodium bicarbonate and 1N aqueous sodium hydroxide solution. The organic layer was dried over sodium sulfate, and the solvent was evaporated under reduced pressure. The residue was purified by basic silica gel column chromatography (ethyl acetate:hexane=7:3→1:0). To the obtained yellow oil was added 4N hydrogen chloride-ethyl acetate solution (1 ml), and the precipitated crystals were collected by filtration and recrystallized to give the title compound (374 mg).
   ¹H NMR (300 MHz, DMSO-d₆) δ ppm 1.91 (3 H, d, J=6.6 Hz), 6.18 (1 H, q), 7.94 (2 H, d, J=1.5 Hz), 8.11 (1 H, t, J=1.7 Hz), 8.22 (1 H, br. s.), 8.44 (1 H, d, J=2.3 Hz), 8.61 (1 H, d, J=1.9 Hz), 8.68 (1 H, br. s.), 9.84 (2 H, br. s.).

### Example 26

### 5-chloro-2-imino-1-(1-(pyridin-3-yl)ethyl)-1,2-dihydropyridine-3-carboxamide dihydrochloride

2-Cyano-2-(3,4-dichloro-5-oxo-2,5-dihydrofuran-2-yl)acetamide (1.00 g), 1-(pyridin-3-yl)ethanamine (0.68 g) and potassium carbonate (1.18 g) were stirred in ethanol (10 ml) overnight at 0°C. The reaction mixture was filtered through celite, and the solvent was evaporated under reduced pressure. The residue was purified by basic silica gel column chromatography (ethyl acetate:hexane=7:3→1:0). To the obtained yellow oil was added 4N hydrogen chloride-ethyl acetate solution (1 ml), and the precipitated crystals were collected by filtration and recrystallized to give the title compound (88 mg).
¹H NMR (300 M Hz, DMSO-d₆) δ ppm 1.98 (3H, d, J=6.4 Hz), 6.52 (1H, q, J=6.7 Hz), 7.91 (1H, dd, J=8.3, 5.3 Hz), 8.25 (1H, s), 8.42 (1H, d, J=8.3 Hz), 8.54 (1H, d, J=2.3 Hz), 8.71 (1H, d, J=1.9 Hz), 8.84 (2H, d, J=5.3 Hz), 9.00 (1H, s), 10.12 (2H, br. s.).

### Example 27

### 5-chloro-1-(1,1-dioxido-3,4-dihydro-2H-thiochromen-4-yl)-2-imino-1,2-dihydropyridine-3-carboxamide hydrochloride

(Step 1) 2,3-Dihydro-4H-thiochromen-4-one (13.0 g) and o-methylhydroxylamine hydrochloride (7.93 g) were stirred in pyridine (30 ml) at room temperature for 4 hr. The reaction solution was poured into water, and the mixture was extracted with ethyl acetate. The extract was washed with 1N hydrochloric acid and saturated brine, dried over magnesium sulfate and filtered. The solvent was evaporated under reduced pressure. To a solution (150 ml) of the obtained residue in tetrahydrofuran was added tetrahydrofuran-borane (200 ml, 1M tetrahydrofuran solution) at 0°C. The reaction mixture was stirred at 90°C for 3 hr, and quenched with ice, and 1N hydrochloric acid (300 ml) was added. The mixture was stirred at 90°C for 2 hr, and ethyl acetate was added thereto. The separated aqueous layer was basified with 8N sodium hydroxide solution, and extracted with ethyl acetate. The extract was washed with saturated brine, dried over magnesium sulfate and filtered. The solvent was evaporated under reduced pressure. The obtained residue was dissolved in methanol, 4N hydrogen chloride-ethyl acetate solution (20 ml) was added, and the obtained precipitate was collected by filtration, and washed with ethyl acetate to give 3,4-dihydro-2H-thiochromen-4-amine hydrochloride (6.16 g).
   ¹H NMR (300 M Hz, DMSO-d₆) δ ppm 2.12-2.27 (1H, m) 2.36-2.48 (1H, m) 2.96-3.29 (2H, m) 4.52 (1H, d, J=3.41 Hz) 7.09-7.31 (3H, m) 7.53 (1H, d, J=7.95 Hz) 8.66 (3H, s).
(Step 2) To a solution of 3,4-dihydro-2H-thiochromen-4-amine hydrochloride (5.3 g) obtained in Step 1 and triethylamine (5.85 g) in tetrahydrofuran (200 ml) was added di-tert-butyl dicarbonate (6.88 g) at room temperature. The mixture was stirred at the same temperature for 3 hr. The reaction solution was poured into water, and the mixture was extracted with ethyl acetate. The extract was washed with 1N hydrochloric acid and saturated brine, dried over magnesium sulfate and filtered. The solvent was evaporated under reduced pressure. The obtained residue was dissolved in ethyl acetate (200 ml), m-chloroperbenzoic acid (18.1 g, containing water: Wako Pure Chemical Industries, Ltd.) was added at room temperature. The reaction mixture was stirred at the same temperature for 6 hr, washed with aqueous sodium hydrogen carbonate solution and saturated brine, dried over magnesium sulfate and filtered. The solvent was evaporated under reduced pressure. The residue was purified by basic silica gel column chromatography (ethyl acetate:hexane=2:1). The obtained residue was dissolved in methanol (30 ml), 4N hydrogen chloride-ethyl acetate solution (15 ml) was added, and the mixture was stirred at 50°C for 1 hr. The reaction mixture was concentrated under reduced pressure, and the obtained precipitate was collected by filtration, and washed with ethyl acetate to give 3,4-dihydro-2H-thiochromen-4-amine 1,1-dioxide hydrochloride (5.3 g).
   ¹H NMR (300 M Hz, DMSO-d₆) δ ppm 2.60-2.80 (2H, m) 3.66-3.86 (2H, m) 4.79 (1H, s) 7.64-7.90 (4H, m) 8.98 (3H, s).
(Step 3) 3,4-Dihydro-2H-thiochromen-4-amine 1,1-dioxide hydrochloride (2.0 g) obtained in Step 2, 2-cyano-2-(3,4-dichloro-5-oxo-2,5-dihydrofuran-2-yl)acetamide (1.0 g) and potassium carbonate (1.18 g) were stirred in ethanol (10 ml) overnight at 70°C. The reaction mixture was filtered through celite, and the solvent was evaporated under reduced pressure. The residue was purified by preparative HPLC. To the obtained yellow oil was added 2N hydrogen chloride-methanol solution at room temperature, and the precipitated crystals were collected by filtration and recrystallized to give the title compound (62 mg) .
   ¹H NMR (300 M Hz, DMSO-d₆) δ ppm 2.69-2.89 (1H, m) 2.91-3.07 (1H, m) 3.68-3.83 (1H, m) 3.84-3.98 (1H, m) 6.44 (1H, dd, J= 4.5, 9.1 Hz) 7.17-7.28 (1H, m) 7.60-7.77 (2H, m) 7.98 (1H, dd, J= 1.5, 7.6 Hz) 8.26 (2H, s) 8.60 (1H, d, J= 1.9 Hz) 8.68 (1H, s) 9.83-10.25 (2H, m).

### Example 28

### 5-chloro-2-imino-1-(1-(pyridin-2-yl)ethyl)-1,2-dihydropyridine-3-carboxamide dihydrochloride

2-Cyano-2-(3,4-dichloro-5-oxo-2,5-dihydrofuran-2-yl)acetamide (1.0 g), 1-(pyridin-2-yl)ethanamine (0.77 g) and potassium carbonate (1.18 g) were stirred in ethanol (10 ml) overnight at 70°C. The reaction mixture was filtered through celite, and the solvent was evaporated under reduced pressure. The residue was purified by basic silica gel column chromatography (ethyl acetate:hexane=7:3→1:0). To the obtained yellow oil was added 4N hydrogen chloride-ethyl acetate solution (1 ml), and the precipitated crystals were collected by filtration and recrystallized to give the title compound (610 mg).
¹H NMR (300 M Hz, DMSO-d₆) δ ppm 1.95 (3H, d, J=6.8 Hz), 6.37 (1H, q), 7.42 (1H, dd, J=6.4, 4.9 Hz), 7.75 (1H, d, J=7.6 Hz), 7.94 (1H, td, J=7.8, 1.9 Hz), 8.21 (1H, s), 8.55 (1H, d, J=4.2 Hz), 8.59 (1H, d, J=2.3 Hz), 8.66 (1H, d, J=1.9 Hz), 8.75 (1H, br. s.), 9.79 (2H, br. s.).

### Example 29

### 5-chloro-2-imino-1-(1-(pyridin-4-yl)ethyl)-1,2-dihydropyridine-3-carboxamide dihydrochloride

2-Cyano-2-(3,4-dichloro-5-oxo-2,5-dihydrofuran-2-yl)acetamide (1.0 g), 1-(pyridin-4-yl)ethanamine dihydrochloride (1.08 g) and potassium carbonate (1.76 g) were stirred in ethanol (10 ml) overnight at 70°C. The reaction mixture was filtered through celite, and the solvent was evaporated under reduced pressure. The residue was purified by basic silica gel column chromatography (ethyl acetate:hexane=7:3→1:0). To the obtained yellow oil was added 4N hydrogen chloride-ethyl acetate solution (1 ml), and the precipitated crystals were collected by filtration and recrystallized to give the title compound (160 mg).
¹H NMR (300 M Hz, DMSO-d₆) δ ppm 1.97 (3H, d, J=6.6 Hz), 6.37 (1H, d), 7.57 - 7.88 (2H, m), 8.25 (1H, br. s.), 8.51 (1H, d, J=2.1 Hz), 8.68 (1H, s), 8.71 - 8.78 (1H, m), 8.81 (2H, d, J=4.7 Hz), 9.93 (2H, br. s.).

### Example 30

### 5-chloro-1-(6-chloro-1,1-dioxido-3,4-dihydro-2H-thiochromen-4-yl)-2-imino-1,2-dihydropyridine-3-carboxamide hydrochloride

(Step 1) 6-Chloro-2,3-dihydro-4H-thiochromen-4-one (10.0 g) and o-methylhydroxylamine hydrochloride (5.47 g) were stirred in pyridine (50 ml) at room temperature for 16 hr. The reaction solution was poured into water, and the mixture was extracted with ethyl acetate. The extract was washed with 1N hydrochloric acid and saturated brine, dried over magnesium sulfate and filtered. The solvent was evaporated under reduced pressure. To a solution (200 ml) of the obtained residue in tetrahydrofuran was added tetrahydrofuran-borane (125 ml, 1M tetrahydrofuran solution) at 0°C. The reaction mixture was stirred at 70°C for 3 hr, and quenched with ice, and 1N hydrochloric acid (200 ml) was added. The reaction mixture was stirred at 70°C for 2 hr, and ethyl acetate was added thereto. The separated aqueous layer was basified with 8N sodium hydroxide solution, and extracted with ethyl acetate. The extract was washed with saturated brine, dried over magnesium sulfate and filtered. The solvent was evaporated under reduced pressure. The obtained residue was dissolved in methanol, 4N hydrogen chloride-ethyl acetate solution (25 ml) was added, and the obtained precipitate was collected by filtration, and washed with ethyl acetate to give 6-chloro-3,4-dihydro-2H-thiochromen-4-amine hydrochloride (3.86 g).
   ¹H NMR (300 M Hz, DMSO-d₆) δ ppm 2.05-2.44 (2H, m) 2.89-3.28 (2H, m) 4.56 (1H, s) 7.16-7.26 (1H, m) 7.28-7.37 (1H, m) 7.62 (1H, s) 8.58 (3H, s).
(Step 2) To a solution of 6-chloro-3,4-dihydro-2H-thiochromen-4-amine hydrochloride (3.8 g) obtained in Step 1 and triethylamine (3.26 g) in tetrahydrofuran (100 ml) was added di-tert-butyl dicarbonate (4.57 g) at room temperature. The mixture was stirred at the same temperature for 16 hr, the reaction solution was poured into 1N hydrochloric acid, and the mixture was extracted with ethyl acetate. The extract was washed with saturated brine, dried over magnesium sulfate and filtered. The solvent was evaporated under reduced pressure. The obtained residue was dissolved in ethyl acetate (200 ml), and m-chloroperbenzoic acid (9.13 g, containing water: Wako Pure Chemical Industries, Ltd.) was added at room temperature. The mixture was stirred at the same temperature for 1 hr, and quenched with aqueous sodium thiosulfate solution. The organic layer was washed with aqueous sodium hydrogen carbonate solution and saturated brine, dried over magnesium sulfate and filtered. The solvent was evaporated under reduced pressure. The residue was purified by basic silica gel column chromatography (ethyl acetate:hexane=1:1). The obtained residue was dissolved in methanol (50 ml), 4N hydrogen chloride-ethyl acetate solution (10 ml) was added, and the mixture was stirred at 60°C for 1 hr. The reaction mixture was concentrated under reduced pressure, and the residue was solidified from methanol-ethyl acetate to give 6-chloro-3,4-dihydro-2H-thiochromen-4-amine 1,1-dioxide hydrochloride (3.54 g).
   ¹H NMR (300 M Hz, DMSO-d₆) δ ppm 2.56-2.84 (2H, m) 3.65-3.92 (2H, m) 4.81 (1H, br. s.) 7.76 (1H, d, J=8.48 Hz) 7.92 (1H, d, J=8.48 Hz) 8.03 (1H, s) 8.94 (3H, br. s.).
(Step 3) 2-Cyano-2-(3,4-dichloro-5-oxo-2,5-dihydrofuran-2-yl)acetamide (0.70 g), 6-chloro-3,4-dihydro-2H-thiochromen-4-amine 1,1-dioxide hydrochloride (1.6 g) obtained in Step 2 and potassium carbonate (0.83 g) were stirred in ethanol (7 ml) at 80°C for 3 hr. The reaction solution was filtered through celite, and the solvent was evaporated under reduced pressure. The residue was purified by basic silica gel column chromatography (ethyl acetate:hexane=7:3-.1:0). To the obtained yellow oil was added 4N hydrogen chloride-ethyl acetate solution (1 ml), and the precipitated crystal was collected by filtration and recrystallized to give the title compound (54 mg).
   ¹H NMR (300 M Hz, DMSO-d₆) δ ppm 2.67-3.03 (2H, m) 3.68-3.99 (2H, m) 6.39-6.51 (1H, m) 7.55 (1H, d, J= 1.5 Hz) 7.77 (1H, dd, J= 2.1, 8.5 Hz) 7.99 (1H, d, J= 8.7 Hz) 8.20-8.33 (2H, m) 8.53-8.62 (1H, m) 8.67 (1H, s) 9.95-10.23 (2H, m).

### Example 31

### 5-chloro-1-(6-fluoro-1,1-dioxido-3,4-dihydro-2H-thiochromen-4-yl)-2-imino-1,2-dihydropyridine-3-carboxamide hydrochloride

(Step 1) 6-Fluoro-2,3-dihydro-4H-thiochromen-4-one (10.0 g) and o-methylhydroxylamine hydrochloride (5.5 g) were stirred in pyridine (50 ml) at room temperature for 16 hr. The reaction solution was poured into water, and the mixture was extracted with ethyl acetate. The extract was washed with 1N hydrochloric acid and saturated brine, dried over magnesium sulfate and filtered. The solvent was evaporated under reduced pressure. To a solution (300 ml) of the obtained residue in tetrahydrofuran was added tetrahydrofuran-borane (137 ml, 1M tetrahydrofuran solution) at 0°C. The reaction mixture was stirred at 70°C for 4 hr, and quenched with ice, and 1N hydrochloric acid (250 ml) was added. The mixture was stirred at 70°C for 2 hr, and ethyl acetate was added to the reaction mixture. The separated aqueous layer was basified with 8N sodium hydroxide solution, and extracted with ethyl acetate. The extract was washed with saturated brine, dried over magnesium sulfate and filtered. The solvent was evaporated under reduced pressure. The obtained residue was dissolved in methanol, 4N hydrogen chloride-ethyl acetate solution (20 ml) was added, and the obtained precipitate was collected by filtration, and washed with ethyl acetate to give 6-fluoro-3,4-dihydro-2H-thiochromen-4-amine hydrochloride (4.52 g).
   ¹H NMR (300 M Hz, DMSO-d₆) δ ppm 2.07-2.44 (2H, m) 2.96-3.28 (2H, m) 4.54 (1H, s) 7.01-7.30 (2H, m) 7.47 (1H, d, J=10.22 Hz) 8.66 (3H, s).
(Step 2) To a solution of 6-fluoro-3,4-dihydro-2H-thiochromen-4-amine hydrochloride (4.4 g) obtained in Step 1 and triethylamine (4.05 g) in tetrahydrofuran (100 ml) was added di-tert-butyl dicarbonate (5.68 g) at 0°C. The mixture was stirred at room temperature for 14 hr, the reaction solution was poured into 1N hydrochloric acid, and the mixture was extracted with ethyl acetate. The extract was washed with saturated brine, dried over magnesium sulfate and filtered. The solvent was evaporated under reduced pressure. The obtained residue was dissolved in ethyl acetate (200 ml), and m-chloroperbenzoic acid (10.86 g, containing water: Wako Pure Chemical Industries, Ltd.) was added at 0°C. The mixture was stirred at room temperature for 4 hr, and the reaction mixture was quenched with aqueous sodium thiosulfate solution. The organic layer was washed with aqueous sodium hydrogen carbonate solution and saturated brine, dried over magnesium sulfate and filtered. The solvent was evaporated under reduced pressure. The residue was purified by basic silica gel column chromatography (ethyl acetate:hexane=1:1). The obtained residue was dissolved in methanol (100 ml), 4N hydrogen chloride-ethyl acetate solution (15 ml) was added, and the mixture was stirred at 60°C for 1 hr. The reaction mixture was concentrated under reduced pressure to give 6-fluoro-3,4-dihydro-2H-thiochromen-4-amine 1,1-dioxide hydrochloride (4.35 g) as a solid.
   ¹H NMR (300 M Hz, DMSO-d₆) δ ppm 2.55-2.84 (2H, m) 3.61-3.91 (2H, m) 4.81 (1H, s) 7.43-7.64 (1H, m) 7.79 (1H, s) 7.99 (1H, dd, J=8.85, 5.65 Hz) 8.95 (3H, s).
(Step 3) In the same manner as in Example 30, Step 3, the title compound was obtained using 2-cyano-2-(3,4-dichloro-5-oxo-2,5-dihydrofuran-2-yl)acetamide and 6-fluoro-3,4-dihydro-2H-thiochromen-4-amine 1,1-dioxide hydrochloride obtained in Step 2.
   ¹H NMR (300 M Hz, DMSO-d₆) δ ppm 2.68-3.04 (2H, m) 3.69-3.98 (2H, m) 6.41-6.54 (1H, m) 7.33 (1H, dd, J= 2.3, 9.8 Hz) 7.56 (1H, td, J= 2.7, 8.5 Hz) 8.06 (1H, dd, J= 5.5, 8.9 Hz) 8.24 (1H, s) 8.31 (1H, s) 8.59 (1H, d, J=1.9 Hz) 8.67 (1H, br.s.) 9.91-10.25 (2H, m).

### Example 32

### 5-chloro-1-[(1R)-1-(3-cyanophenyl)ethyl]-2-imino-1,2-dihydropyridine-3-carboxamide L-tartrate

5-Chloro-1-[(1R)-1-(3-cyanophenyl)ethyl]-2-imino-1,2-dihydropyridine-3-carboxamide hydrochloride (1.2 g) obtained in Example 23 was dissolved in a mixed solvent of ethanol and ethyl acetate, 1N sodium hydroxide (30 ml) was added, and the mixture was extracted with ethyl acetate. The organic layer was washed with saturated brine, dried over magnesium sulfate and filtered. The solvent was evaporated under reduced pressure. To the residue (0.25 g) was added L-(+)-tartaric acid (0.13 g), and the mixture was stirred in a mixed solvent of ethanol (4 ml) and water (1 ml) overnight at room temperature. The precipitated crystals were collected by filtration, and recrystallized from ethanol, water and ethyl acetate to give the title compound (220 mg).
¹H NMR (300 M Hz, DMSO-d₆) δ ppm 1.84 (3H, d, J= 6.8 Hz) 3.94 (2H, s) 6.14 (1H, q, J= 6.9 Hz) 7.55-7.74 (2H, m) 7.78-7.99 (3H, m) 8.11-8.23 (2H, m) 8.38-8.67 (1H, m).

### Example 33

### 5-chloro-1-[(1R)-1-(3-cyanophenyl)ethyl]-2-imino-1,2-dihydropyridine-3-carboxamide sulfate

5-Chloro-1-[(1R)-1-(3-cyanophenyl)ethyl]-2-imino-1,2-dihydropyridine-3-carboxamide hydrochloride (0.75 g) obtained in Example 23 was dissolved in water, saturated sodium hydrogen carbonate (50 ml) was added, and the mixture was extracted three times with ethyl acetate. The combined organic layers were washed with saturated brine, dried over magnesium sulfate and filtered. The solvent was evaporated under reduced pressure. The residue was dissolved in ethanol (10 ml), concentrated sulfuric acid (142 µl) was added at room temperature, and the mixture was stirred overnight. The precipitated crystals were collected by filtration, and recrystallized from ethanol, water and ethyl acetate to give the title compound (0.81 g).
¹H NMR (300 M Hz, DMSO-d₆) δ ppm 1.93 (3H, d, J= 6.8 Hz) 6.06 (1H, q, J= 6.8 Hz) 7.58-7.78 (2H, m) 7.85-7.97 (2H, m) 8.22 (1H, s) 8.43 (1H, d, J= 2.3 Hz) 8.50-8.64 (2H, m) 9.42-9.86 (3H, m).

### Example 34

### 5-chloro-1-[(1S,25) -2-hydroxy-2,3-dihydro-1H-inden-1-yl]-2-imino-1,2-dihydropyridine-3-carboxamide hydrochloride

A solution of 2-cyano-2-(3,4-dichloro-5-oxo-2,5-dihydrofuran-2-yl)acetamide (0.79 g), (1S,2S)-1-amino-2,3-dihydro-1H-inden-2-ol (0.5 g) and diisopropylethylamine (1.7 ml) in ethanol (5 ml) was stirred overnight at 70°C. The reaction mixture was poured into 1N sodium hydroxide solution, and the mixture was extracted twice with ethyl acetate. The combined organic layers were washed with saturated brine, dried over magnesium sulfate and filtered. The solvent was evaporated under reduced pressure. The residue was purified by basic column chromatography. The obtained residue was dissolved in ethanol, and 4N hydrogen chloride-ethyl acetate solution was added. The precipitated crystals were collected by filtration, and recrystallized from ethanol and ethyl acetate to give the title compound (114 mg).
¹H NMR (300 M Hz, DMSO-d₆) δ ppm 2.87 (1H, dd, J= 7.2, 15.4 Hz) 3.30 (1H, dd, J= 7.2, 15.4 Hz) 4.81 (1H, q, J= 7.0 Hz) 6.03-6.31 (2H, m) 7.19-7.26 (1H, m) 7.27-7.36 (1H, m) 7.36-7.46 (2H, m) 8.03 (1H, br.s.) 8.26 (1H, s) 8.74 (1H, br.s.) 9.60-10.15 (2H, m).

The structural formulas of the compounds of Examples are shown in Table 1.

**Table 1**

| | | | | |
|---|---|---|---|---|
| Example 1 | Example 2 | Example 3 | Example 4 | Example 5 |
| 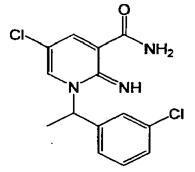 | 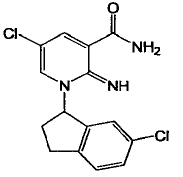 | 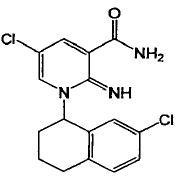 | 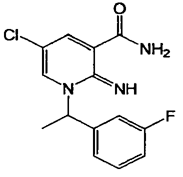 | 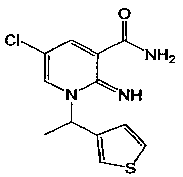 |
| | | | HCl | HCl |
| | | | | |
| Example 6 | Example 7 | Example 8 | Example 9 | Example 10 |
| 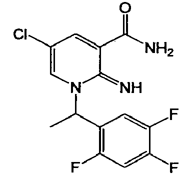 | 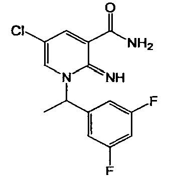 | 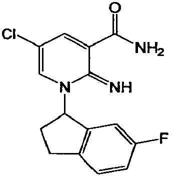 | 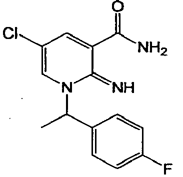 | 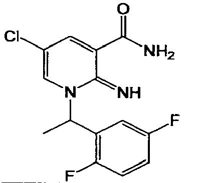 |
| HCl | HCl | HCl | HCl | HCl |
| | | | | |
| Example 11 | Example 12 | Example 13 | Example 14 | Example 15 |
| 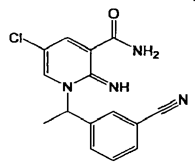 | 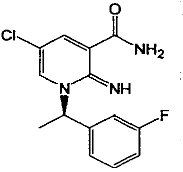 | 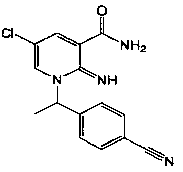 | 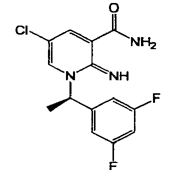 | 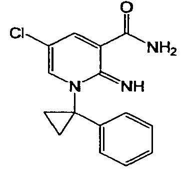 |
| HCl | HCl | HCl | HCl | |
| | | | | |
| Example 16 | Example 17 | Example 18 | Example 18 | Example 20 |
| 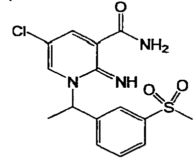 | 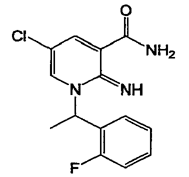 | 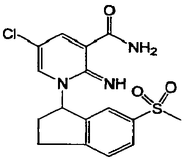 | 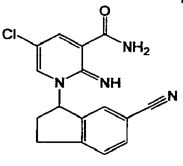 | 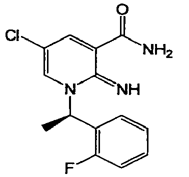 |
| HCl | HCl | HCl | HCl | HCl |
| | | | | |
| Example 21 | Example 22 | Example 23 | Example 24 | Example 25 |
| 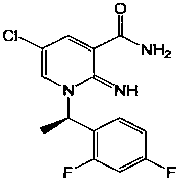 | 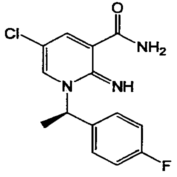 | 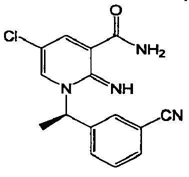 | 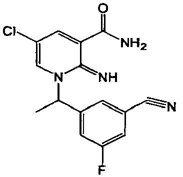 | 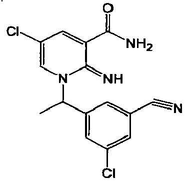 |
| HCl | HCl | HCl | HCl | HCl |
| Example 26 | Example 27 | Example 28 | Example 29 | Example 30 |
| 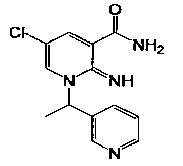 | 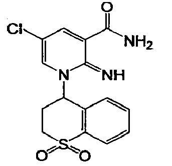 | 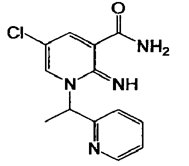 | 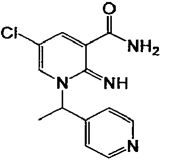 | 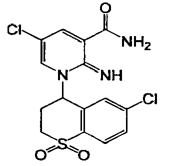 |
| 2HCl | HCl | 2HCl | 2HCl | HCl |
| | | | | |
| Example 31 | Example 32 | Example 33 | Example 34 | |
| 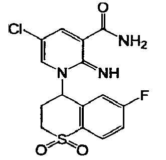 | 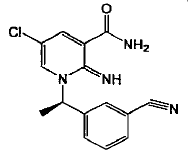 | 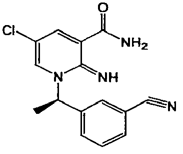 | 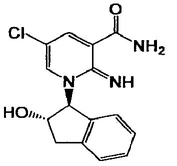 | |
| HCl | L-tartarate | H₂SO₄ | HCl | |

### Experimental Example 1

### Measurement of α_{1D} adrenergic receptor binding inhibitory activity

Genetic manipulation methods described below are based on the methods described in Maniatis et al., Molecular Cloning, Cold Spring Harbor Laboratory, 1989, the protocol appended to a reagent and the like.

### (i) Preparation of human α_{1D} adrenergic receptor expression plasmid

α_{1D} Adrenergic receptor gene was cloned from human liver cDNA by the PCR method. PCR reaction was performed by Gene Amp PCR System 9700 (Applied Biosystems) with 50 pmol each of the primer set 5'-CCGACGGCCGCTAGCGAGATGACTTTCCGCGATCTCCTGAGCGTC-3' [SEQ ID NO: 1] and 5'-GCTCTGGGTACCTTAAATATCGGTCTCCCGTAGGTTGC-3' [SEQ ID NO: 2] prepared in reference to the base sequence of the α_{1D} adrenergic receptor gene reported by DEBRA A. et al. (J. Pharamacol. Exp. Ter., 272, 134-142 (1995)),200 ng of human brain hippocampus cDNA library (Takara Shuzo Co., Ltd.) as a template and TaKaRa LA-Taq DNA Polymerase (Takara Shuzo Co., Ltd.) (reaction conditions: 45 cycles of 94°C for 15 sec, 68°C for 3.5 min).
The PCR fragment obtained above was digested with restriction enzymes NheI (Takara Shuzo Co., Ltd.) and Kpn I (Takara Shuzo Co., Ltd.), and applied to agarose gel electrophoresis to recover DNA fragments. The DNA fragments were ligated with animal cell expression plasmid pcDNA3.1/Zeo (Invitrogen) digested with NheI and Kpn I, by DNA Ligation Kit Ver.2 (Takara Shuzo Co., Ltd.), and transformed the competent cells of Escherichia coli JM109 to obtain plasmid, pcDNA3.1/Zeo-Adreα_{1D}.

### (ii) Introduction of human α_{1D} adrenergic receptor expression plasmid into CHO-K1 cells and preparation of membrane fraction

CHO-K1 cells passage cultured in HamF12 medium (Invitrogen) containing 10% fetal bovine serum (TRACE SCIENCETIFIC) in a 150 cm² culture flask (Corning Coaster) were detached with 0.5 g/L trypsin-0.2 g/L EDTA (Invitrogen), and the cells were washed with D-PBS(-) (Invitrogen) and centrifuged (1000 rpm, 5 min). Then, using Gene Pulser II (BioRad), DNA was introduced into the cells under the following conditions. 1x10⁷ cells suspended in D-PBS(-) (700 µl) and 10 µg of pcDNA3.1/Zeo-Adrea_{1D} were added in a 0.4 cm gap cuvette (BioRad), and electroporation was performed under voltage 0.25 kV, capacitance 960 µF. The cells were cultured in HamF12 medium containing 10% fetal bovine serum and 250 µg/mL Zeocin (Invitrogen) and the Zeocin resistance clones were selected.
Plurality of Zeocin resistance clones were selected and cultured in a cell culture flask (150 cm²) until semiconfluent, and the cellular membrane fraction was prepared as follows.
The semiconfluent cells were detached with 0.02% EDTA containing D-PBS(-) and recovered by centrifugation. The cells were suspended in membrane preparation buffer (10 mM NaHCO₃ pH 7.4, protease inhibitor cocktail (Roche)) and disrupted by 3 times of treatment in a polytron homogenizer (model PT-3100, KINEMATICA AG) at 20000 rpm for 20 seconds. After disruption, the cells were centrifuged at 2000 rpm for 10 min and the supernatant containing membrane fractions was obtained. The supernatant was centrifuged using an ultracentrifuge (model L8-70M, rotor 70 Ti, Beckman Instruments) at 30000 rpm for 1 hr to obtain a precipitate containing membrane fractions. The obtained membrane fraction of each clone was subjected to the binding experiment shown below.
The membrane fraction (20 µg/well) and [³H]-prazosin (2.5 nM, PerkinElmer Lifescience), as a ligand, were diluted with a binding assay buffer (50 mM Tris-HCl, 10 mM MgCl₂, 0.5% BSA, protease inhibitor cocktail pH 7.5), added to a 96 well microplate, and reacted at room temperature for 1 hr. For the measurement of non-specific binding, phentolamine (Sigma) was further added to 10 µM. Then, the reaction mixture was filtered and transferred to unifilter GF/C (PerkinElmer Lifescience) by using a cell harvester (PerkinElmer Lifescience). The filter was washed 3 times with ice-cooled 50 mM Tris buffer (pH 7.5). After drying the filter, MicroScinti 0 (PerkinElmer Lifescience) was added to the filter and the radioactivity was measured by TopCount (PerkinElmer Lifescience). Membrane fractions for compound evaluation shown below were prepared by a method similar to the above-mentioned method from the clones that showed the most superior S/B value (total binding radioactivity/non-specific binding radioactivity) in the binding measurement using the membrane fractions.

### (iii) Evaluation of Example compound

The membrane fraction (20 µg/well), the compound and [³H]-prazosin (2.5 nM, PerkinElmer Lifescience) were diluted with a binding assay buffer, added to a 96 well microplate, and the mixture was reacted at room temperature for 1 hr. For the measurement of non-specific binding, phentolamine (Sigma), which is a cold ligand, was further added to 10 µM. Then, the reaction mixture was filtered and transferred to unifilter GF/C (PerkinElmer Lifescience) by using a cell harvester (PerkinElmer Lifescience). The filter was washed 3 times with cooled 50 mM Tris buffer (pH 7.5). After drying the filter, MicroScinti 0 (PerkinElmer Lifescience) was added to the filter and the radioactivity was measured by TopCount (PerkinElmer Lifescience).
The concentration of the compound necessary for decreasing the amount of binding of [³H]-prazosin to the membrane fraction to 50% (IC₅₀) was calculated by GlaphPad Prism Ver3.2 (GlaphPad Software).
The results measured by the above-mentioned method (α_{1D} adrenergic receptor binding inhibitory rate at 1 µM) are shown in Table 2.

**Table 2**

| Test compound (Example No.) | Binding inhibitory rate (%) |
|---|---|
| 1 | 100.1 |
| 2 | 101.5 |
| 5 | 79.4 |
| 12 | 97.0 |
| 14 | 100.0 |
| 15 | 62.0 |
| 19 | 94.8 |
| 22 | 96.1 |
| 23 | 100.0 |
| 30 | 96.9 |

### Formulation Example 1

| | | |
|---|---|---|
| (1) | compound of Example 1 | 10 mg |
| (2) | lactose | 60 mg |
| (3) | cornstarch | 35 mg |
| (4) | hydroxypropylmethylcellulose | 3 mg |
| (5) | magnesium stearate | 2 mg |

A mixture of the compound (10 mg) obtained in Example 1, lactose (60 mg) and cornstarch (35 mg) is granulated using 10 wt% aqueous hydroxypropylmethylcellulose solution (0.03 mL, 3 mg as hydroxypropylmethylcellulose), dried at 40°C and passed through a sieve. The obtained granules are mixed with magnesium stearate (2 mg), and the mixture is compressed. The obtained core tablet is coated with a sugar coating of a suspension of saccharose, titanium dioxide, talc and gum arabic in water. The coated tablet is polished with beeswax to give a coated tablet.

### Formulation Example 2

| | | |
|---|---|---|
| (1) | compound of Example 1 | 10 mg |
| (2) | lactose | 70 mg |
| (3) | cornstarch | 50 mg |
| (4) | soluble starch | 7 mg |
| (5) | magnesium stearate | 3 mg |

The compound (10 mg) obtained in Example 1 and magnesium stearate (3 mg) are granulated with an aqueous soluble starch solution (0.07 mL, 7 mg as soluble starch), dried, and mixed with lactose (70 mg) and cornstarch (50 mg). The mixture is compressed to give a tablet.

### Industrial Applicability

The compound of the present invention has a superior selective α_{1D} adrenergic receptor antagonistic action, and is useful as an agent for the prophylaxis or treatment of a lower urinary tract disease and the like.

This application is based on patent application No. 113130/2008 filed in Japan, the contents of which are hereby incorporated by reference.

## Claims

1. A compound represented by the formula 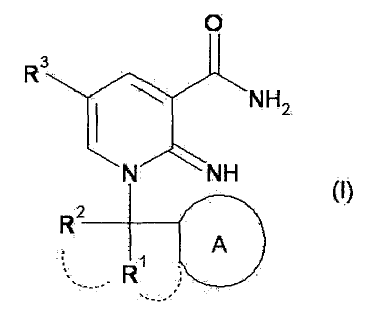 wherein
ring A is a phenyl group, a cycloalkyl group or a 5- or 6-membered aromatic heterocyclic group, each of which optionally has substituent(s),
R¹ is a methyl group, or R¹ and ring A in combination optionally form a fused cyclic group optionally having substituent(s),
R² is a hydrogen atom or a methyl group, or R¹ and R² in combination optionally form, together with the adjacent carbon atom, a cycloalkane ring, and
R³ is a hydrogen atom, a halogen atom, a cyano group, a hydrocarbon group optionally having substituent(s), an acyl group, a heterocyclic group optionally having substituent(s), an amino group optionally having substituent(s), a hydroxy group optionally having a substituent or a mercapto group optionally having a substituent,
provided that
5-chloro-1-(2,3-dihydro-1H-inden-1-yl)-2-imino-1,2-dihydropyridine-3-carboxamide,
5-chloro-2-imino-1-(1-phenylethyl)-1,2-dihydropyridine-3-carboxamide and
5-chloro-2-imino-1-(1,2,3,4-tetrahydronaphthalen-1-yl)-1,2-dihydropyridine-3-carboxamide
are excluded,
or a salt thereof.

2. The compound of claim 1, wherein ring A is a phenyl group optionally having substituent(s).

3. The compound of claim 1, wherein R³ is a halogen atom.

4. The compound of claim 1, wherein the group represented by the partial structural formula of the formula (I) 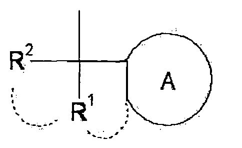 is a group represented by 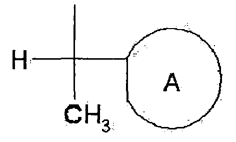 wherein ring A is as defined in claim 1.

5. The compound of claim 4, wherein ring A is (1) a phenyl group having 1 to 3 substituents selected from (a) a halogen atom, (b) a cyano group and (c) an alkylsulfonyl group, (2) a pyridyl group optionally having substituent(s), or (3) a thienyl group optionally having substituent(s).

6. The compound of claim 4, wherein ring A is (1) a phenyl group having 1 to 3 substituents selected from (a) a halogen atom, (b) a cyano group and (c) a C₁₋₆ alkylsulfonyl group, (2) a pyridyl group, or (3) a thienyl group.

7. The compound of claim 4, wherein
ring A is (1) a phenyl group having 1 to 3 substituents selected from (a) a halogen atom, (b) a cyano group and (c) a C₁₋₆ alkylsulfonyl group, (2) a pyridyl group, or (3) a thienyl group, and
R³ is a halogen atom.

8. The compound of claim 4, wherein
ring A is a phenyl group having 1 to 2 substituents selected from (a) a halogen atom and (b) a cyano group, and
R³ is a halogen atom.

9. The compound of claim 1, wherein the group represented by the partial structural formula of the formula (I) 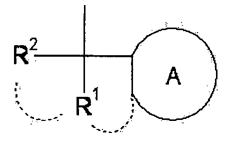 is a fused cyclic group represented by 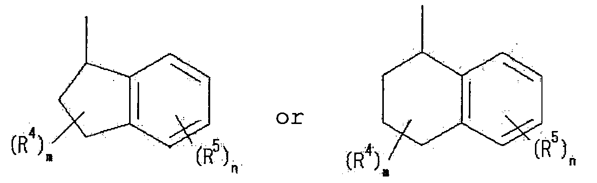 wherein
R⁴ and R⁵ are the same or different and each is a substituent selected from a hydroxy group, a halogen atom, a cyano group and -S(O)ₚ-R⁶ wherein R⁶ is an alkyl group, and p is an integer of 0 to 2,
m is an integer of 0 to 3, and
n is an integer of 0 to 4.

10. The compound of claim 9, wherein
R⁴ is a hydroxy group,
R⁵ is a substituent selected from a halogen atom, a cyano group and -S(O)ₚ-R⁶ wherein R⁶ is a C₁₋₆ alkyl group, and p is an integer of 0 to 2, and
m+n=1 or 2, provided that m and n are the same or different and each is 0 or 1.

11. The compound of claim 9, wherein
R³ is a halogen atom,
R⁴ is a hydroxy group,
R⁵ is a substituent selected from a halogen atom, a cyano group and -S(O)ₚ-R⁶ wherein R⁶ is a C₁₋₆ alkyl group, and p is an integer of 0 to 2, and
m+n=1 or 2, provided that m and n are the same or different and each is 0 or 1.

12. The compound of claim 9, wherein
R³ is a halogen atom,
R⁵ is a halogen atom,
m is 0, and
n is 1.

13. The compound of claim 1, wherein the group represented by the partial structural formula of the formula (I) 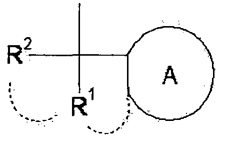 is a fused cyclic group represented by 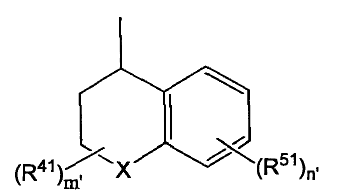 wherein R⁴¹ and R⁵¹ are the same or different and each is a substituent selected from a hydroxy group, a halogen atom, a cyano group and -S(O)ₚ,-R⁶¹ wherein R⁶¹ is an alkyl group, and p' is an integer of 0 to 2,
X is S, SO or SO₂,
m' is an integer of 0 to 3, and
n' is an integer of 0 to 4.

14. The compound of claim 13, wherein
R³ is a halogen atom,
R⁵¹ is a halogen atom,
m' is 0, and
n' is 0 or 1.

15. The compound of claim 13, wherein
R³ is a halogen atom,
R⁵¹ is a halogen atom,
X is SO₂,
m' is 0, and
n' is 1.

16. The compound of claim 1, wherein the group represented by the partial structural formula of the formula (I) 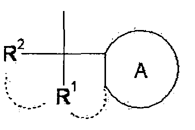 is a group represented by 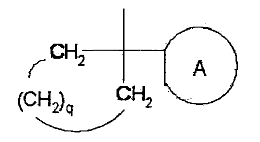 wherein q is an integer of 0 to 4, and ring A is as defined in claim 1.

17. 5-Chloro-1-[1-(3-chlorophenyl)ethyl]-2-imino-1,2-dihydropyridine-3-carboxamide or a salt thereof.

18. 5-Chloro-1-(6-chloro-2,3-dihydro-1H-inden-1-yl)-2-imino-1,2-dihydropyridine-3-carboxamide or a salt thereof.

19. 5-Chloro-1-[(1R)-1-(3-fluorophenyl)ethyl]-2-imino-1,2-dihydropyridine-3-carboxamide or a salt thereof.

20. 5-Chloro-1-[(1R)-1-(3,5-difluorophenyl)ethyl]-2-imino-1,2-dihydropyridine-3-carboxamide or a salt thereof.

21. 5-Chloro-1-[(1R)-1-(3-cyanophenyl)ethyl]-2-imino-1,2-dihydropyridine-3-carboxamide or a salt thereof.

22. 5-Chloro-1-(6-chloro-1,1-dioxido-3,4-dihydro-2H-thiochromen-4-yl)-2-imino-1,2-dihydropyridine-3-carboxamide or a salt thereof.]

23. A prodrug of the compound of claim 1.

24. A pharmaceutical agent comprising the compound of claim 1 or a prodrug thereof.

25. The pharmaceutical agent of claim 24, which is α_{1D} adrenoceptor antagonist.

26. The pharmaceutical agent of claim 24, which is an agent for the prophylaxis or treatment of lower urinary tract diseases.

27. A method for the prophylaxis or treatment of lower urinary tract diseases in a mammal, which comprises administering an effective amount of the compound of claim 1 or a prodrug thereof to the mammal.

28. Use of the compound of claim 1 or a prodrug thereof for the production of an agent for the prophylaxis or treatment of lower urinary tract diseases.


### Sequence Listing

SEQUENCE LISTING

<110>  Takeda Pharmaceutical Company Limited

<120>  Iminopyridine derivatives and use thereof

<130>  091377

<150>  JP2008-113130
<151>  2008-04-23

<160>  2

<170>  PatentIn version 3.3

<210>  1
<211>  45
<212>  DNA
<213>  Artificial

<220>
<223>  Primer for cloning human alpha1D adrenaline receptor gene

<400>  1
ccgacggccg ctagcgagat gactttccgc gatctcctga gcgtc                45


<210>  2
<211>  38
<212>  DNA
<213>  Artificial

<220>
<223>  Primer for cloning human alpha1D adrenaline receptor gene

<400>  2
gctctgggta ccttaaatat cggtctcccg taggttgc                        38